# EUROPEAN PATENT APPLICATION

(11) **EP 4 272 642 A1**
(43) Date of publication of application: **08.11.2023**
(21) Application number: 23170949.4
(22) Date of filing: 02.05.2023
(51) Int. Cl.: A61B 5/16, A61B 5/00

(54) **TEST METHOD AND APPARATUS FOR EVALUATING COGNITIVE FUNCTION DECLINE**

(30) Priority: 02.05.2022 KR 20220054152; 29.07.2022 KR 20220094657
(71) Applicant: Emocog Co., Ltd., Seoul 08813 (KR)
(72) Inventor: NOH, Yoo Hun, 06346 Gangnam-gu, Seoul (KR); KIM, Hai Rin, 08830 Gwanak-gu, Seoul (KR); KIM, Ju Hye, 05266 Gangdong-gu, Seoul (KR)
(74) Representative: Hoffmann Eitle

(57) **Abstract**

Provided are a test method and apparatus for evaluating cognitive function decline. The test method for evaluating cognitive function decline includes performing a testing stage of testing how many missions presented for each of a plurality of test items a user performs, and calculating a test score for each of the plurality of test items, performing a category classifying stage of classifying at least one test item from among the plurality of test items as one neurocognitive category from among a plurality of neurocognitive categories, and performing an evaluating stage of evaluating whether there is cognitive function decline in the one neurocognitive category, based on a test score of the at least one test item classified as the one neurocognitive category.

## Description

### BACKGROUND

### 1. Field

The disclosure relates to a test method and apparatus for evaluating a cognitive function decline.

### 2. Description of the Related Art

As the aging society continues, the number of dementia patients is rapidly increasing. However, there is no cure for dementia. In this regard, the most innovative counterplan for dementia is preventive treatment by detecting, at an early stage, mild cognitive impairment that is highly likely to develop into dementia. The mild cognitive impairment indicates a state of high-risk group in a pre-dementia stage. About 10 to 15 % of mild cognitive impairment patients are reported to have dementia.

However, there is no standardized diagnosis method for mild cognitive impairment, and a doctor treats a patient face-to-face and determines the mild cognitive impairment by comprehensively examining a condition of the patient. General tests for evaluating cognitive function decline mainly consist of tests that evaluate memory. Such tests are effective when Alzheimer's disease characterized by memory decline is evaluated, but may have low accuracy when another dementia characterized by cognitive function decline other than memory is evaluated.

The aforementioned background technology is technical information possessed by the inventor for derivation of the disclosure or acquired by the inventor during the derivation of the disclosure, and is not necessarily prior art disclosed to the public before the application of the disclosure.

### SUMMARY

One aspect of the disclosure is to provide a user interface that is explicit and easy to understand during a test for evaluating cognitive function decline online.

One aspect of the disclosure is to perform a test by directly involving a user and reduce involvement of a tester, during a test for evaluating cognitive function decline online.

One aspect of the disclosure is to immediately identify a test result after a test for evaluating cognitive function decline online is completed, and enable tracing by identifying a stored past result.

Additional aspects will be set forth in part in the description which follows and, in part, will be apparent from the description, or may be learned by practice of the presented embodiments of the disclosure.

According to an embodiment of the disclosure, a test method for evaluating cognitive function decline is executed by a processor of a test apparatus for evaluating cognitive function decline, and includes performing a testing stage of testing how many missions presented for each of a plurality of test items a user performs, and calculating a test score for each of the plurality of test items, performing a category classifying stage of classifying at least one test item from among the plurality of test items as one neurocognitive category from among a plurality of neurocognitive categories, and performing an evaluating stage of evaluating whether there is cognitive function decline in the one neurocognitive category, based on a test score of the at least one test item classified as the one neurocognitive category.

According to another embodiment of the disclosure, a test apparatus for evaluating cognitive function decline includes a processor, and a memory operatively connected to the processor and storing at least one code performed by the processor, wherein the memory stores at least one code which, when executed through the processor, causes the processor to perform test processes of testing how may missions presented for each of a plurality of test items a user performs, and calculating a test score for each of the plurality of test items, perform a category classification process of classifying at least one test item from among the plurality of test items as one neurocognitive category from among a plurality of neurocognitive categories, and perform an evaluation process of evaluating whether there is cognitive function decline in the one neurocognitive category, based on a test score of the at least one test item classified as the one neurocognitive category.

In addition, provided are another method for implementing the disclosure, another system for implementing the disclosure, and a computer-readable recording medium having stored therein a computer program for executing the method.

Other aspects, features, and advantages may become clear from the following drawings, the claims, and the detailed description of the disclosure.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram of a test environment for evaluating cognitive function decline, according to an embodiment;
FIG. 2 is a block diagram for schematically describing a configuration of a test apparatus for evaluating cognitive function decline, according to an embodiment;
FIG. 3 is a block diagram for schematically describing a configuration of a test management unit in the test apparatus of FIG. 2 for evaluating cognitive function decline;
FIGS. 4 through 15K illustrate examples of screens provided on a user terminal to execute a test for evaluating cognitive function decline, according to an embodiment;
FIG. 16 is a block diagram for schematically describing a configuration of a test apparatus for evaluating cognitive function decline, according toother an embodiment; and
FIGS. 17 through 20 are flowcharts of test methods for evaluating cognitive function decline, according to embodiments.

### DETAILED DESCRIPTION

Advantages and features of the disclosure and methods of accomplishing the same may be understood more readily by reference to the following detailed description of the embodiments and the accompanying drawings. However, it should be understood that the disclosure is not limited to the embodiments presented below, but may be implemented in various different forms, and include all transformations, equivalents, and substitutes included in the spirit and scope of the disclosure. The embodiments presented below are provided to complete the disclosure and to fully inform one of ordinary skill in the art of the scope of the disclosure. In the description of the disclosure, certain detailed explanations of related art are omitted when it is deemed that they may unnecessarily obscure the essence of the disclosure.

Also, the terms used in the present specification are only used to describe specific embodiments, and are not intended to limit the disclosure. An expression used in the singular encompasses the expression in the plural, unless it has a clearly different meaning in the context. In the present specification, it is to be understood that terms such as "including" or "having", etc., are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. While such terms as "first", "second", etc., may be used to describe various components, such components are not limited to the above terms. The above terms are used only to distinguish one component from another.

Furthermore, in the specification, the term "unit" or "-or/er" may be a hardware component such as a processor or circuit and/or a software component that is executed by a hardware component such as a processor.

One or more embodiments will be described below in more detail with reference to the accompanying drawings. Those components that are the same or are in correspondence with each other are rendered the same reference numeral regardless of the figure number, and redundant explanations are omitted.

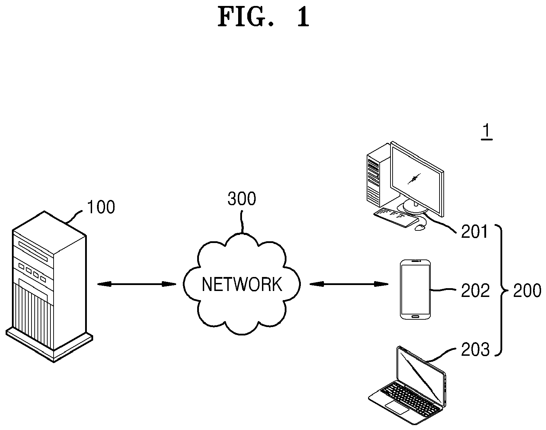

FIG. 1 is a diagram of a test environment 1 for evaluating cognitive function decline, according to an embodiment. Referring to FIG. 1, the test environment 1 for evaluating cognitive function decline may include a test apparatus 100 for evaluating cognitive function decline, a user terminal 200, and a network 300.

The test apparatus 100 may perform test processes of testing how many missions presented for each of a plurality of test items a user performs, and calculating a test score for each of the plurality of test items. In the present embodiment, the test processes may be represented as a testing stage in the claims described below.

In the present embodiment, the test apparatus 100 may perform the test processes including a first test process through an eleventh test process.

The first test process may include a process of performing a test for a first test item, in which it is evaluated how many words corresponding to a pre-set presented category are uttered within a time limit (for example, 30 seconds), and how many drawings of indicated designs are drawn within the time limit, and calculating a first test score. Here, the first test item may include a fluency test.

In the present embodiment, the first test process may include a (1-1)th test process and a (1-2)th test process.

The (1-1)th test process may include a process of performing a test for a (1-1)th test item, in which it is evaluated how many words corresponding to the pre-set presented category are uttered within a first time limit, and calculating a (1-1)th test score. Here, the (1-1)th test item may include a language fluency test. In this regard, the (1-1)th test process may include a process of performing the language fluency test on a user and calculating the (1-1)th test score.

The (1-2)th test process may include a process of performing a test for a (1-2)th test item, in which it is evaluated how many drawings of the indicated designs are drawn within a second time limit, and calculating a (1-2)th test score. Here, the (1-2)th test item may include a design fluency test. In this regard, the (1-2)th test process may include a process of performing the design fluency test on the user and calculating the (1-2)th test score.

A difference between the fluency test included in the first test process according to the present embodiment and a general fluency test may be as follows. In the fluency test according to the present embodiment, content uttered by the user may be recorded by using a voice recognition technology, and converted into a text in real time. Also, a test score may be calculated by comparing the text with a correct answer. In the design fluency test among the fluency test according to the present embodiment, a result of the user self-dragging and connecting a figure on a screen provided on the user terminal 200 may be received.

The second test process may be performed after the performing of the first test process is completed. The second test process may include a process of performing a test for a second test item, in which words are learned while indicating a pre-set category clue and it is evaluated how many learned words are recalled after a pre-set time (for example, 20 minutes), and calculating a second test score. Here, the second test item may include a language clue memory test.

In the present embodiment, the second test process may include a (2-1)th test process and a (2-2)th test process.

The (2-1)th test process may include a process of performing a test for a (2-1)th test item, in which it is evaluated how much ability to learn words is performed while indicating a pre-set category clue, and calculating a (2-1)th test score. Here, the (2-1)th test item may include a learning test among the language clue memory test. In this regard, the (2-1)th test process may include a process of performing the learning test among the language clue memory test on the user and calculating the (2-1)th test score.

The (2-2)th test process may be performed when a pre-set time has elapsed after the performing of the (2-1)th test process is completed. In the present embodiment, other test processes may be performed from a time when the performing of the (2-1)th test process is completed to the pre-set time. For example, the (2-2)th test process is not performed after the performing of the (2-1)th test process is completed, but the (2-2)th test process may be performed only after the performing of the (2-1)th test process is completed and then the third test process through the seventh test process are performed.

The (2-2)th test process may include a process of performing a test for a (2-2)th test item, in which it is evaluated how many words learned in the (2-1)th test process are recalled after the pre-set time has elapsed, and calculating the (2-2)th test score. Here, the (2-2)th test item may include a word recall and recognition test among the language clue memory test. In this regard, the (2-2)th test process may include a process of performing the word recall and recognition test among the language clue memory test on the user and calculating the (2-2)th test score.

In the language clue memory test included in the second test process according to the present embodiment, a one-time memory and a long-term memory may be formed through processes of 1) registering or encoding, 2) storing and retaining, and 3) retrieving. Also, memory evaluation, including determination on whether the one-time memory is damaged, and severity or characteristics of the damage, may be performed based on detailed and comprehensive evaluation on these three processes. A memory function may be accurately determined through the language clue memory test according to the present embodiment, and a diagnostic value for evaluating a dementia type may be secured. Here, the accurately determining of the memory function may include determining whether there is a retrieval failure issue or a memory scheme issue.

A difference between the language clue memory test included in the second test process according to the present embodiment and a general language clue memory test may be as follows. The general language clue memory test may be test of instructing a plurality of words and determining how many words are recalled. However, in the language clue memory test according to the present embodiment, a clue indicating a category of a word is provided and learned, and a question is asked while instructing a category of a word even during free recall.

A normal group and mild cognitive impairment, and a normal group and Alzheimer's disease may be evaluated through the language clue memory test included in the second test process according to the present embodiment. The language clue memory test according to the present embodiment may exhibit a significant correlation with gray matter volume of parahippocampus that is an area of a brain related to episodic memory. Thus, the language clue memory test according to the present embodiment may accurately evaluate language memory within a short period of time.

The third test process may be performed after the performing of the (2-1)th test process is completed. The third test process may include a process of performing a test for a third test item, in which a pre-set presented image is learned and it is evaluated how much the learned presented image is recalled after a pre-set time (for example, 20 minutes), and calculating a third test score. Here, the third test item may include a visual graphic memory test.

In the present embodiment, the third test process may include a (3-1)th test process and a (3-2)th test process.

The (3-1)th test process may include a process of performing a test for a (3-1)th test item, in which a pre-set first presented image is shown and visual perception stimulation is input through a process of determining the number of colors in the first presented image, and calculating a (3-1)th test score. Here, the (3-1)th test item may include a learning test among the visual graphic memory test. In this regard, the (3-1)th test process may include a process of performing the learning test among the visual graphic memory test on the user and calculating the (3-1)th test score.

The (3-2)th test process may be performed when a pre-set time has elapsed after the performing of the (3-1)th test process is completed. In the present embodiment, other test processes may be performed from a time when the performing of the (3-1)th test process is completed to the pre-set time. For example, the (3-2)th test process is not performed after the performing of the (3-1)th test process is completed, but the (3-2)th test process may be performed only after the performing of the (3-1)th test process is completed, the fourth test process through the seventh test process are performed, and then the (2-2)th test process is performed.

In the present embodiment, an order of performing the test processes maybe as follows. First, the first test process may be performed, the (2-1)th test process may be performed next, the (3-1)th test process may be performed next, the fourth test process through the seventh test process may be sequentially performed next, the (2-2)th test process may be performed next, the (3-2)th test process may be performed next, and then the eighth test process through the eleventh test process may be sequentially performed next.

In the present embodiment, the (2-2)th test process and the (3-2)th test process are performed necessarily after the performing of the seventh test process is completed, but the disclosure is not limited thereto. In other words, the (2-2)th test process and the (3-2)th test process may be performed after performing of one of the fourth test process through the eleventh test process is completed.

The (3-2)th test process may include a process of performing a test for a (3-2)th test item, in which it is evaluated how many presented images learned in the (3-1)th test process are recalled after the pre-set time has elapsed, and calculating a (3-2)th test score. Here, the (3-2)th test item may include a recognition test among the visual graphic memory test. In this regard, the (3-2)th test process may include a process of performing the recognition test among the visual graphic memory test on the user and calculating the (3-2)th test score.

A difference between the visual graphic memory test included in the third test process according to the present embodiment and a general visual graphic memory test may be as follows. In the visual graphic memory test according to the present embodiment, interference caused by semantic memory may be excluded by using an abstract image that is unable to be expressed in language. Also, a set of images may be configured by changing a color, a pattern, or an overlapping degree. Also, when performing the recognition test among the visual graphic memory test, an image included in a same set as a learned image may be presented as an interference image. Also, by choosing and presenting an arbitrary image in a set including a same clue, a learning effect of choosing a familiar image according to past tests may be excluded.

The fourth test process may be performed after the performing of the (3-1)th test process is completed. The fourth test process may include a process of performing a test for a fourth test item, in which it is evaluated whether a pre-set presented word and a color of the pre-set presented word are accurately uttered, and calculating a fourth test score. Here, the fourth test item may include a Stroop test.

In the present embodiment, the fourth test process may include a (4-1)th test process and a (4-2)th test process.

The (4-1)th test process may include a process of performing a test for a (4-1)th test item, in which it is evaluated whether a pre-set word is accurately uttered, and calculating a (4-1)th test score. Here, the (4-1)th test item may include a first Stroop test. In this regard, the (4-1)th test process may include a process of performing the first Stroop test on the user and calculating the (4-1)th test score.

The (4-2)th test process may include a process of performing a test for a (4-2)th test item, in which it is evaluated whether a color of a pre-set presented word is accurately uttered, and calculating a (4-2)th test score. Here, the (4-2)th test item may include a second Stroop test. In this regard, the (4-2)th test process may include a process of performing the second Stroop test on the user and calculating the (4-2)th test score.

A difference between the Stroop test included in the fourth test process according to the present embodiment and a general Stroop test may be as follows. In the general Stroop test, a test is performed by using a questionnaire to read provided words continuously, and the number of words that have been accurately read is counted to calculate a test score. However, the Stroop test according to the present embodiment may include a word reading task of choosing a same word as a word indicating a name of a color, and a color reading task of choosing a word matching a color of a word. The Stroop test according to the present embodiment may reduce a performance time considering a time that may show significant test results, and evaluate the number of positive responses during the performance time.

The fifth test process may be performed after the performing of the fourth test process is completed. The fifth test process may include a process of performing a test for a fifth test item, in which it is evaluated whether a line having a similar angle as a pre-set presented line is found, and calculating a fifth test score. Here, the fifth test item may include a line angle perception test. In this regard, the fifth test process may include a process of performing the line angle perception test on the user and calculating the fifth test score.

A difference between the line angle perception test included in the fifth test process according to the present embodiment and a general line angle perception test may be as follows. The line angle perception test according to the present embodiment may be a test of finding a choice line corresponding to a presented line by comparing a pair of presented lines with a pre-presented plurality of choice lines. In the line angle perception test according to the present embodiment, a decline level of visuospatial perception may be evaluated while reducing an angle (gradient) difference between the pair of presented lines and the plurality of choice lines.

The sixth test process may be performed after the performing of the fifth test process is completed. The sixth test process may include a process of performing a test for a sixth test item, in which it is evaluated whether a figure having a color different from a color of a pre-set presented figure is found, and calculating a sixth test score. Here, the sixth test item may include a color perception test. In this regard, the sixth test process may include a process of performing the color perception test on the user and calculating the sixth test score.

A difference between the color perception test included in the sixth test process according to the present embodiment and a general color perception test may be as follows. The Farnsworth Munsell 100 hue test, as the general color perception test, distinguishes and lists similar colors in four color ranges, based on hue, chroma, and brightness, i.e., three attributes of colors. It is verified whether the user distinguishes differences in colors by arranging an order of colors, based on colors at both ends. On the other hand, in the color perception test according to the present embodiment, colors may be selected and configured based on color systems of green and blue series, considering that dementia patients with Parkinson's disease have difficulty in distinguishing between blue and green. Unlike the general test in which all colors are listed, the color perception test according to the present embodiment may be configured by selecting two colors and setting a difficulty level high when a color difference is small and a difficulty level low when a color difference is large.

The seventh test process may be performed after the performing of the sixth test process is completed. The seventh test process may perform a test for a seventh test item, in which it is evaluated whether sizes and weights of pre-set presented objects are arranged in a descending order, and calculate a seventh test score. Here, the seventh test item may include a size weight test.

In the present embodiment, the seventh test process may include a (7-1)th test process and a (7-2)th test process.

In the (7-1)th test process, a test for a (7-1)th test item, in which it is evaluated whether sizes of pre-set presented objects are arranged in a descending order, may be performed, and a (7-1)th test score may be calculated. Here, the (7-1)th test item may include a size test. In this regard, the (7-1)th test process may include a process of performing the size test on the user and calculating the (7-1)th test score.

In the (7-2)th test process, a test for a (7-2)th test item, in which it is evaluated whether weights of pre-set presented objects are arranged in a descending order, may be performed, and a (7-2)th test score may be calculated. Here, the (7-2)th test item may include a weight test. In this regard, the (7-2)th test process may include a process of performing the weight test on the user and calculating the (7-2)th test score.

A difference between the size weight test included in the seventh test process according to the present embodiment and a general size weight test may be as follows. In the size weight test according to the present embodiment, objects according to sizes and objects according to weights may each be classified according to a plurality of categories (for example, 5), and difficulty levels may be set to low/medium/high by bundles of levels between categories. Combinations of categories, in which a weight difference and a size difference are large, may have low difficulty levels. Combinations of categories, in which a weight difference and a size difference are small, may have high difficulty levels. Also, questions may be easy in the beginning and become difficult towards the end. An image of an object may be configured such that features the object (for example, an animal or a thing) are presented well in a line drawing. Also, an object that is not familiar to aged Korean people may be excluded.

The eighth test process may be performed after the performing of the (3-2)th test process is completed. The eighth test process may include a process of performing a test for an eighth test item, in which it is evaluated whether intensity of an emotion shown on a pre-set face presented image is recognized, and calculating an eighth test score. Here, the eighth test item may include a face emotion test. In this regard, the eighth test process may include a process of performing the face emotion test on the user and calculating the eighth test score.

A difference between the face emotion test included in the eighth test process according to the present embodiment and a general face emotion test may be as follows. Generally, a dementia patient may have an emotion recognition disorder due to a change in a cognitive function. Also, the dementia patient may experience a difficulty in exchanging emotions with others compared to normal people, and have a low empathy ability in personal relations. In particular, frontotemporal dementia may exhibit changes in interpersonal behavior, such as insensitivity, a social processing error, and damage to social perception. Compared to a normal group, the frontotemporal dementia may exhibit low face emotion recognition. In this regard, the face emotion test according to the present embodiment may provide a plurality of first face presented images including six basic emotions (joy, fear, sadness, anger, disgust, and surprise), and a plurality of second face presented images that do not include emotions, i.e., emotionless, and determine whether an emotion included in each facial expression is sensitively perceived.

The ninth test process may be performed after the performing of the eighth test process is completed. The ninth test process may include a process of performing a test for a ninth test item, in which it is evaluated whether a pre-set presented paragraph is accurately uttered, and calculating a ninth test score. Here, the ninth test item may include a paragraph reading test. In this regard, the ninth test process may include a process of performing the paragraph reading test on the user and calculating the ninth test score.

A difference between the paragraph reading test included in the ninth test process according to the present embodiment and a general paragraph reading test may be as follows. Generally, primary progressive aphasia (PPA) may include a disorder in which a decline of a language function is gradually progressed without another cognitive function disorder. The PPA may be classified into, according to symptoms, progressive non-fluent aphasia, semantic dementia, and logopenic variant primary progressive aphasia. A patient with progressive non-fluent aphasia may know the meaning of a word, but may have difficulty in speaking the word by him/herself, exhibit a lot of grammatical errors, and exhibit phonemic paraphasia or stammer during naming. A patient with semantic dementia may relatively maintain eloquentness, but may have a severe naming disorder at an early stage due to no concept in words or objects. A patient with logopenic variant primary progressive aphasia may maintain relatively good language understanding and fluency, but may have difficulty in recalling a name of an object, exhibit a low uttering speed, and exhibit a disorder in understanding and repeating a long sentence.

In the paragraph reading test according to the present embodiment, a standardized paragraph, in which frequency of graphemes are adjusted such that a sentence is phonologically balanced, may be configured. Also, content of the paragraph may include content familiar to aged people. In the paragraph reading test according to the present embodiment, uttered content may be recorded and uttering speed may be measured. Also, the paragraph reading test according to the present embodiment may evaluate a reading ability by checking a phonological processing ability, such as phoneme substitution, omission, addition, and synthesis, by using a voice recognition technology. The paragraph reading test according to the present embodiment may calculate a test score by extracting an error from an utterance text, and evaluate an uttering speed, a pause length, voice pitch by analyzing recorded uttered voice.

The tenth test process may be performed after the performing of the ninth test process is completed. In the tenth test process, a test for a tenth test item, in which it is evaluated whether locations and order of pre-set presented figures are recalled, may be performed, and a tenth test score may be calculated. Here, the tenth test item may include a visuospatial attention test.

In the present embodiment, the tenth test process may include a (10-1)th test process and a (10-2)th test process.

In the (10-1)th test process, a test for a (10-1)th test item, in which it is evaluated whether locations and order pre-set presented figures appear in a pre-set region are sequentially recalled, may be performed, and a (10-1)th test score may be calculated. Here, the (10-1)th test item may include a first visuospatial attention test. In this regard, the (10-1)th test process may include a process of performing the first visuospatial attention test on the user and calculating the (10-1)th test score.

In the (10-2)th test process, a test for a (10-2)th test item, in which it is evaluated whether locations and order pre-set presented figures appear in a pre-set region are recalled in reverse order, may be performed, and a (10-2)th test score may be calculated. Here, the (10-2)th test item may include a second visuospatial attention test. In this regard, the (10-2)th test process may include a process of performing the second visuospatial attention test on the user and calculating the (10-2)th test score.

A difference between the visuospatial attention test included in the tenth test process according to the present embodiment and a general visuospatial attention test may be as follows. The visuospatial attention test according to the present embodiment may include a test for evaluating a working memory, in particular, a spatial working memory, among an executive function area. The visuospatial attention test according to the present embodiment may measure attention by having the user to recall locations and order figures are presented, and sequentially and repeatedly input the locations of the figures. Also, the visuospatial attention test according to the present embodiment may measure the working memory by having the user to repeatedly input the locations of the figures in reverse order. The number of figures presented in the visuospatial attention test according to the present embodiment may increase, and the number of successes, in which the user has accurately recalled the locations and order, may be a memory span. The visuospatial attention test according to the present embodiment may automatically record test results, and may stop when an input fails. The visuospatial attention test according to the present embodiment may evaluate a response speed by measuring a response time of choosing a figure.

The eleventh test process may be performed after the performing of the tenth test process is completed. In the eleventh test process, a test for an eleventh test item, in which it is evaluated whether a word corresponding to a condition is accurately chosen such that a pre-set presented incomplete sentence becomes a complete sentence, may be performed, and an eleventh test score may be calculated. Here, the eleventh test item may include a Hayling test.

In the present embodiment, the eleventh test process may include a (11-1)th test process and a (11-2)th test process.

In the (11-1)th test process, a test for a (11-1)th test item, in which it is evaluated whether an accurate word is chosen such that a pre-set presented incomplete sentence becomes a complete sentence, may be performed and a (11-1)th test score may be calculated. Here, the (11-1)th test item may include a first Hayling test. In this regard, the (11-1)th test process may include a process of performing the first Hayling test on the user and calculating the (11-1)th test score.

In the (11-2)th test process, a test for a (11-2)th test item, in which it is evaluated whether an awkward word is chosen such that a pre-set presented incomplete sentence becomes a complete sentence, may be performed and a (11-2)th test score may be calculated. Here, the (11-2)th test item may include a second Hayling test. In this regard, the (11-2)th test process may include a process of performing the second Hayling test on the user and calculating the (11-2)th test score.

A difference between the Hayling test included in the eleventh test process according to the present embodiment and a general Hayling test may be as follows. The general Hayling test is a test in which a word is recalled to make a natural sentence or an unnatural sentence. However, in the Hayling test according to the present embodiment, a word suitable to a condition and an awkward (wrong word) are presented and one of the words is chosen. In the Hayling test according to the present embodiment, language control may be measured by reducing a time taken to retrieve a word. In the Hayling test according to the present embodiment, a test score is automatically calculated, and an automatic condition (choosing a natural word) response and a control condition (choosing an awkward word) response may be compared.

After the performing of the first test process through the eleventh test process is completed, the test apparatus 100 may a category classification process of classifying at least one test item from among a plurality of test items as one neurocognitive category from among a plurality of neurocognitive categories. The category classification process according to the present embodiment may be represented as a category classifying stage in the claims described below.

The test apparatus 100 may classify the second test item (language clue memory test) and the third test item (visual graphic memory test) as a first neurocognitive category for evaluating cognitive function decline regarding memory.

The test apparatus 100 may classify the eighth test item (face emotion test) as a second neurocognitive category for determining a neurocognitive disorder regarding social cognition. Here, the second neurocognitive category is newly added to a test for evaluating the cognitive function decline, according to the present embodiment.

The test apparatus 100 may classify the seventh test item (size weight test) and the ninth test item (paragraph reading test) as a third neurocognitive category for evaluating cognitive function decline regarding language.

The test apparatus 100 may classify the fifth test item (line angle perception test) and the sixth test item (color perception test) as a fourth neurocognitive category for evaluating cognitive function decline regarding perception.

The test apparatus 100 may classify the first test item (fluency test), the fourth test item (Stroop test), the tenth test item (visuospatial attention test), and the eleventh test item (Hayling test) as a fifth neurocognitive category for evaluating cognitive function decline regarding attention execution.

After the performing of the category classification process is completed, the test apparatus 100 may perform an evaluation process of evaluating whether there is cognitive function decline in a neurocognitive category, based on a test score of a test item classified as the neurocognitive category. In the present embodiment, the evaluation process may be represented as an evaluating stage in the claims described below.

The test apparatus 100 may perform a first evaluation process of evaluating whether there is cognitive function decline in the first neurocognitive category as the memory, based on the second test score (language clue memory test score) and the third test score (visual graphic memory test score).

The test apparatus 100 may perform a second evaluation process of evaluating whether there is cognitive function decline in the second neurocognitive category as the social cognition, based on the eighth test score (face emotion test score).

The test apparatus 100 may perform a third evaluation process of evaluating whether there is cognitive function decline in the third neurocognitive category as the language, based on the seventh test score (size weight test score) and the ninth test score (paragraph reading test score).

The test apparatus 100 may perform a fourth evaluation process of evaluating whether there is cognitive function decline in the fourth neurocognitive category as the perception, based on the fifth test score (line angle perception test score) and the sixth test score (color perception test score).

The test apparatus 100 may perform a fifth evaluation process of evaluating whether there is cognitive function decline in the fifth neurocognitive category as the attention execution, based on the first test score (fluency test score), the fourth test score (Stroop test score), the tenth test score (visuospatial attention test score), and the eleventh test score (Hayling test score).

In the present embodiment, the test apparatus 100 may be present independently in the form of a server, or a test function for evaluating cognitive function decline, provided by the test apparatus 100, may be realized in the form of an application and mounted on the user terminal 200.

The user terminal 200 may receive a test service for evaluating cognitive function decline by accessing a test application for evaluating cognitive function decline and/or a test website for evaluating cognitive function decline, provided by the test apparatus 100. Here, the user terminal 200 may include a terminal possessed by a user to be tested, i.e., a testee.

The user terminal 200 may include a communication terminal for performing functions of a computing device (not shown), and may include, in addition to a desktop computer 201, a smartphone 202, and a laptop computer 203 operated by the user, a tablet personal computer (PC), a smart television (TV), a mobile phone, a personal digital assistant (PDA), a media player, a micro-server, a global positioning system (GPS) device, an electronic book terminal, a digital broadcasting terminal, a navigation device, a kiosk, an MP3 player, a digital camera, a home appliance, and other mobile or non-mobile computing devices, but is not limited thereto. Also, the user terminal 200 may include a wearable device, such as a watch, glasses, a hair band, and a ring, including a communication function and a data processing function. The user terminal 200 is not limited by the above-described details, and a terminal capable of web browsing may be unlimitedly employed. The user terminal 200 may include a microphone for recording the voice of a user, and a GPS sensor for locating the user terminal 200.

The network 300 may connect the test apparatus 100 and the user terminal 200 to each other. Examples of the network 300 include wired networks, such as a local area network (LAN), a wide area network (WAN), a metropolitan area network (MAN), and an integrated service digital network (ISDN), and a wireless network, such as wireless LAN (WLAN), code-division multiple access (CDMA), and satellite communication, but the scope of the disclosure is not limited thereto. Also, the network 300 may transmit/receive information by using short-range communication and/or long-range communication. Here, the short-range communication may include Bluetooth, radio frequency identification (RFID), infrared data association (IrDA), ultra-wideband (UWB), ZigBee, or Wi-Fi technology, and the long-range communication may include code-division multiple access (CDMA), frequency-division multiple access (FDMA), time-division multiple access (TDMA), orthogonal frequency-division multiple access (OFDMA), or single carrier frequency-division multiple access (SC-FDMA) technology.

The network 300 may include a connection of network elements, such as a hub, a bridge, a router, or a switch. The network 300 may include one or more connected networks including a public network, such as the Internet, and a private network, such as a safe corporate private network, for example, a multi-network environment. An access to the network 300 may be provided through one or more wired or wireless access networks.

In addition, the network 300 may support controller area network (CAN) communication, vehicle-to-infrastructure (V2I) communication, vehicle-to-everything (V2X) communication, and wireless access in vehicular environment (WAVE) communication technology, and an Internet of things (loT) network, in which information is exchanged between distributed components, such as things, and processed, and/or 5th generation (5G) communication.

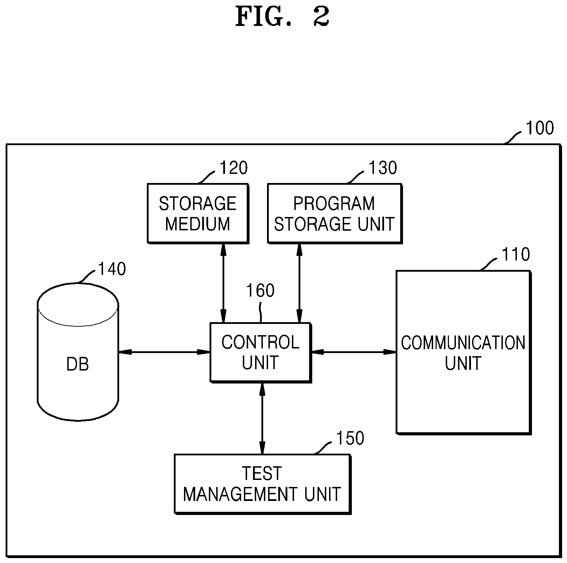

FIG. 2 is a block diagram for schematically describing a configuration of the test apparatus 100 for evaluating cognitive function decline, according to an embodiment. In the descriptions below, descriptions about details overlapping those of FIG. 1 are omitted. Referring to FIG. 2, the test apparatus 100 may include a communication unit 110, a storage medium 120, a program storage unit 130, a database 140, a test management unit 150, and a control unit 160.

The communication unit 110 may provide a communication interface required to provide, in the form of packet data, a transmission/reception signal between the test apparatus 100 and the user terminal 200, in association with the network 300. In addition, the communication unit 110 may transmit request information of the test management unit 150 to the user terminal 200, and receive response information of the user terminal 200. Here, a communication network is a medium performing a function of connecting the test apparatus 100 and the user terminal 200 to each other, and may include a path that provides an access path for the user terminal 200 to access the test apparatus 100 and then transmit/receive information. Also, the communication unit 110 may be a device including hardware and software required to transmit/receive a signal, such as a control signal or a data signal, through a wired/wireless connection with another network device.

The storage medium 120 performs a function of temporarily or permanently storing data processed by the control unit 160. Here, the storage medium 120 may include a magnetic storage medium or a flash storage medium, but the scope of the disclosure is not limited thereto. The storage medium 120 may include an internal memory and/or an external memory, and may include a volatile memory, such as a dynamic random-access memory (DRAM), a static RAM (SRAM), or a synchronous DRAM (SDRAM), a nonvolatile memory, such as a one-time programmable read-only memory (OTPROM), a programmable ROM (PROM), an erasable and programmable ROM (EPROM), an electrically EPROM (EEPROM), a mask ROM, a flash ROM, a NAND flash memory, or a NOR flash memory, a flash drive, such as a solid state drive (SSD), a compact flash (CF) card, a secure digital (SD) card, a micro-SD card, a mini-SD card, an extreme digital (XD) card, or a memory stick, or a storage medium, such as a hard disk drive (HDD).

The program storage unit 130 may be equipped with control software for executing a task of performing the test process of testing how many missions presented for each of a plurality of test items the user performs, and calculating a test score of each of the plurality of test items, a task of performing the category classification process of classifying at least one test item from among the plurality of test items as a neurocognitive category from among a plurality of neurocognitive categories, and a task of performing the evaluation process of evaluating whether there is cognitive function decline in a neurocognitive category, based on a test score of a test item classified as the neurocognitive category.

The database 140 may include a management database storing test information for evaluating cognitive function decline. For example, the management database may store information about the first test process through the eleventh test process, information about a first neurocognitive category classification process through a fifth neurocognitive category classification process, information about the first evaluation process through the fifth evaluation process, information about a process of evaluating Alzheimer's disease, information about a process of evaluating frontotemporal dementia, and information about a process of evaluating Parkinson's disease and dementia with Lewy bodies.

Also, the management database may store a correct answer corresponding to an instruction described below. For example, the management database may store a correct text corresponding to a (1-1)th instruction, a correct order corresponding to a (1-2)th instruction, a correct word corresponding to a (2-1-1)th instruction, a correct text corresponding to a (2-1-2)th instruction, a correct text corresponding to a (2-2-1)th instruction, a correct word corresponding to a (2-2-2)th instruction, a correct answer corresponding to a (3-1)th instruction, a correct answer corresponding to a (3-2)th instruction, correct answers corresponding to a (4-1)th instruction and a (4-2)th instruction, a correct line corresponding to a fifth instruction, a correct answer corresponding to a sixth instruction, correct answers corresponding to a (7-1)th instruction and a (7-2)th instruction, a correct answer corresponding to an eighth instruction, a correct number of word segments corresponding to a ninth instruction, correct answers corresponding to a (10-1)th instruction and a (10-2)th instruction, and correct answers corresponding to a (11-1)th instruction and a (11-2)th instruction.

Also, the management database may store a method of calculating a test score. According to the present embodiment, a series of inputs received from the user terminal 200 in response to an instruction may be compared with correct answers, and a test score may be calculated by counting the number of wrong answers compared to all correct answers. Here, the test score may decrease when the number of wrong answers increases, and the test score may increase when the number of wrong answers decreases.

In the present embodiment, a test score may vary depending on a result of counting a spent time described below. For example, the test score may further decrease when the result of counting the spent time exceeds a time limit.

Also, the management database may store a reference score for evaluating cognitive function decline. A process of calculating a reference score may be as follows.

During a first process, the first through eleventh test processes may be performed on a normal group. Here, the normal group is a group of normal people without cognitive function decline, and may be differently classified according to gender/education/age. The first through eleventh test scores of the normal group may be calculated as results of performing the first through eleventh test processes on the normal group.

During a second process, first through eleventh average values and first through eleventh standard deviations may be calculated for the first through eleventh test scores of the normal group.

During a third process, at least one of the first through eleventh test items of the normal group may be classified as one of the first neurocognitive category through the fifth neurocognitive category. The second test item and third test item of the normal group may be classified as the first neurocognitive category, the eighth test item of the normal group may be classified as the second neurocognitive category, the seventh test item and the ninth test item of the normal group may be classified as the third neurocognitive category, the fifth test item and the sixth test item of the normal group may be classified as the fourth neurocognitive category, and the first test item, the fourth test item, the tenth test item, and the eleventh test item of the normal group may be classified as the fifth neurocognitive category.

During a fourth process, first through eleventh Z-scores may be calculated by using the first through eleventh average values and the first through eleventh standard deviations for the first through eleventh test scores. Here, a Z-score may be a score for determining how far a test score is from an average value in units of standard deviation.

According to the present embodiment, when the Z-score is equal to or less than the first standard deviation (for example, -1.5 SD), it may be evaluated that there is cognitive function decline in a neurocognitive category including a corresponding test item. In this regard, the reference score may be the test score of the normal group, in which the Z-score is the first standard deviation (for example, -1.5 SD). In the present embodiment, the reference score may include a first reference score through an eleventh reference score. In the present embodiment, the Z-score may also include the first Z-score through the eleventh Z-score.

According to a selective embodiment, the reference score may be represented as a criterion cut-off score. Here, the criterion cut-off score may be the test score of the normal group, in which the Z-score is the first standard deviation (for example, -1.5 SD). In the present embodiment, the criterion cut-off score may include a first criterion cut-off score through an eleventh criterion cut-off score.

In the present embodiment, the reference score may be periodically updated. The reference score may be updated in real time whenever the user executes a test application for evaluating cognitive function decline or accesses a test website for evaluating cognitive function decline through the user terminal 200, to perform a test for evaluating cognitive function decline.

Also, the database 140 may store a user database storing information about the user who is to receive a test service for evaluating cognitive function decline. The information about the user may include unique information about the user, including a name, affiliation, personal data, gender, age, education, contact number, email address, address, and image of the user, information about authentication (login) of the user, such as an identification (ID) (or an email address) and a password, and access-related information, such as an access country, an access location, information about a device used for access, and an accessed network environment.

Also, the user database may store the information about the user, information received by the user who accessed the test application for evaluating cognitive function decline or the test website for evaluating cognitive function decline, and/or a category history, information about a configuration set by the user, information about resource usage used by the user, and charging and payment information corresponding to the resource usage of the user.

The test management unit 150 may perform the test processes of testing how many missions presented for each of the plurality of test items the user performs, and calculating the test score for each of the plurality of test items.

The test management unit 150 may perform the category classification process of classifying at least one test item from among the plurality of test items as a neurocognitive category from among the plurality of neurocognitive categories, after the performing of the test processes is completed.

After the performing of the category classification process is completed, the test management unit 150 may perform the evaluation process of evaluating whether there is cognitive function decline in a neurocognitive category, based on a test score of a test item classified as the neurocognitive category.

After the performing of the evaluation process is completed, the test management unit 150 may transmit an evaluation result to the user terminal 200 and/or a terminal (not shown) of a practitioner (for example, a doctor).

The control unit 160 is a type of a central processing unit, and may control all operations of the test apparatus 100 by driving the control software mounted on the program storage unit 130. The control unit 160 may include any type of devices capable of processing data, such as a processor. Here, the "processor" may denote, for example, a data processing device embedded in hardware, which includes a physically structured circuit to perform a function expressed in code or instruction included in a program. Examples of the data processing device embedded in hardware may include processing devices, such as a microprocessor, a central processing unit (CPU), a processor core, a multiprocessor, an application-specific integrated circuit (ASIC), and a field programmable gate array (FPGA), but the scope of the disclosure is not limited thereto.

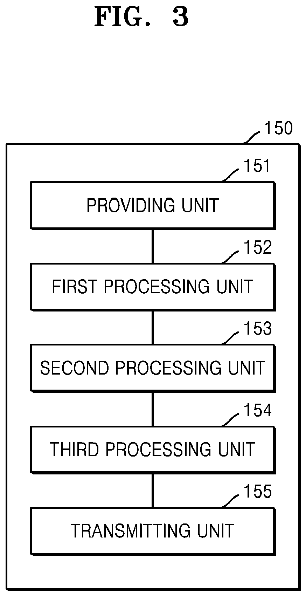

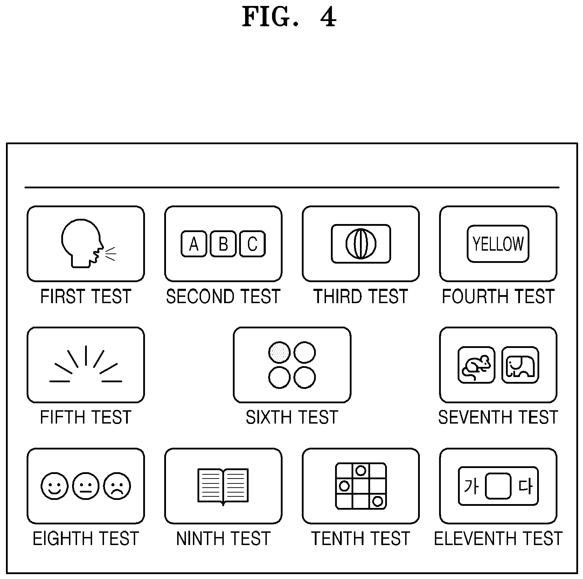

FIG. 3 is a block diagram for schematically describing a configuration of the test management unit 150 in the test apparatus 100 of FIG. 2 for evaluating cognitive function decline, and FIGS. 4 through 15K illustrate examples of screens provided on the user terminal 200 to execute a test for evaluating cognitive function decline, according to an embodiment. In the descriptions below, descriptions about details overlapping those of FIGS. 1 and 2 are omitted. Referring to FIGS. 3 through 15, the test management unit 150 according to the present embodiment may include a providing unit 151, a first processing unit 152, a second processing unit 153, a third processing unit 154, and a transmitting unit 155.

The providing unit 151 may provide various types of information for a test to the user terminal 200 that accessed the test application for evaluating cognitive function decline and/or the test website for evaluating cognitive function decline. Hereinafter, the diagrams in FIGS. 4 through 15 may be screens output on the user terminal 200 through processing by the providing unit 151.

FIG. 4 illustrates an example of a first screen provided to the user terminal 200 by the providing unit 151 so as to perform a test for evaluating cognitive function decline. Referring to FIG. 4, the fist screen may include an icon and a text representing a test. According to an embodiment, the providing unit 151 may provide, to the user terminal 200, an icon and a text (first test) indicating a first test, with respect to the first test process. Here, the first test indicated in the text is an abbreviation of the first test process and may be simply and plainly provided considering aged people. In such a manner, the providing unit 151 may provide, to the user terminal 200, icons and texts indicating a second test through an eleventh test, with respect to the second test process through the eleventh test process.

Pieces of data processed by the first processing unit 152 through the third processing unit 154 may be provided to the user terminal 200 by the providing unit 151. In particular, a series of data provided by the first processing unit 152 is originally provided by the providing unit 151, but for convenience of descriptions, it is assumed that the series of data is provided by the first processing unit 152.

The first processing unit 152 may perform the first test process including the (1-1)th test process and the (1-2)th test process. According to the present embodiment, the first processing unit 152 may perform the first test process a plurality of times, excluding a practice test.

When the (1-1)th test process is performed, the first processing unit 152 may provide, to the user terminal 200, the (1-1)th instruction, in which it is instructed to utter at least one word corresponding to a pre-set presented category within the first time limit.

The first processing unit 152 may receive an utterance result of the at least one word corresponding to the pre-set presented category in response to the (1-1)th instruction, and convert the utterance result into an utterance text.

The first processing unit 152 may calculate the (1-1)th test score by comparing the utterance text with a correct text. According to the present embodiment, the (1-1)th test score may be calculated to be high when there are many utterance texts determined to be correct answers compared to the total number of times the (1-1)th test processes have been performed.

According to the present embodiment, before the (1-1)th test process is performed, the first processing unit 152 may provide, in a text, a method of performing the (1-1)th test process to the user terminal 200, and perform a practice test for the (1-1)th test process.

The first processing unit 152 may perform the (1-2)th test process after the performing of the (1-1)th test process is completed.

When the (1-2)th test process is performed, the first processing unit 152 may provide, to the user terminal 200, the (1-2)th instruction, in which it is instructed to draw at least one design by connecting at least two of a plurality of figures within the second time limit.

The first processing unit 152 may receive an order of drawing and connecting at least two of the plurality of figures, in response to the (1-2)th instruction from the user terminal 200, and store the order.

The first processing unit 152 may calculate the (1-2)th test score by comparing the order of drawing and connecting with a correct order. According to the present embodiment, the (1-2)th test score may be calculated to be high when there are many drawing connection orders determined to be correct answers compared to the total number of times the (1-2)th test processes have been performed.

According to the present embodiment, before the (1-2)th test process is performed, the first processing unit 152 may provide, in a text, a method of performing the (1-2)th test process to the user terminal 200, and perform a practice test for the (1-2)th test process.

According to a selective embodiment, the first processing unit 152 may calculate the first test score by adding the (1-1)th test score and the (1-2)th test score. Alternatively, the first processing unit 152 may not add the (1-1)th test score and the (1-2)th test score, but may use the (1-1)th test score and the (1-2)th test score each to evaluate cognitive function decline. According to the present embodiment, it may be evaluated that fluency is good when the first test score is higher than the first criterion cut-off score, and is declined when the first test score is lower than the first criterion cut-off score.

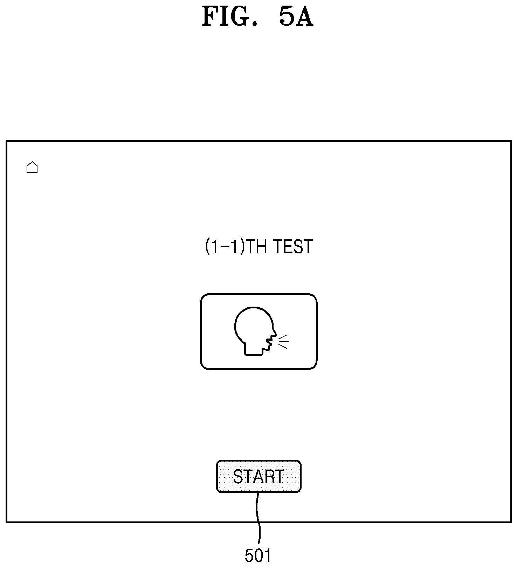

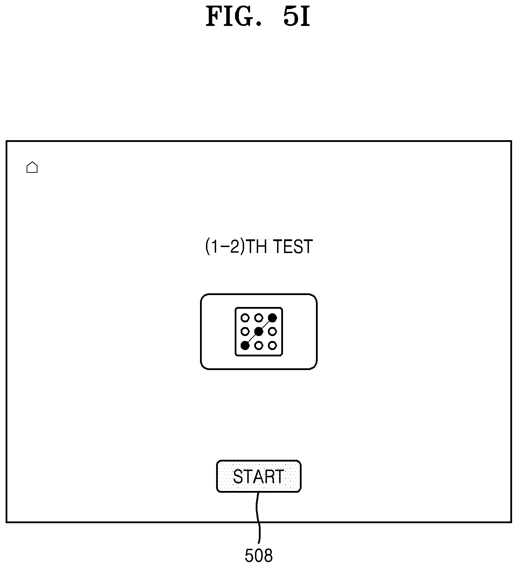

FIGS. 5A through 5S illustrate examples of screens provided to the user terminal 200 to perform the first test process. In the present embodiment, FIGS. 5A through 5H illustrate examples of screens for describing the (1-1)th test process, and FIGS. 5I through 5S illustrate examples of screens for describing the (1-2)th test process.

FIG. 5A illustrates a start screen of the (1-1)th test process. When an input of a start button 501 is received, a next screen may be displayed.

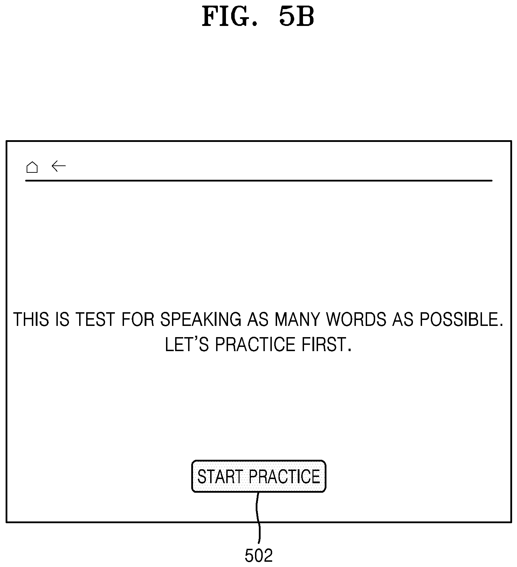

FIG. 5B illustrates a screen in which a method of performing the (1-1)th test process is provided in a text and a start of a practice test process ((1-1)th practice test process) for the (1-1)th test process is notified. When a practice start button 502 is input, a next screen may be displayed.

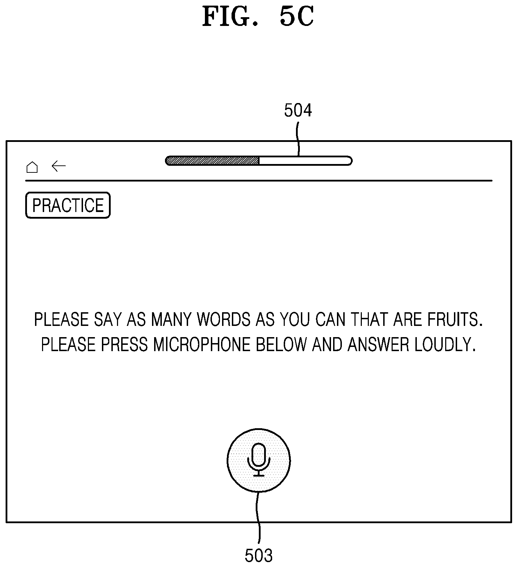

FIG. 5C illustrates a screen for performing the (1-1)th practice test process. The user may check a (1-1)th practice instruction, input a microphone button 503, and utter words while looking at a progress bar 504. In the present embodiment, the progress bar 504 may perform at least one of a function of counting a time limit and a function of illustrating a work progress degree.

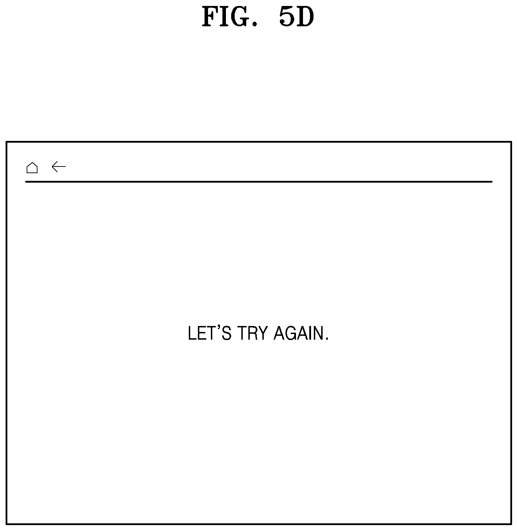

FIG. 5D illustrates a screen indicating a rerun of the (1-1)th practice test process. The (1-1)th practice test process may be rerun when the user has not input the microphone button 503, the number of uttered words is less than a pre-set number (for example, 3), or uttered voice volume is low.

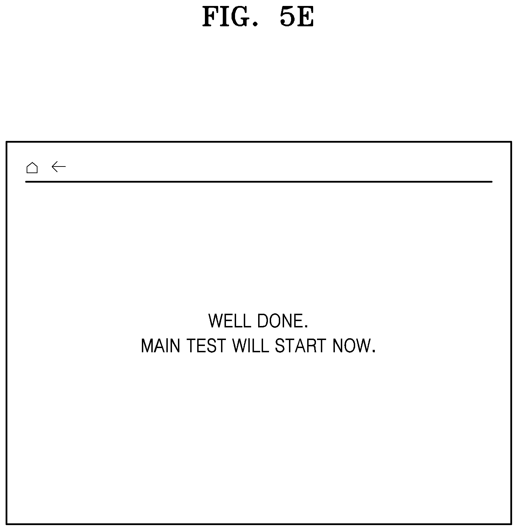

FIG. 5E illustrates a screen indicating completion of the performing of the (1-1)th practice test process, and performing of a main test.

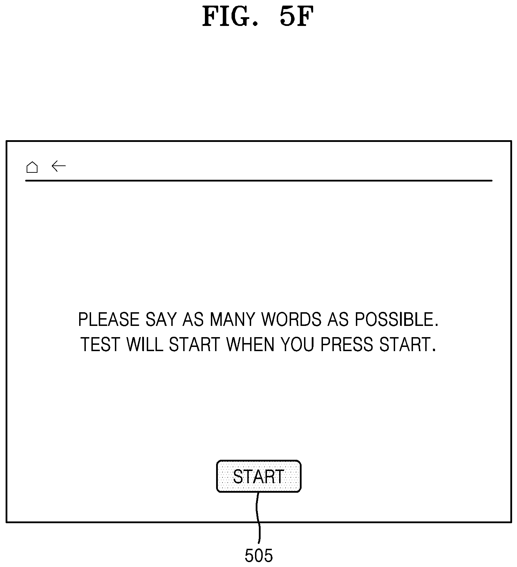

FIG. 5F illustrates a screen indicating the (1-1)th instruction and a start of the (1-1)th test process. When a start button 505 is input, a next screen may be displayed.

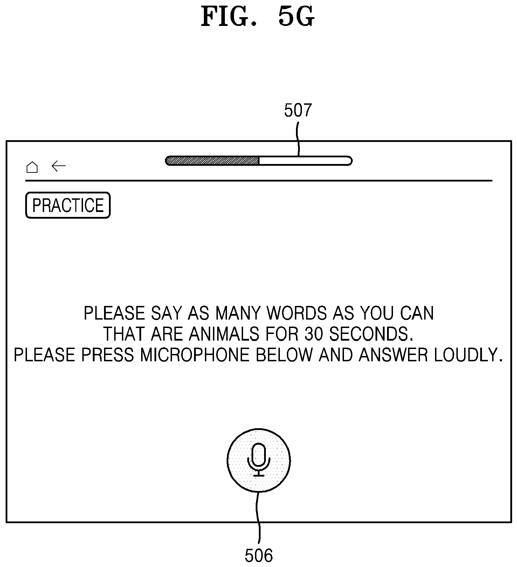

FIG. 5G illustrates a screen for performing the (1-1)th test process. The user may check the (1-1)th instruction, input a microphone button 506, and utter words while looking at a progress bar 507, in response to the (1-1)th instruction. The recorded voice may be transmitted from the user terminal 200 to the test apparatus 100. The control unit 160 of the test apparatus 100 may convert the voice data to a text using a speech recognition algorithm and count the number of words included in the text.

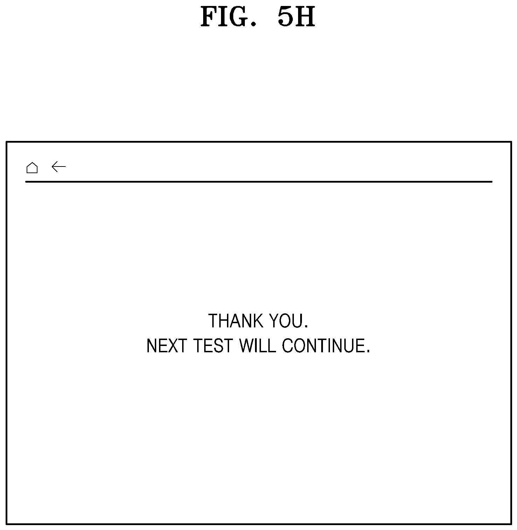

FIG. 5H illustrates a screen indicating completion of the performing of the (1-1)th test process and a start of a next test process.

FIG. 5I illustrates a start screen of the (1-2)th test process. When an input of a start button 508 is received, a next screen may be displayed.

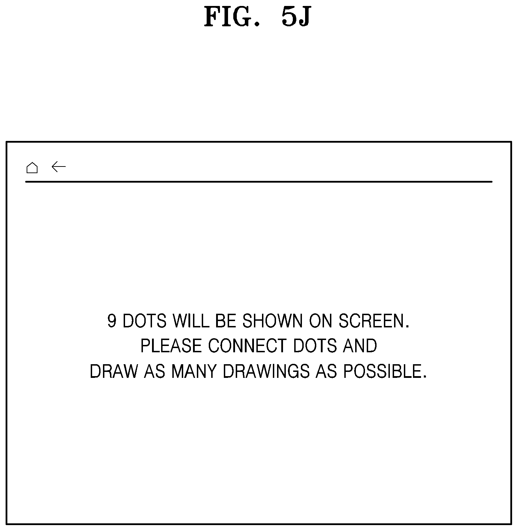

FIG. 5J illustrates a screen providing a method of performing the (1-2)th test process, in a text. For example, the method of performing the (1-2)th test process may include a text indicating to connect dots shown on a screen and draw as many drawings as possible.

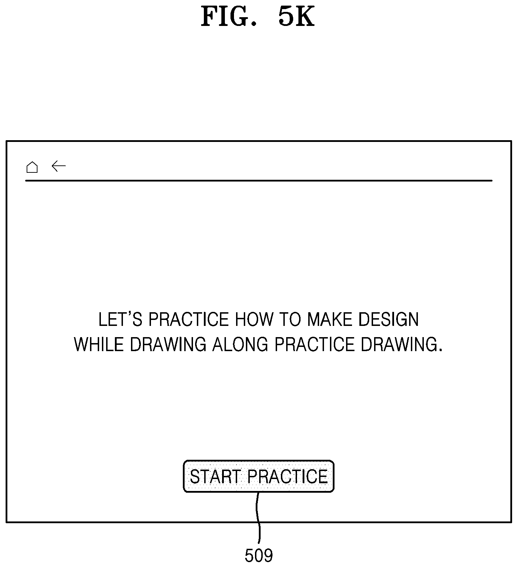

FIG. 5K illustrates a screen indicating a start of a practice test process ((1-2)th practice test process) for the (1-2)th test process. When a practice start button 509 is input, a next screen may be displayed.

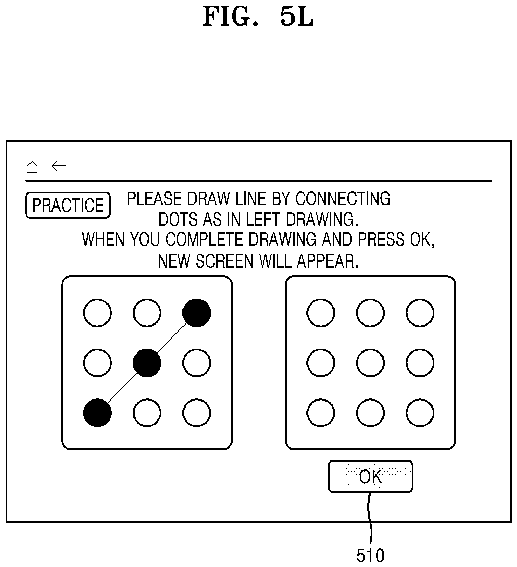

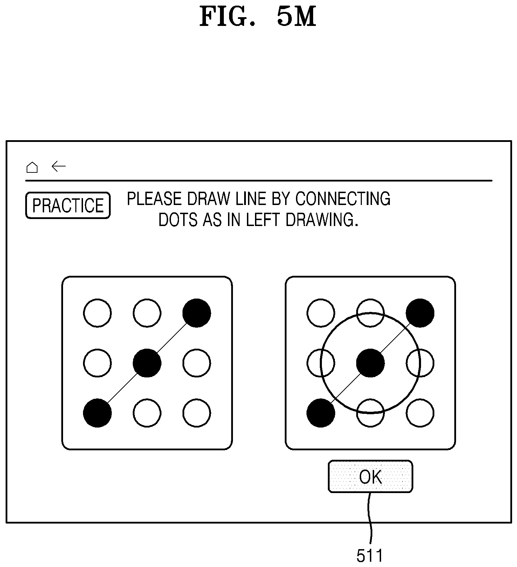

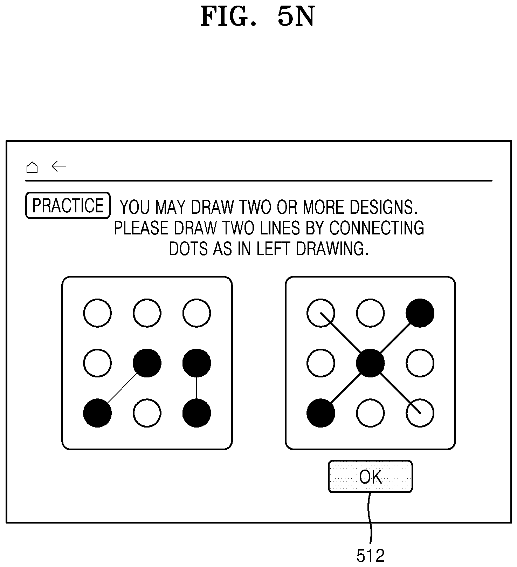

FIGS. 5L, 5M, and 5N illustrate screens for performing the (1-2)th practice test process. The user may check a (1-2)th practice instruction and draw, on a right region, a same design as a left region. When OK buttons 510, 511, and 512 are input after the drawing of the design is completed, a next screen may be displayed. FIGS. 5L, 5M, and 5N may further include a progress bar (not shown).

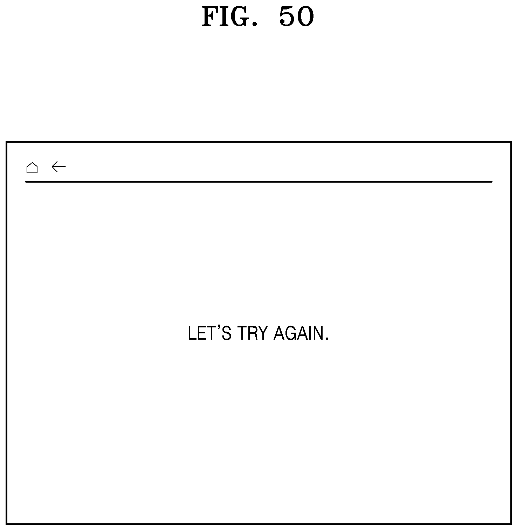

FIG. 5O illustrates a screen indicating a rerun of the (1-2)th practice test process. The (1-2)th practice test process may be rerun when the drawing of the design is not started within a time limit or a result of performing the (1-2)th practice test process is a wrong answer.

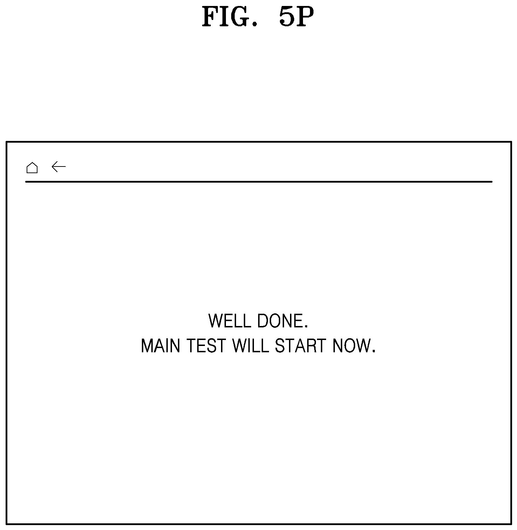

FIG. 5P illustrates a screen indicating completion of the (1-2)th practice test process, and performing of a main test.

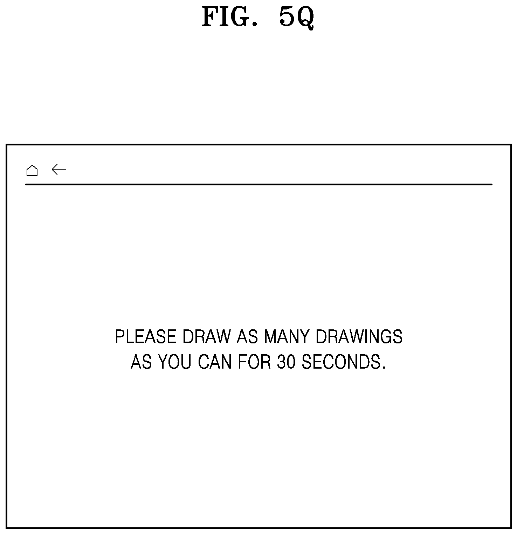

FIG. 5Q illustrates a screen indicating a time limit applied when the (1-2)th test process is performed.

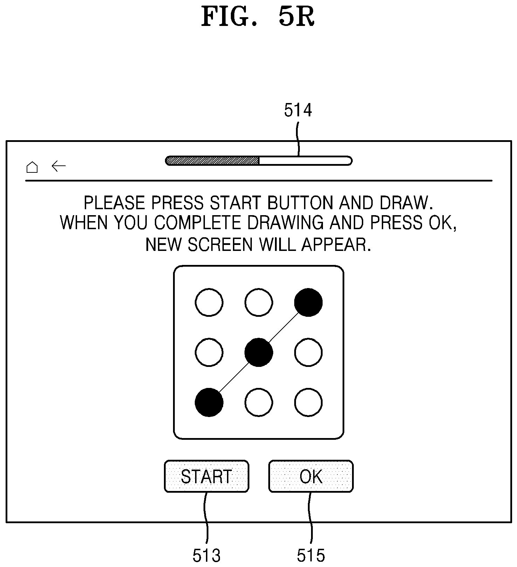

FIG. 5R illustrates a screen for performing the (1-2)th test process. The user may check the (1-2)th instruction and then press a start button 513 and draw, on a right region, a same design as a left region, while looking at a progress bar 514. When an OK button 515 is input after the drawing of the design is completed, a next screen may be displayed.

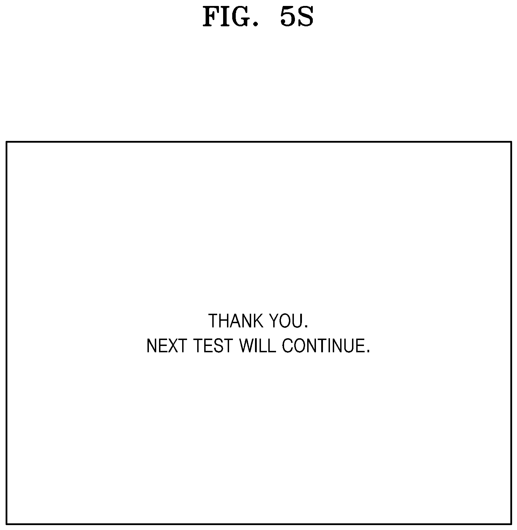

FIG. 5S illustrates a screen indicating completion of the performing of the (1-2)th test process and a start of a next test process.

Referring back to FIG. 3, the first processing unit 152 may perform the second test process including the (2-1)th test process and the (2-2)th test process. According to the present embodiment, the first processing unit 152 may perform the second test process a plurality of times.

When the (2-1)th test process is performed, the first processing unit 152 may illustrate a plurality of presented words and provide, to the user terminal 200, the (2-1-1)th instruction, in which it is instructed to choose a word corresponding to a pre-set category clue from among the plurality of presented words.

The first processing unit 152 may receive, from the user terminal 200, a word choice result of choosing one word from among the plurality of presented words in response to the (2-1-1)th instruction.

The first processing unit 152 may calculate the (2-1-1)th test score by comparing the word choice result with a correct word. According to the present embodiment, the (2-1-1)th test score may be calculated to be high when there are many word choice results determined to be correct answers compared to the total number of times (2-1-1)th test processes have been performed.

The first processing unit 152 may provide, to the user terminal 200, the (2-1-2)th instruction, in which it is instructed to recall and utter a word belonging to a pre-set category clue from among the plurality of presented words shown previously, without providing an illustration of the plurality of presented words.

The first processing unit 152 may receive, from the user terminal 200, an utterance result of a word belonging to the pre-set category clue, in response to the (2-1-2)th instruction.

The first processing unit 152 may convert the utterance result into an utterance text, and calculate the (2-1-2)th test score by comparing the utterance text with a correct text. According to the present embodiment, the (2-1-2)th test score may be calculated to be high when there are many utterance texts determined to be correct answers compared to the total number of times (2-1-2)th test processes have been performed.

According to the present embodiment, before the (2-1)th test process is performed, the first processing unit 152 may provide, in a text, a method of performing the (2-1)th test process to the user terminal 200.

The first processing unit 152 may perform the (2-2)th test process when a pre-set time has elapsed after the performing of the (2-1)th test process is completed.

When the (2-2)th test process is performed, the first processing unit 152 may provide, to the user terminal 200, the (2-2-1)th instruction, in which it is instructed to recall and utter a presented word corresponding to a category clue from among the plurality of presented words shown during a (2-1)th testing stage.

The first processing unit 152 may receive, from the user terminal 200, an utterance result of the presented word corresponding to the category clue from among the plurality of presented words shown during the (2-1)th testing stage, in response to the (2-2-1)th instruction.

The first processing unit 152 may convert the utterance result into an utterance text, and calculate the (2-2-1)th test score by comparing the utterance text with a correct text. According to the present embodiment, the (2-2-1)th test score may be calculated to be high when there are many utterance texts determined to be correct answers compared to the total number of times (2-2-1)th test processes have been performed.

The first processing unit 152 may provide, to the user terminal 200, the (2-2-2)th instruction, in which it is instructed to choose whether a pre-set presented word is a word shown in a previous test, together with the pre-set presented word.

The first processing unit 152 may calculate the (2-2-2)th test score by comparing a result of choosing whether the pre-set presented word is a word shown in the previous test, in response to the (2-2-2)th instruction, with a correct word. According to the present embodiment, the (2-2-2)th test score may be calculated to be high when there are many choice results determined to be correct answers compared to the total number of times (2-2-2)th test processes have been performed.

According to the present embodiment, before the (2-2)th test process is performed, the first processing unit 152 may provide, in a text, a method of performing the (2-2)th test process to the user terminal 200.

According to a selective embodiment, the first processing unit 152 may calculate the second test score by adding at least one of the (2-1-1)th test score, the (2-1-2)th test score, the (2-2-1)th test score, and the (2-2-2)th test score. Alternatively, the first processing unit 152 may use each of the (2-1-1)th test score, the (2-1-2)th test score, the (2-2-1)th test score, and the (2-2-2)th test score to evaluate cognitive function decline, without adding the (2-1-1)th test score, the (2-1-2)th test score, the (2-2-1)th test score, and the (2-2-2)th test score. According to the present embodiment, it may be evaluated that language memory is good when the second test score is higher than the second criterion cut-off score, and is declined when the second test score is lower than the second criterion cut-off score.

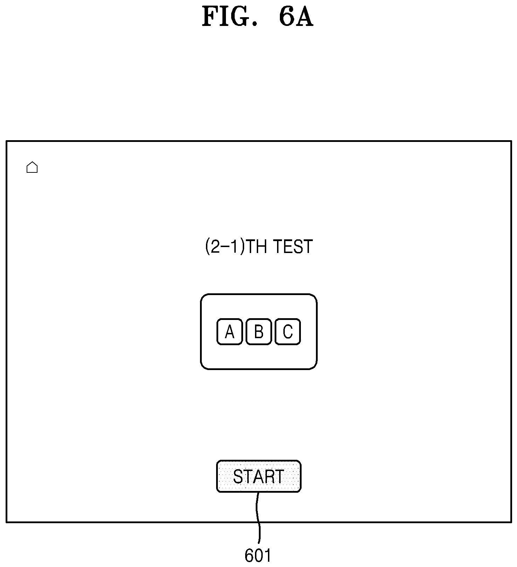

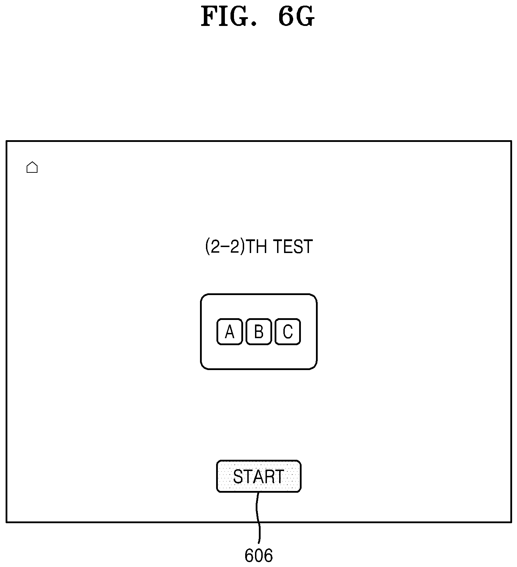

FIGS. 6A through 6M illustrate examples of screens provided to the user terminal 200 to perform the second test process. FIGS. 6A through 6F illustrate examples of screens for describing the (2-1)th test process and FIGS. 6G through 6M illustrate examples of screens for describing the (2-2)th test process.

FIG. 6A illustrates a start screen of the (2-1)th test process. When an input of a start button 601 is received, a next screen may be displayed.

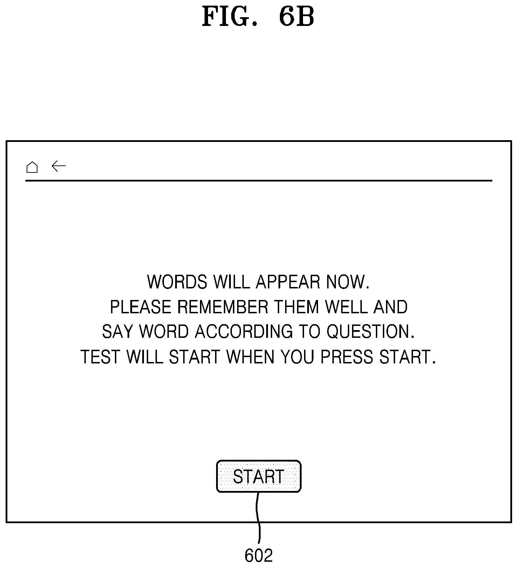

FIG. 6B illustrates a screen providing, in a text, a method of performing the (2-1)th test process, and indicating a start of the (2-1)th test process. When a start button 602 is input, a next screen may be displayed.

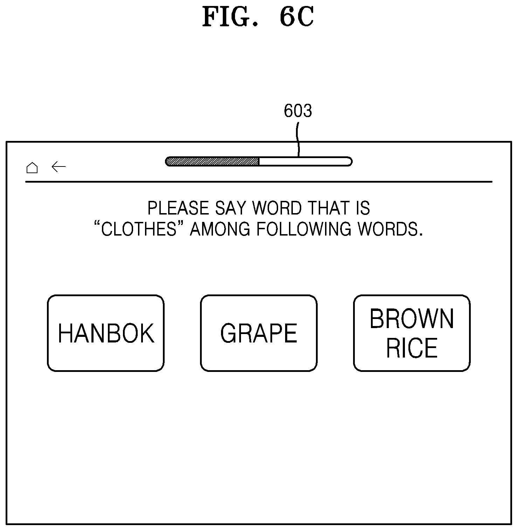

FIG. 6C illustrates a screen for performing a part of the (2-1)th test process. The user may check the (2-1-1)th instruction, and then choose a word from among a plurality of presented words while looking at a progress bar 603.

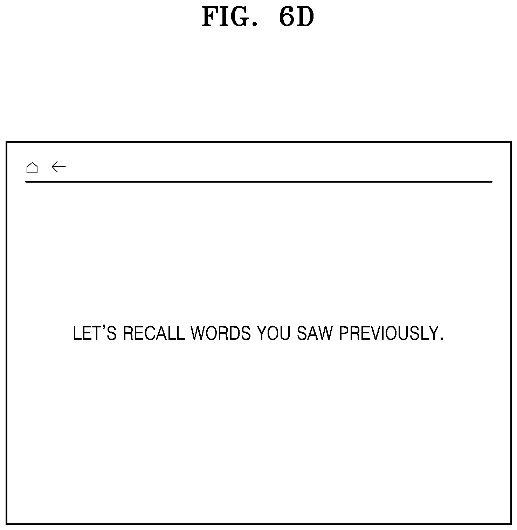

FIG. 6D illustrates a screen recommending to recall the plurality of presented words shown in FIG. 6C.

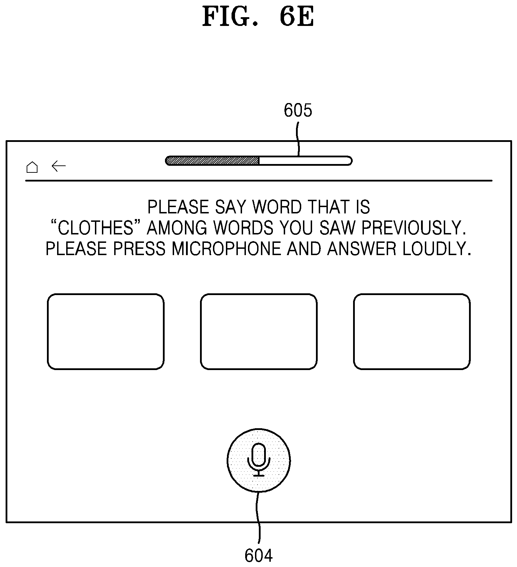

FIG. 6E illustrates a screen for performing a remaining part of the (2-1)th test process. The user may check the (2-1-2)th instruction, input a microphone button 604, and recall and utter a word corresponding to a pre-set category clue from among the plurality of presented words shown previously while looking at a progress bar 605.

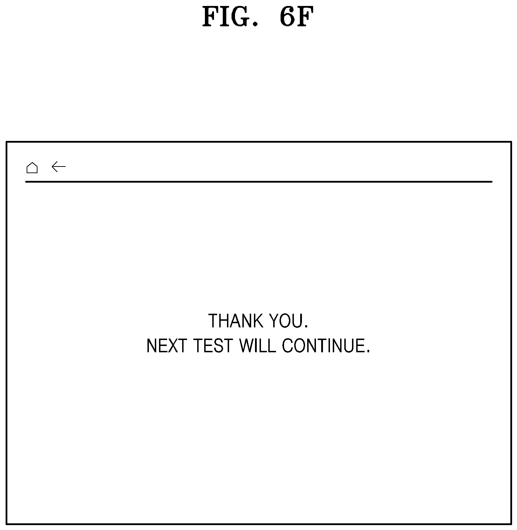

FIG. 6F illustrates a screen indicating completion of the performing of the (2-1)th test process and a start of a next test process.

FIG. 5G illustrates a start screen of the (2-2)th test process. When an input of a start button 606 is received, a next screen may be displayed. Here, the start screen of the (2-2)th test process may be provided to the user terminal 200 after the performing of the seventh test process is completed.

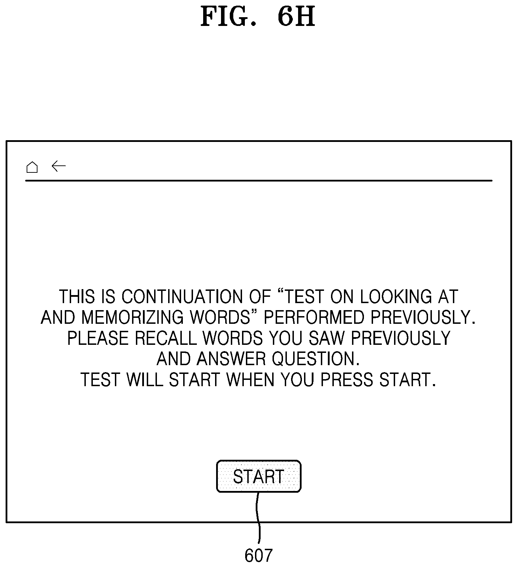

FIG. 6H illustrates a screen providing, in a text, a method of performing a part of the (2-2)th test process, and indicating a start of the part of the (2-2)th test process. When a start button 607 is input, a next screen may be displayed.

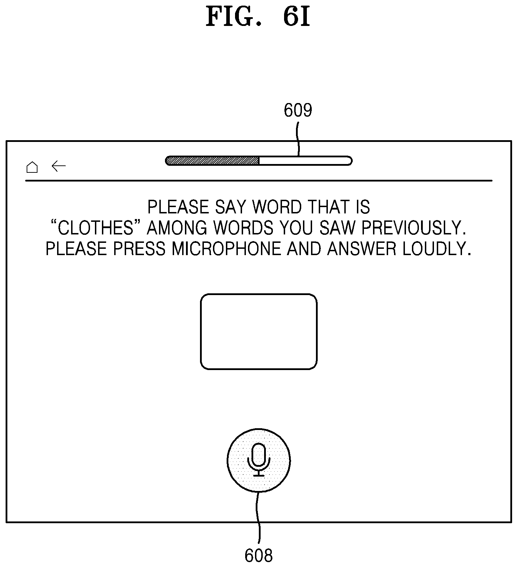

FIG. 6I illustrates a screen for performing the part of the (2-2)th test process. The user may check the (2-2-1)th instruction, input a microphone button 608, and recall and utter a word corresponding to a pre-set category clue from among the plurality of presented words shown previously while looking at a progress bar 609.

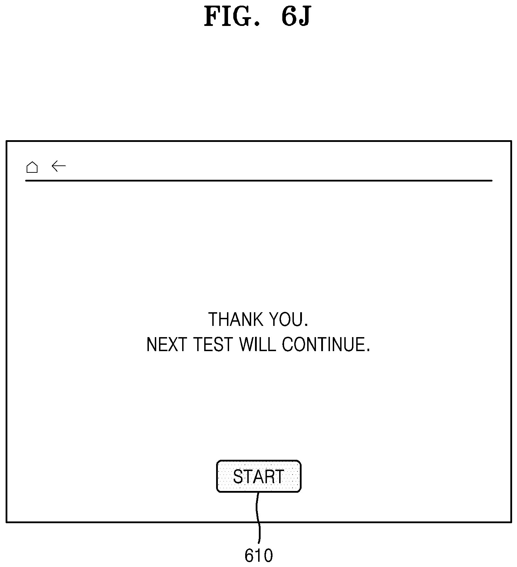

FIG. 6J illustrates a screen indicating performing of a remaining part of the (2-2)th test process after the performing of the part of the (2-2)th test process is completed. When a start button 610 is input, a next screen may be displayed.

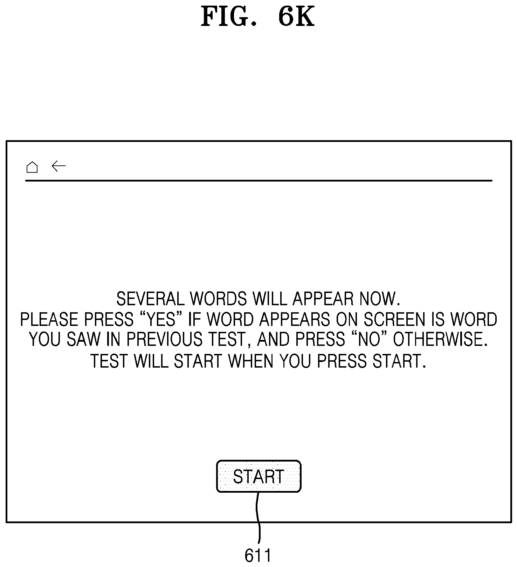

FIG. 6K illustrates a screen providing, in a text, a method of performing the remaining part of the (2-2)th test process, and indicating a start of the remaining part of the (2-2)th test process. When a start button 611 is input, a next screen may be displayed.

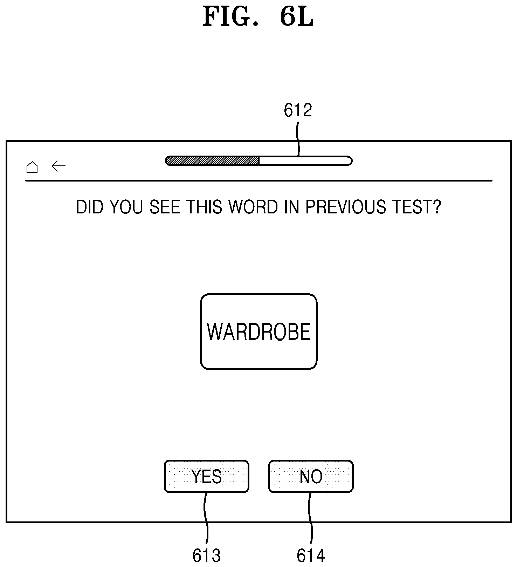

FIG. 6L illustrates a screen for performing the remaining part of the (2-2)th test process. The user may check the (2-2-2)th instruction and choose one of a Yes button 613 and a No button 614 while looking at a progress bar 612.

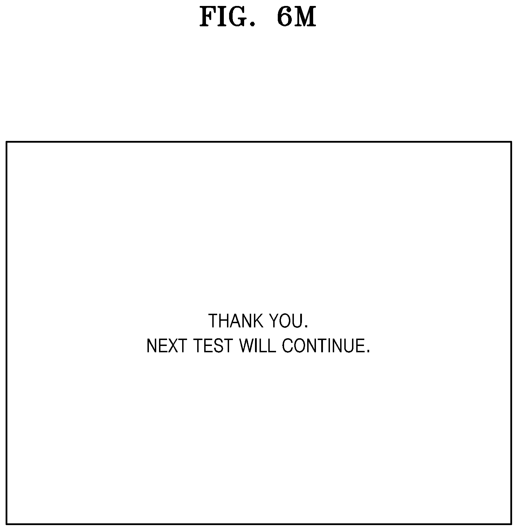

FIG. 6M illustrates a screen indicating completion of the performing of the (2-2)th test process and a start of a next test process.

Referring back to FIG. 3, the first processing unit 152 may perform the third test process including the (3-1)th test process and the (3-2)th test process. According to the present embodiment, the first processing unit 152 may perform the third test process a plurality of times.

When the (3-1)th test process is performed, the first processing unit 152 may illustrate a pre-set first presented image and provide, to the user terminal 200, the (3-1)th instruction, in which it is instructed to choose whether the number of colors in the pre-set first presented image is equal to or greater than a pre-set number.

The first processing unit 152 may receive, from the user terminal 200, a result of choosing whether the number of colors in the pre-set first presented image is equal to or greater than the pre-set number in response to the (3-1)th instruction, and calculate the (3-1)th test score by comparing the result with a correct answer. According to the present embodiment, the (3-1)th test score may be calculated to be high when there are many results determined to be correct answers compared to the total number of times the (3-1)th test processes have been performed.

According to the present embodiment, before the (3-1)th test process is performed, the first processing unit 152 may provide, in a text, a method of performing the (3-1)th test process to the user terminal 200.

The first processing unit 152 may perform the (3-2)th test process when a pre-set time has elapsed after the performing of the (3-1)th test process is completed.

When the (3-2)th test process is performed, the first processing unit 152 may illustrate a pre-set second presented image and provide, to the user terminal 200, the (3-2)th instruction, in which it is instructed to choose whether the pre-set second presented image is the same as the pre-set first presented image shown previously.

The first processing unit 152 may receive, from the user terminal 200, a result of choosing whether the pre-set second presented image is the same as the pre-set first presented image shown previously, in response to the (3-2)th instruction, and compare the result with a correct answer.

The first processing unit 152 may count a first spent time from when the (3-2)th instruction is provided to when it is chosen whether the pre-set second presented image is the same as the pre-set first presented image shown previously.

The first processing unit 152 may calculate the (3-2)th test score by reflecting a result of the comparing and a result of the counting. According to the present embodiment, the (3-2)th test score may be calculated differently according to the results of choosing determined to be correct answers compared to the total number of times the (3-2)th test processes have been performed, and the result of counting. Even when there are many results of choosing, determined to be correct answers, compared to the total number of times the (3-2)th test processes have been performed, the (3-2)th test score may be low when the result of counting the first spent time is high. Alternatively, even when there are few results of choosing, determined to be correct answers, compared to the total number of times the (3-2)th test processes have been performed, the (3-2)th test score may be high when the result of counting the first spent time is low.

According to the present embodiment, before the (3-2)th test process is performed, the first processing unit 152 may provide, in a text, a method of performing the (3-2)th test process to the user terminal 200.

According to a selective embodiment, the first processing unit 152 may calculate the third test score by adding the (3-1)th test score and the (3-2)th test score. Alternatively, the first processing unit 152 may not add the (3-1)th test score and the (3-2)th test score, but may use the (3-1)th test score and the (3-2)th test score each to evaluate cognitive function decline. According to the present embodiment, it may be evaluated that visual perception memory is good when the third test score is higher than the third criterion cut-off score, and is declined when the third test score is lower than the third criterion cut-off score.

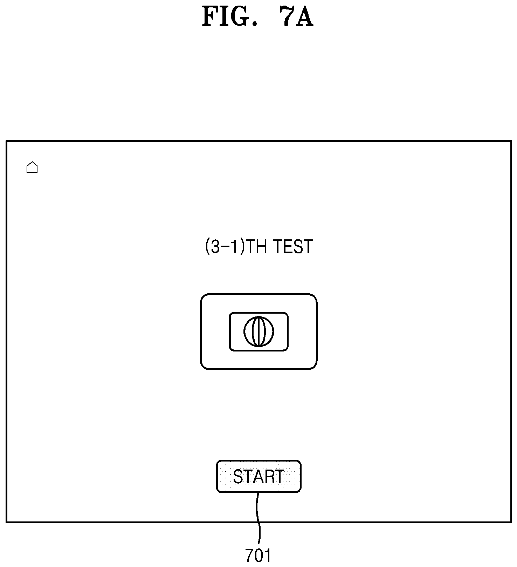

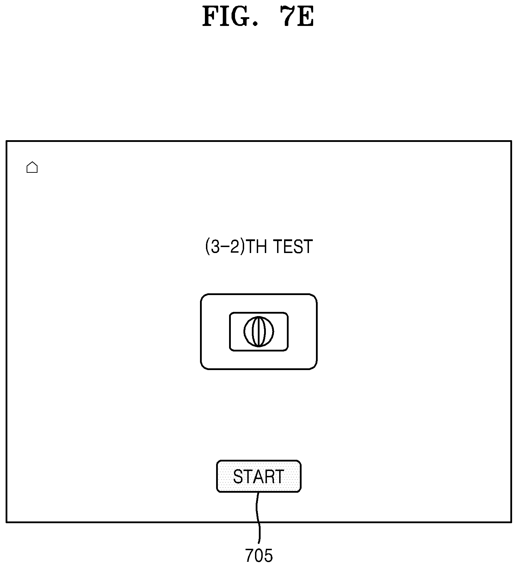

FIGS. 7A through 7H illustrate examples of screens provided to the user terminal 200 to perform the third test process. FIGS. 7A through 7D illustrate examples of screens for describing the (3-1)th test process and FIGS. 7E through 7H illustrate examples of screens for describing the (3-2)th test process.

FIG. 7A illustrates a start screen of the (3-1)th test process. When an input of a start button 701 is received, a next screen may be displayed.

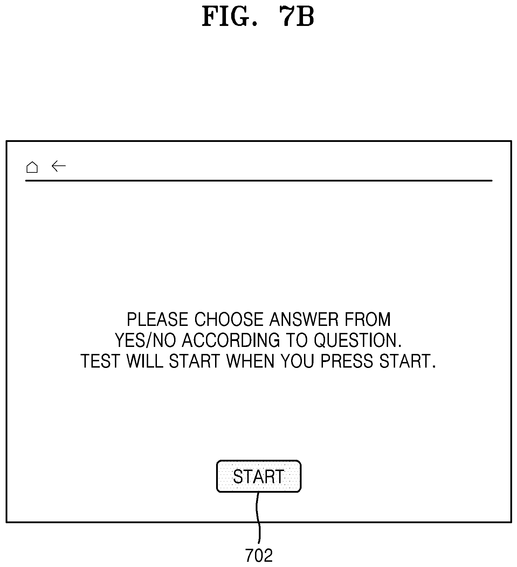

FIG. 7B illustrates a screen providing, in a text, a method of performing the (3-1)th test process, and indicating a start of the (3-1)th test process. When a start button 702 is input, a next screen may be displayed.

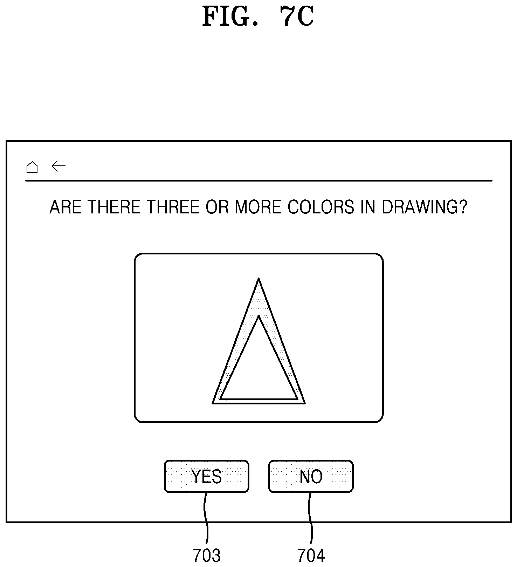

FIG. 7C illustrates a screen for performing the (3-1)th test process. The user may check the (3-1)th instruction and choose one of a Yes button 703 and a No button 704 in response to whether the number of colors in the first presented image is equal to or greater than a pre-set number. Here, a progress bar (not shown) performing at least one of a function of counting a time limit and a function of illustrating a work progress degree may be further provided.

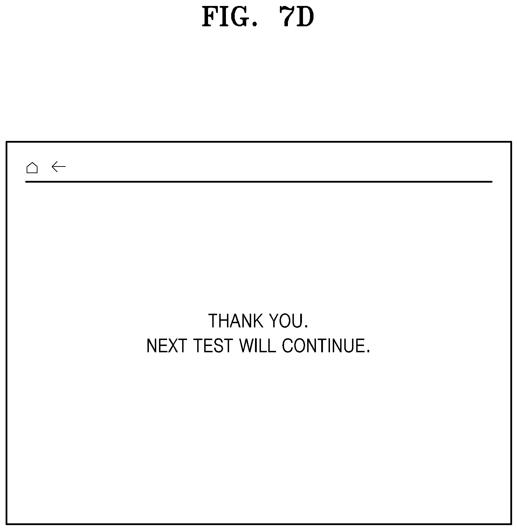

FIG. 7D illustrates a screen indicating completion of the performing of the (3-1)th test process and a start of a next test process.

FIG. 7E illustrates a start screen of the (3-2)th test process. When an input of a start button 705 is received, a next screen may be displayed. Here, the start screen of the (3-2)th test process may be provided to the user terminal 200 after the performing of the (2-2)th test process is completed.

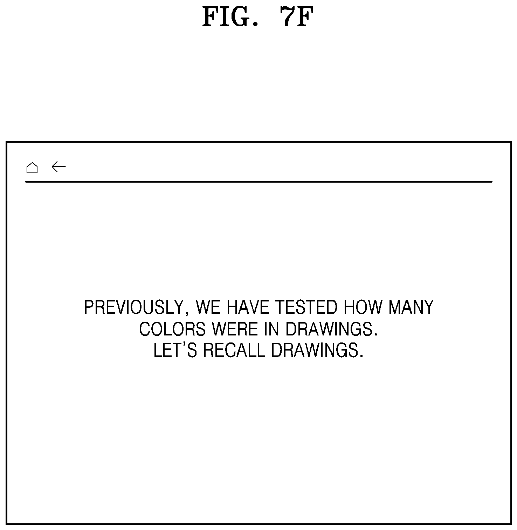

FIG. 7F illustrates a screen recommending to recall the first presented image provided while the (3-1)th test process was performed.

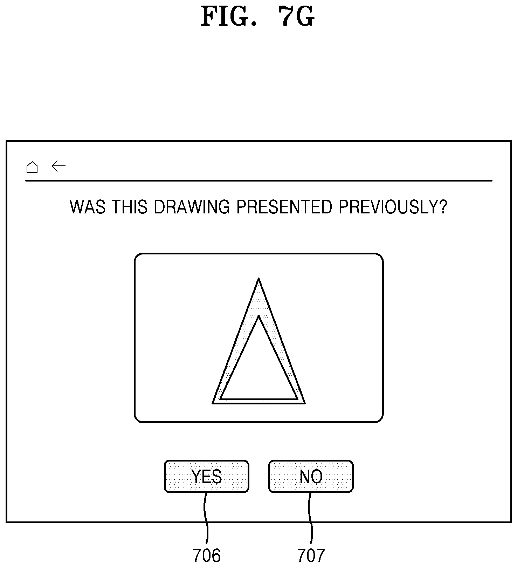

FIG. 7G illustrates a screen for performing the (3-2)th test process. The user may check the (3-2)th instruction and choose one of a Yes button 706 and a No button 707 in response to whether the second presented image is the same as the first presented image. Here, a progress bar (not shown) performing at least one of a function of counting a time limit and a function of illustrating a work progress degree may be further provided.

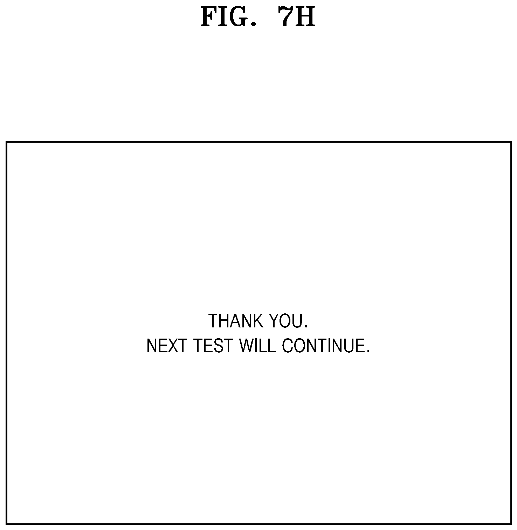

FIG. 7H illustrates a screen indicating completion of the performing of the (3-2)th test process and a start of a next test process.

Referring back to FIG. 3, the first processing unit 152 may perform the fourth test process including the (4-1)th test process and the (4-2)th test process. According to the present embodiment, the first processing unit 152 may perform the fourth test process a plurality of times, excluding a practice test.

When the (4-1)th test process is performed, the first processing unit 152 may illustrate a pre-set presented word and two choice words, and provide, to the user terminal 200, the (4-1)th instruction, in which it is instructed to choose a same word as the pre-set presented word from among the two choice words.

The first processing unit 152 may receive, from the user terminal 200, a result of choosing the same word as the pre-set presented word from among the two choice words, in response to the (4-1)th instruction, and calculate the (4-1)th test score by comparing the result with a correct answer. According to the present embodiment, the (4-1)th test score may be calculated to be high when there are many results determined to be correct answers compared to the total number of times the (4-1)th test processes have been performed.

According to the present embodiment, before the (4-1)th test process is performed, the first processing unit 152 may provide, in a text, a method of performing the (4-1)th test process to the user terminal 200, and perform a practice test for the (4-1)th test process.

The first processing unit 152 may perform the (4-2)th test process after the performing of the (4-1)th test process is completed.

When the (4-2)th test process is performed, the first processing unit 152 may illustrate a pre-set presented word and two color choice words and provide, to the user terminal 200, the (4-2)th instruction, in which it is instructed to choose a word having a same color as the pre-set presented word from among the two color choice words.

The first processing unit 152 may receive, from the user terminal 200, a result of choosing the word having the same color as the pre-set presented word from among the two color choice words, in response to the (4-2)th instruction, and calculate the (4-2)th test score by comparing the result with a correct answer. According to the present embodiment, the (4-2)th test score may be calculated to be high when there are many results determined to be correct answers compared to the total number of times the (4-2)th test processes have been performed.

According to the present embodiment, before the (4-2)th test process is performed, the first processing unit 152 may provide, in a text, a method of performing the (4-2)th test process to the user terminal 200, and perform a practice test for the (4-2)th test process.

According to a selective embodiment, the first processing unit 152 may calculate the fourth test score by adding the (4-1)th test score and the (4-2)th test score. Alternatively, the first processing unit 152 may not add the (4-1)th test score and the (4-2)th test score, but may use the (4-1)th test score and the (4-2)th test score each to evaluate cognitive function decline. According to the present embodiment, it may be evaluated that an inhibitory function is good when the fourth test score is higher than the fourth criterion cut-off score, and is declined when the fourth test score is lower than the fourth criterion cut-off score.

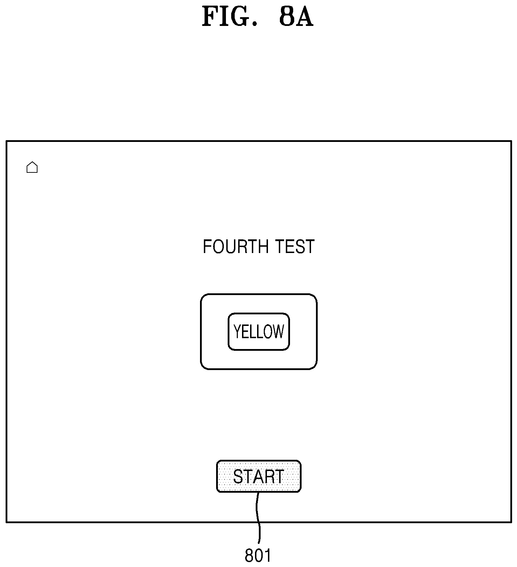

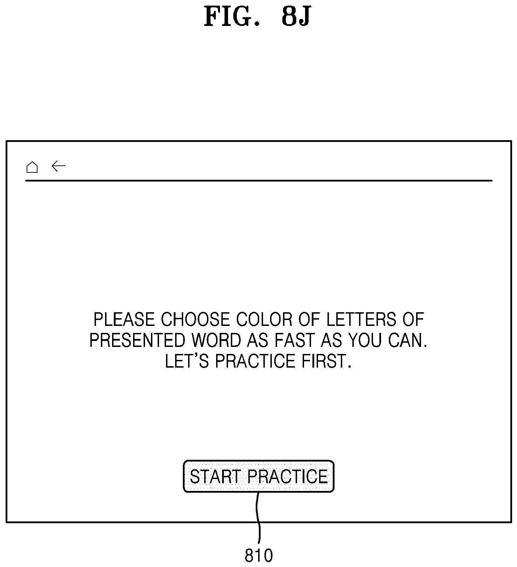

FIGS. 8A through 8N illustrate examples of screens provided to the user terminal 200 to perform the fourth test process. FIGS. 8A through 8I illustrate examples of screens for describing the (4-1)th test process and FIGS. 8J through 8N illustrate examples of screens for describing the (4-2)th test process.

FIG. 8A illustrates a start screen of the fourth test process. When an input of a start button 801 is received, a screen for the (4-1)th test process may be displayed.

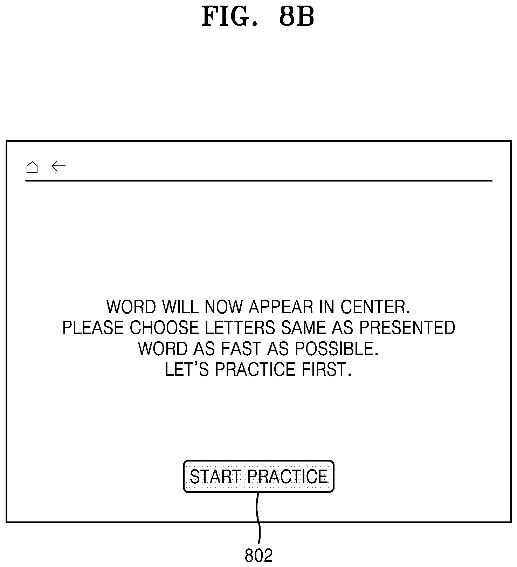

FIG. 8B illustrates a screen in which a method of performing the (4-1)th test process is provided in a text and a start of a practice test process ((4-1)th practice test process) for the (4-1)th test process is notified. When a practice start button 802 is input, a next screen may be displayed.

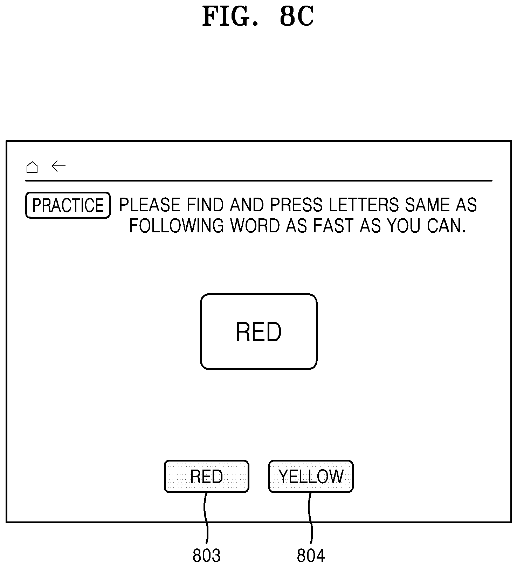

FIG. 8C illustrates a screen for performing the (4-1)th practice test process. After checking a (4-1)th practice instruction, the user may choose a same word as a presented word from among a first choice word 803 and a second choice word 804. Here, a progress bar (not shown) performing at least one of a function of counting a time limit and a function of illustrating a work progress degree may be further provided.

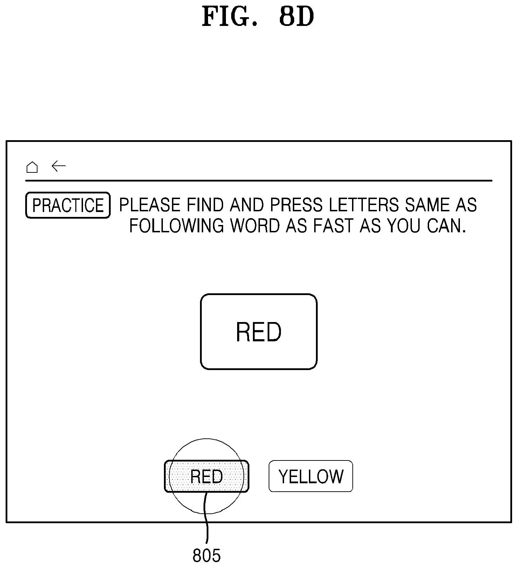

FIG. 8D illustrates a screen indicating whether a result of choosing a first choice word 805 from FIG. 8C is a correct answer. Here, the first choice word 805 is the same as the first choice word 803 of FIG. 8C.

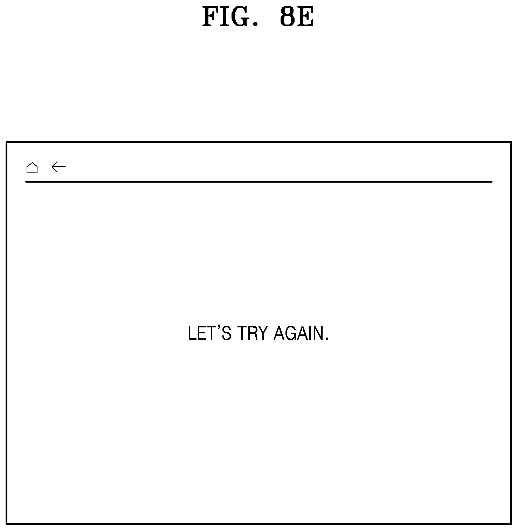

FIG. 8E illustrates a screen indicating a rerun of the (4-1)th practice test process. The (4-1)th practice test process may be rerun when a result of performing the (4-1)th practice test process is a wrong answer.

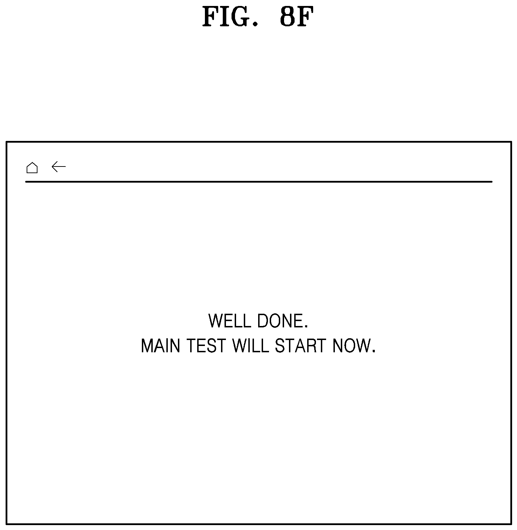

FIG. 8F illustrates a screen indicating completion of the performing of the (4-1)th practice test process, and performing of a main test.

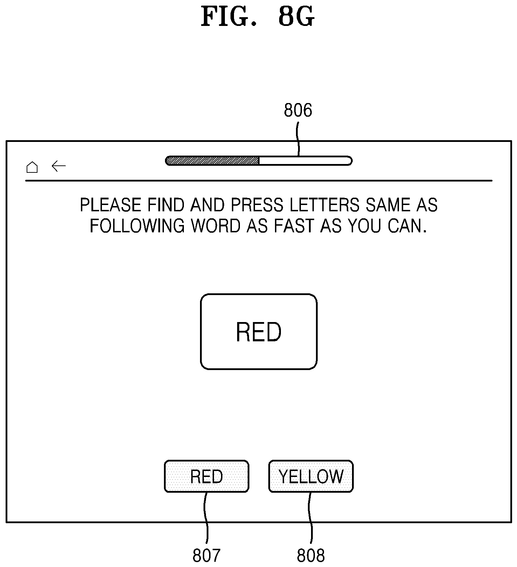

FIG. 8G illustrates a screen for performing the (4-1)th test process. After checking the (4-1)th instruction, the user may choose a same word as a presented word from among a first choice word 807 and a second choice word 808, while looking at a progress bar 806.

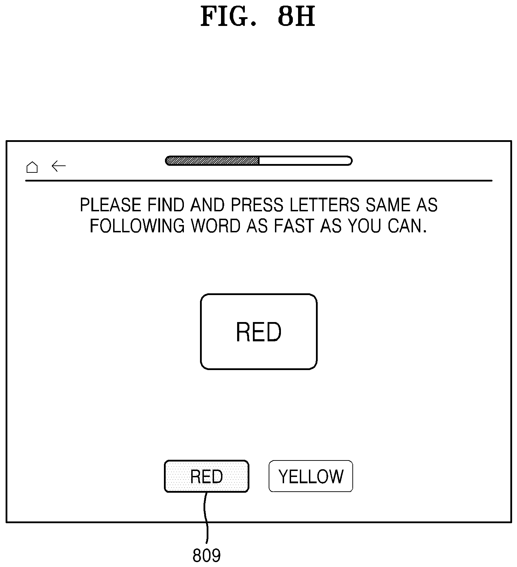

FIG. 8H illustrates a screen indicating whether a result of choosing a first choice word 809 from FIG. 8G is a correct answer. Here, the first choice word 809 is the same as the first choice word 807 of FIG. 8G.

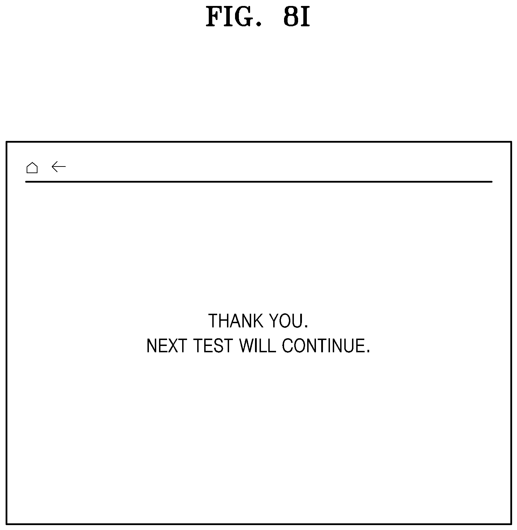

FIG. 8I illustrates a screen indicating completion of the performing of the (4-1)th test process and a start of a next test process.

FIG. 8J illustrates a screen in which a method of performing the (4-2)th test process is provided in a text and a start of a practice test process ((4-2)th practice test process) for the (4-2)th test process is notified. When a practice start button 810 is input, a next screen may be displayed.

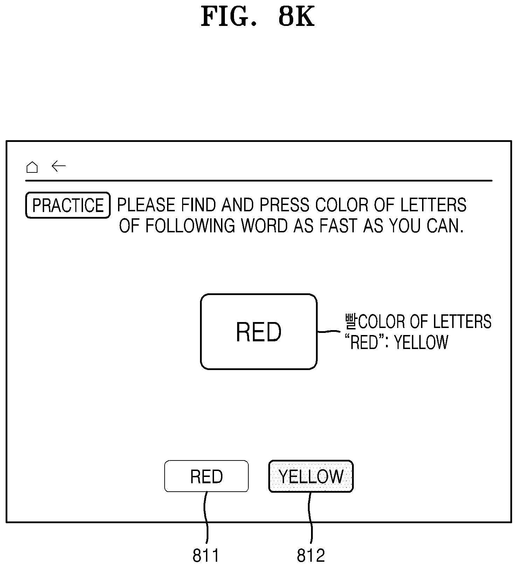

FIG. 8K illustrates a screen for performing the (4-2)th practice test process. After checking a (4-2)th practice instruction, the user may choose a word having a same color as a presented word from among a first choice word 811 and a second choice word 812. Here, a progress bar (not shown) performing at least one of a function of counting a time limit and a function of illustrating a work progress degree may be further provided. FIG. 8K illustrates an example in which the user chose the second choice word 812.

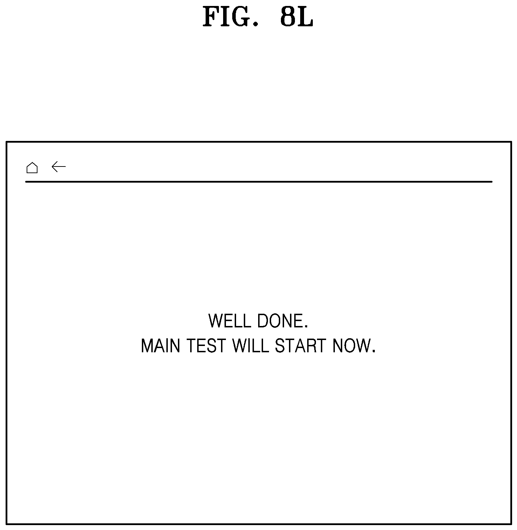

FIG. 8L illustrates a screen indicating completion of the performing of the (4-2)th practice test process, and performing of a main test.

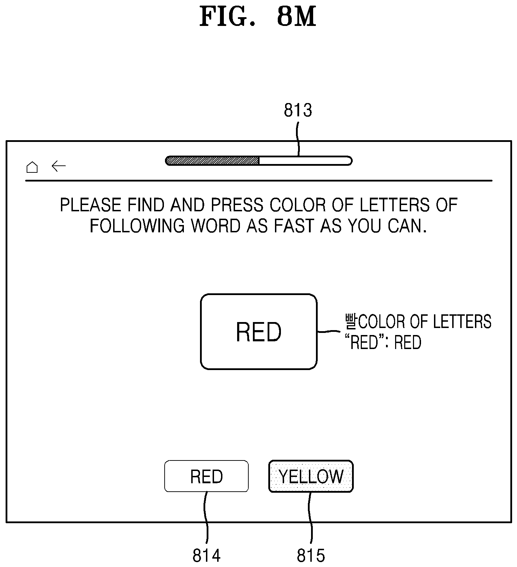

FIG. 8M illustrates a screen for performing the (4-2)th test process. After checking the (4-2)th instruction, the user may choose a word having a same color as a presented word from among a first choice word 814 and a second choice word 815, while looking at a progress bar 813. FIG. 8M illustrates an example in which the user chose the second choice word 815.

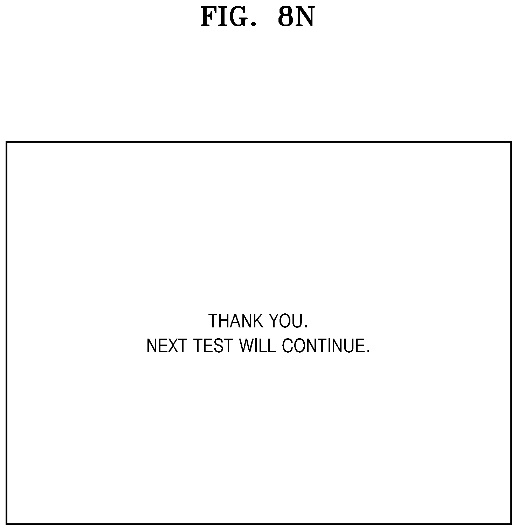

FIG. 8N illustrates a screen indicating completion of the performing of the (4-2)th test process and a start of a next test process.

Referring back to FIG. 3, the first processing unit 152 may perform the fifth test process. According to the present embodiment, the first processing unit 152 may perform the fifth test process a plurality of times, excluding a practice test.

When the fifth test process is performed, the first processing unit 152 may illustrate a pre-set presented line and a plurality of choice lines having different angles, and provide, to the user terminal 200, the fifth instruction, in which it is instructed to choose a line having a most similar angle as the presented line from among the plurality of choice lines.

The first processing unit 152 may receive, from the user terminal 200, a result of choosing a line having a most similar angle as the presented line from among the plurality of choice lines, in response to the fifth instruction, and calculate the fifth test score by comparing the result with a correct line. According to the present embodiment, the fifth test score may be calculated to be high when there are many results determined to be correct answers compared to the total number of times the fifth test processes have been performed.

According to the present embodiment, before the fifth test process is performed, the first processing unit 152 may provide, in a text, a method of performing the fifth test process to the user terminal 200, and perform a practice test for the fifth test process.

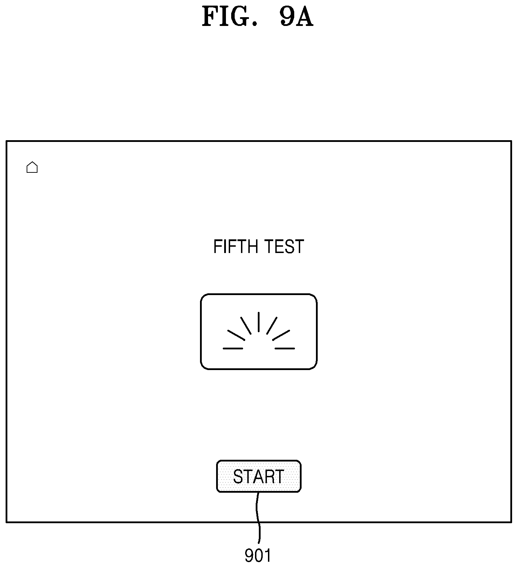

According to the present embodiment, it may be evaluated that visuospatial perception is good when the fifth test score is higher than the fifth criterion cut-off score, and is declined when the fifth test score is lower than the fifth criterion cut-off score. Also, as shown in FIGS. 9A through 9H, hemidysesthesia may be analyzed from test results by changing locations of a presented line and a plurality of choice lines while testing the user.

FIGS. 9A through 9H illustrate examples of screens provided to the user terminal 200 to perform the fifth test process.

FIG. 9A illustrates a start screen of the fifth test process. When an input of a start button 901 is received, a next screen may be displayed.

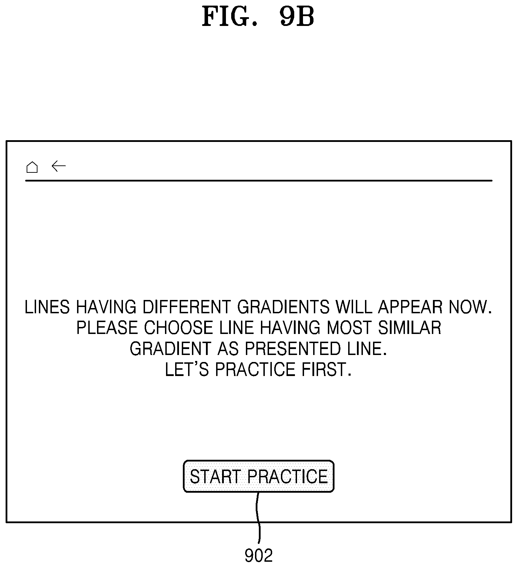

FIG. 9B illustrates a screen in which a method of performing the fifth test process is provided in a text and a start of a practice test process (fifth practice test process) for the fifth test process is notified. When a practice start button 902 is input, a next screen may be displayed.

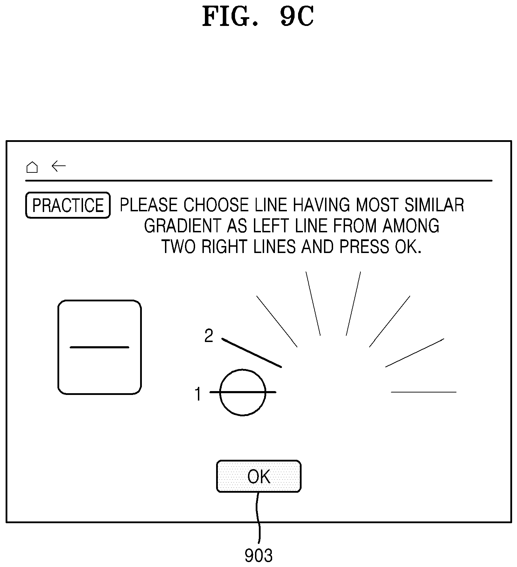

FIG. 9C illustrates a screen for performing the fifth practice test process. The user may check a fifth practice instruction and choose a line having a most similar angle as a presented line shown at the left from among a plurality of choice lines shown at the right. Here, a progress bar (not shown) performing at least one of a function of counting a time limit and a function of illustrating a work progress degree may be further provided. When the user chooses a line and inputs an OK button 903, a next screen may be displayed.

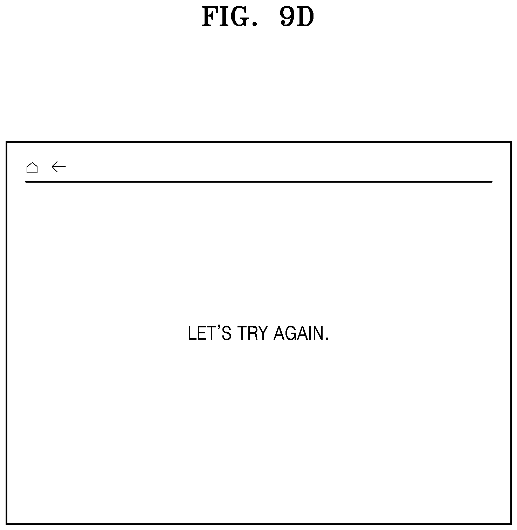

FIG. 9D illustrates a screen indicating a rerun of the fifth practice test process. The fifth practice test process may be rerun when a result of performing the fifth practice test process is a wrong answer.

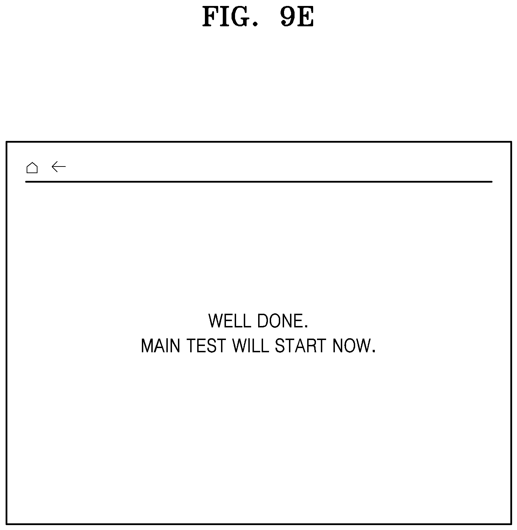

FIG. 9E illustrates a screen indicating completion of the performing of the fifth practice test process, and performing of a main test.

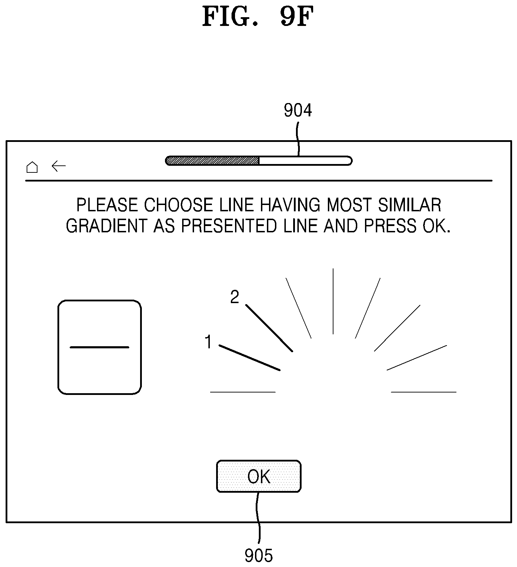

FIG. 9F illustrates an embodiment of a screen for performing the fifth test process. The user may check the fifth instruction and choose a line having a most similar angle as a presented line shown at the left from among a plurality of choice lines shown at the right, while looking at a progress bar 904. When the user chooses a line and inputs an OK button 905, a next screen may be displayed.

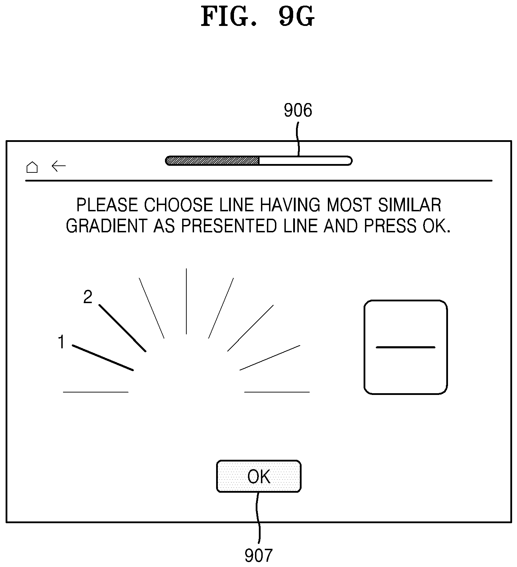

FIG. 9G illustrates another embodiment of a screen for performing the fifth test process. The user may check the fifth instruction and choose a line having a most similar angle as a presented line shown at the right from among a plurality of choice lines shown at the left, while looking at a progress bar 906. When the user chooses a line and inputs an OK button 907, a next screen may be displayed.

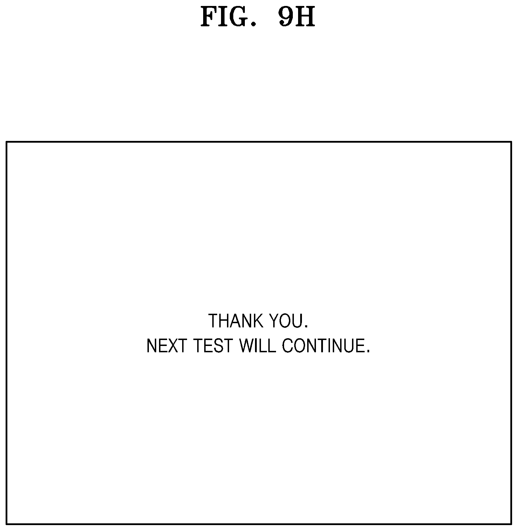

FIG. 9H illustrates a screen indicating completion of the performing of the fifth test process and a start of a next test process.

Referring back to FIG. 3, the first processing unit 152 may perform the sixth test process. According to the present embodiment, the first processing unit 152 may perform the sixth test process a plurality of times, excluding a practice test.

When the sixth test process is performed, the first processing unit 152 may illustrate a plurality of pre-set presented figures and provide, to the user terminal 200, a sixth instruction, in which it is instructed to choose a figure having a different color from among the plurality of pre-set presented figures.

The first processing unit 152 may receive, from the user terminal 200, a result of choosing a figure having a different color from among the plurality of pre-set presented figures, in response to the sixth instruction, and calculate the sixth test score by comparing the result with a correct answer. According to the present embodiment, the sixth test score may be calculated to be high when there are many results determined to be correct answers compared to the total number of times the sixth test processes have been performed.

According to the present embodiment, before the sixth test process is performed, the first processing unit 152 may provide, in a text, a method of performing the sixth test process to the user terminal 200, and perform a practice test for the sixth test process.

According to the present embodiment, it may be evaluated that a color classifying ability is good when the sixth test score is higher than the sixth criterion cut-off score, and is declined when the sixth test score is lower than the sixth criterion cut-off score.

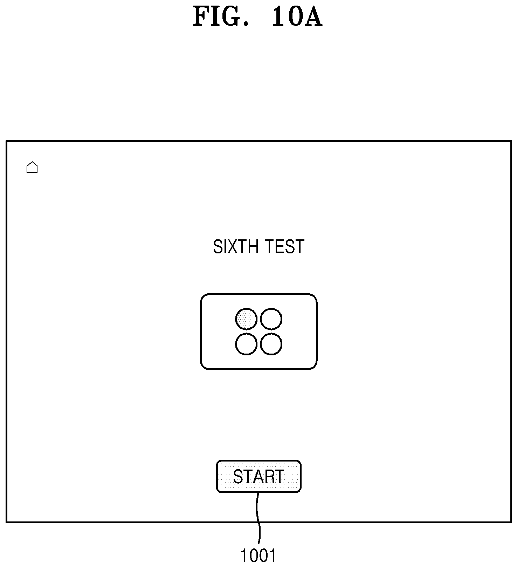

FIGS. 10A through 10J illustrate examples of screens provided to the user terminal 200 to perform the sixth test process.

FIG. 10A illustrates a start screen of the sixth test process. When an input of a start button 1001 is received, a next screen may be displayed.

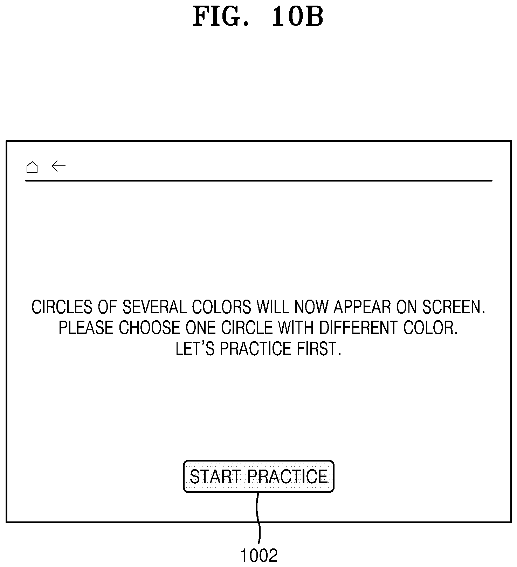

FIG. 10B illustrates a screen in which a method of performing the sixth test process is provided in a text and a start of a practice test process (sixth practice test process) for the fifth test process is notified. When a practice start button 1002 is input, a next screen may be displayed.

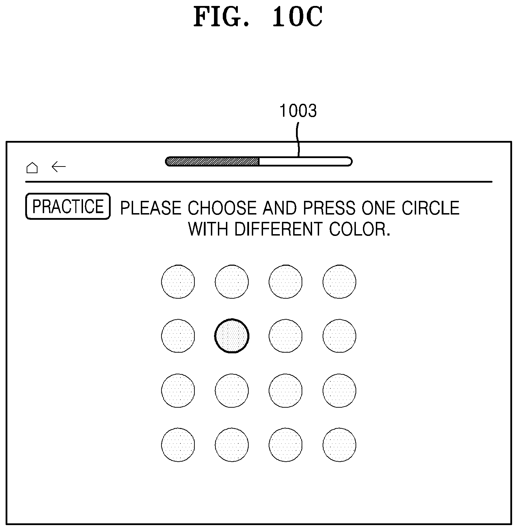

FIG. 10C illustrates a screen for performing the sixth practice test process. The user may check a sixth practice instruction, and choose a figure having a different color from among a plurality of pre-set presented figures while looking at a progress bar 1003. A next screen may be displayed when the choosing of a figure is completed or counting of a time limit is completed.

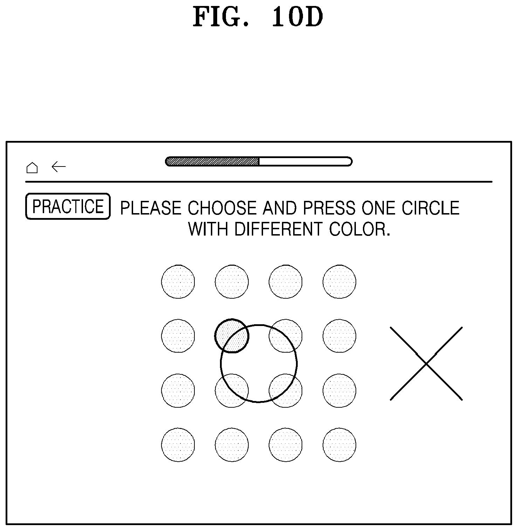

FIG. 10D illustrates a screen showing a result of performing the sixth practice test process of FIG. 10C. When the chosen figure is a correct answer, the correct answer may be indicated by using a correct answer sign and/or a correct answer sound effect, and when the chosen figure is a wrong answer, the wrong answer may be indicated by using a wrong answer sign and/or a wrong answer sound effect.

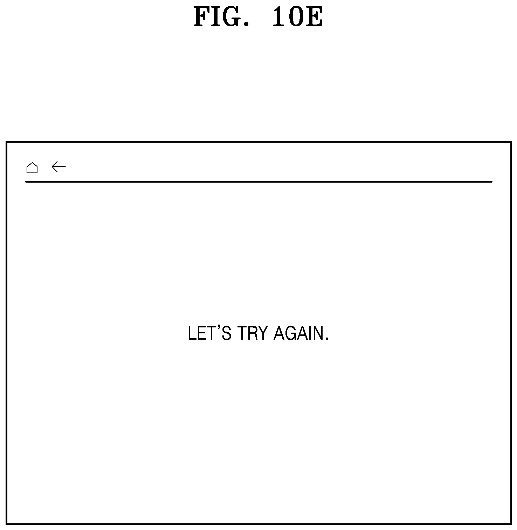

FIG. 10E illustrates a screen indicating a rerun of the sixth practice test process. The sixth practice test process may be rerun when a result of performing the sixth practice test process is a wrong answer.

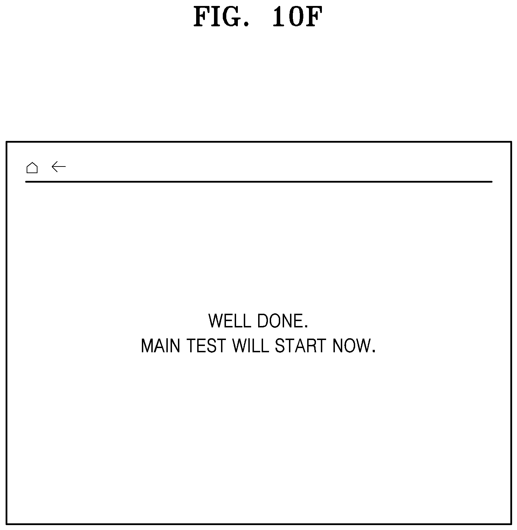

FIG. 10F illustrates a screen indicating completion of the performing of the sixth practice test process, and performing of a main test.

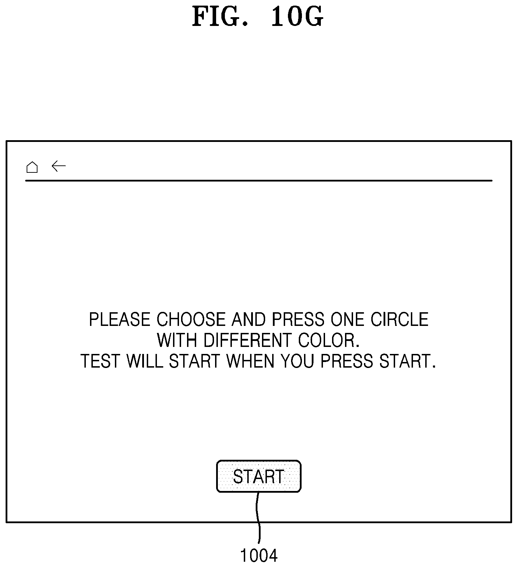

FIG. 10G illustrates a screen providing, in a text, a method of performing the sixth test process, and indicating a start of the sixth test process. When a start button 1004 is input, a next screen may be displayed.

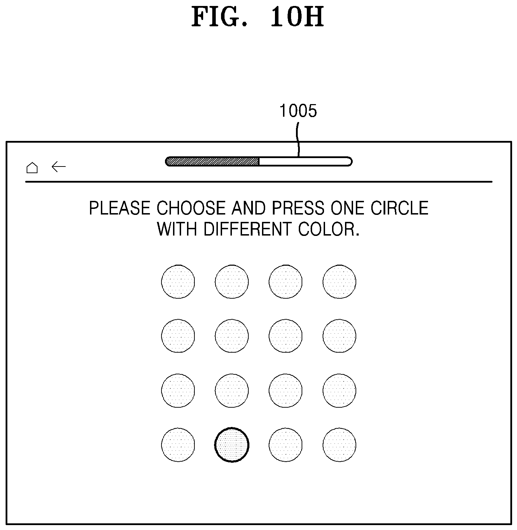

FIG. 10H illustrates a screen for performing the sixth test process. The user may check a sixth instruction, and choose a figure having a different color from among a plurality of pre-set presented figures while looking at a progress bar 1005. A next screen may be displayed when the choosing of a figure is completed or counting of a time limit is completed.

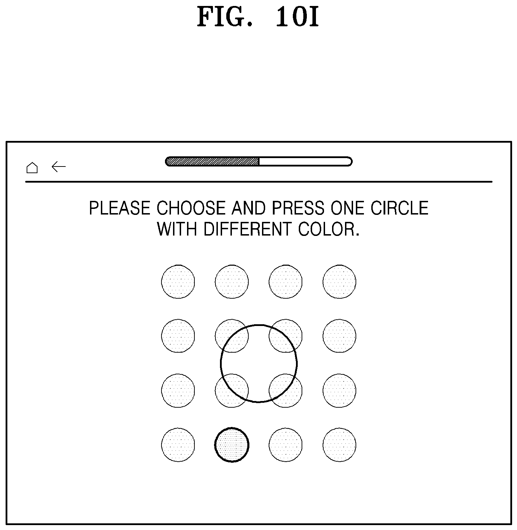

FIG. 10I illustrates a screen showing a result of performing the sixth test process of FIG. 10H. When the chosen figure is a correct answer, the correct answer may be indicated by using a correct answer sign and/or a correct answer sound effect. Also, when the chosen figure is a wrong answer, the wrong answer may be indicated by using a wrong answer sign and/or a wrong answer sound effect.

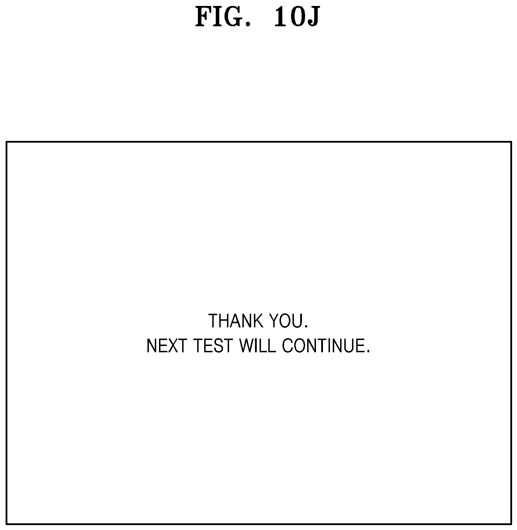

FIG. 10J illustrates a screen indicating completion of the performing of the sixth test process and a start of a next test process.

Referring back to FIG. 3, the first processing unit 152 may perform the seventh test process including the (7-1)th test process and the (7-2)th test process. According to the present embodiment, the first processing unit 152 may perform the seventh test process a plurality of times.

When the (7-1)th test process is performed, the first processing unit 152 may illustrate a plurality of first object presented images including names of first objects and shapes of the first objects, and provide, to the user terminal 200, the (7-1)th instruction, in which it is instructed to choose the plurality of first object presented images in a descending order by determining sizes of the first objects.

The first processing unit 152 may receive, from the user terminal 200, a result of choosing the first object presented images in the descending order, in response to the (7-1)th instruction, and calculate the (7-1)th test score by comparing the result with a correct answer. According to the present embodiment, the (7-1)th test score may be calculated to be high when there are many results determined to be correct answers compared to the total number of times the (7-1)th test processes have been performed.

According to the present embodiment, before the (7-1)th test process is performed, the first processing unit 152 may provide, in a text, a method of performing the (7-1)th test process to the user terminal 200.

The first processing unit 152 may perform the (7-2)th test process after the performing of the (7-1)th test process is completed.

When the (7-2)th test process is performed, the first processing unit 152 may illustrate a plurality of second object presented images including shapes of second objects, and provide, to the user terminal 200, the (7-2)th instruction, in which it is instructed to choose the plurality of second object presented images in a descending order by determining weights of the second objects.

The first processing unit 152 may receive, from the user terminal 200, a result of choosing the second object presented images in the descending order, in response to the (7-2)th instruction, and calculate the (7-2)th test score by comparing the result with a correct answer.

According to the present embodiment, before the (7-2)th test process is performed, the first processing unit 152 may provide, in a text, a method of performing the (7-2)th test process to the user terminal 200. According to the present embodiment, the (7-2)th test score may be calculated to be high when there are many results determined to be correct answers compared to the total number of times the (7-2)th test processes have been performed.

According to a selective embodiment, the first processing unit 152 may calculate the seventh test score by adding the (7-1)th test score and the (7-2)th test score. Alternatively, the first processing unit 152 may not add the (7-1)th test score and the (7-2)th test score, but may use the (7-1)th test score and the (7-2)th test score each to evaluate cognitive function decline. According to the present embodiment, it may be evaluated that semantic memory is good when the seventh test score is higher than the seventh criterion cut-off score, and is declined when the seventh test score is lower than the seventh criterion cut-off score.

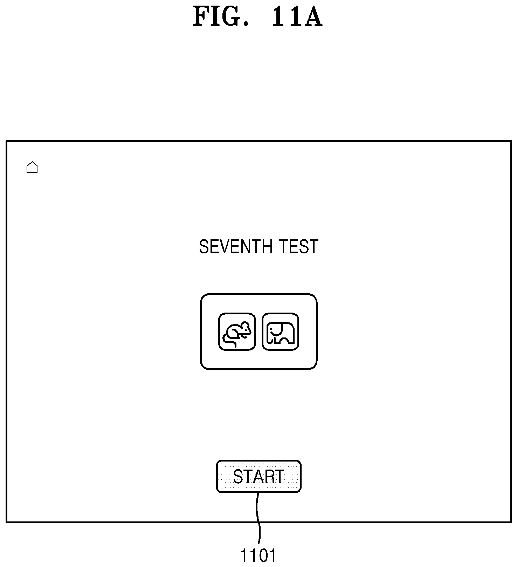

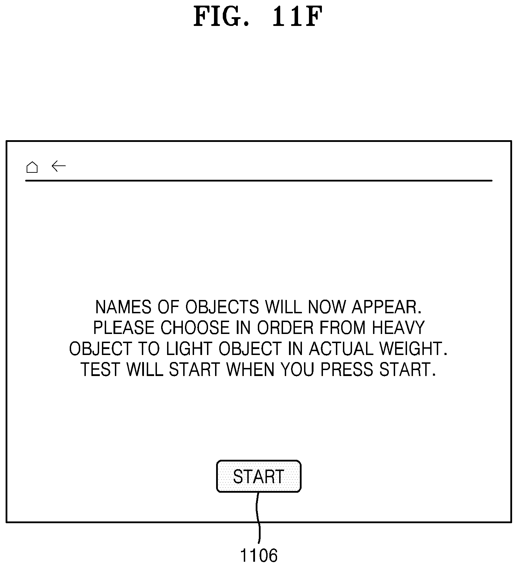

FIGS. 11A through 11H illustrate examples of screens provided to the user terminal 200 to perform the seventh test process. FIGS. 11A through 11E illustrate examples of screens for describing the (7-1)th test process and FIGS. 11F through 11H illustrate examples of screens for describing the (7-2)th test process.

FIG. 11A illustrates a start screen of the seventh test process. When an input of a start button 1101 is received, a screen for the (7-1)th test process may be displayed.

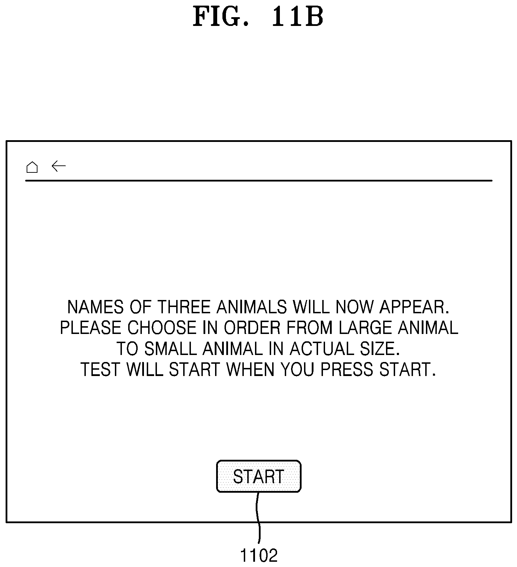

FIG. 11B illustrates a screen providing, in a text, a method of performing the (7-1)th test process, and indicating a start of the (7-1)th test process. When a start button 1102 is input, a next screen may be displayed.

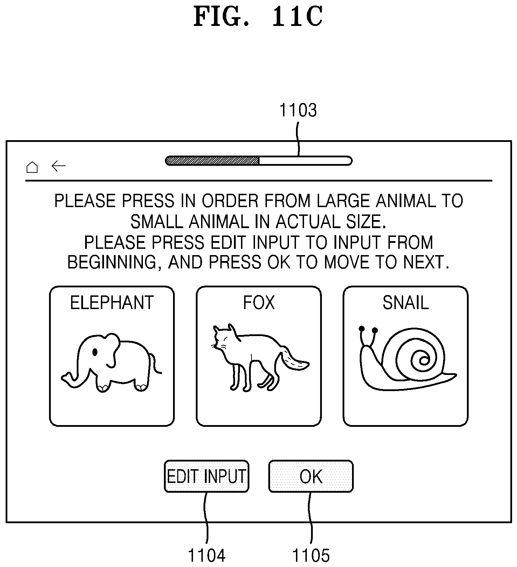

FIG. 11C illustrates a screen for performing the (7-1)th test process. The user may check the (7-1)th instruction, and then choose a plurality of first object presented images according to size while looking at a progress bar 1103. The user may re-choose by inputting an edit input button 1104 when the choice needs to be edited. When the user inputs an OK button 1105 after completing the choice, a next screen may be displayed.

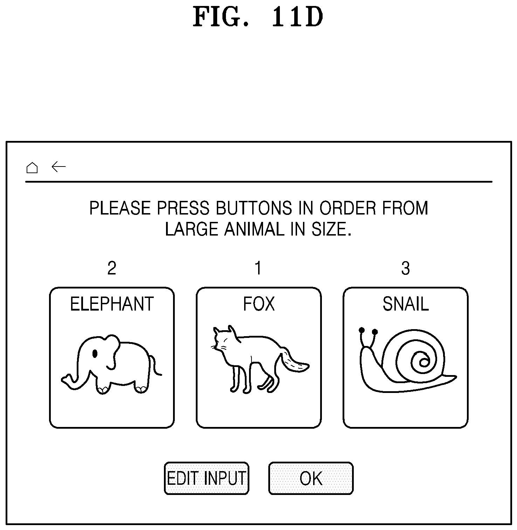

FIG. 11D illustrates a screen showing a result of performing the (7-1)th test process. Numbers indicating the order the user chose the first object presented images may be output on the first object presented images.

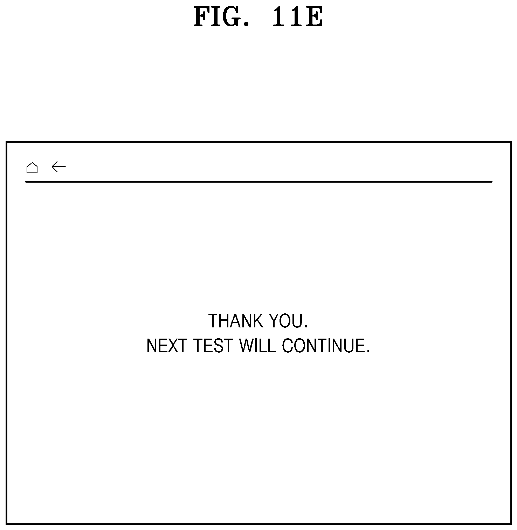

FIG. 11E illustrates a screen indicating completion of the performing of the (7-1)th test process and a start of a next test process.

FIG. 11F illustrates a screen providing, in a text, a method of performing the (7-2)th test process, and indicating a start of the (7-2)th test process. When a start button 1106 is input, a next screen may be displayed.

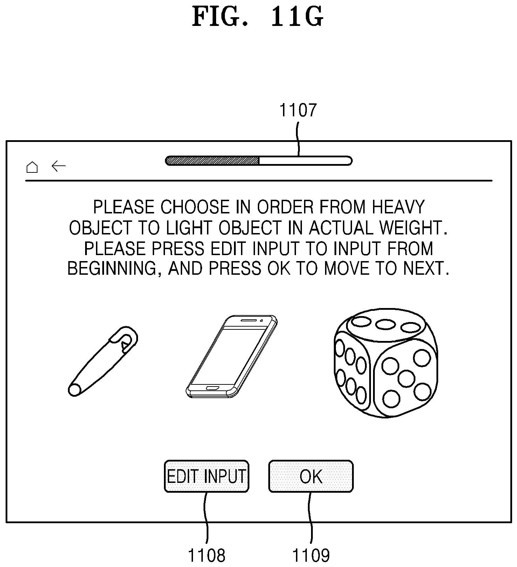

FIG. 11G illustrates a screen for performing the (7-2)th test process. The user may check the (7-2)th instruction, and then choose a plurality of second object presented images according to weight while looking at a progress bar 1107. The user may re-choose by inputting an edit input button 1108 when the choice needs to be edited. When the user inputs an OK button 1109 after completing the choice, a next screen may be displayed.

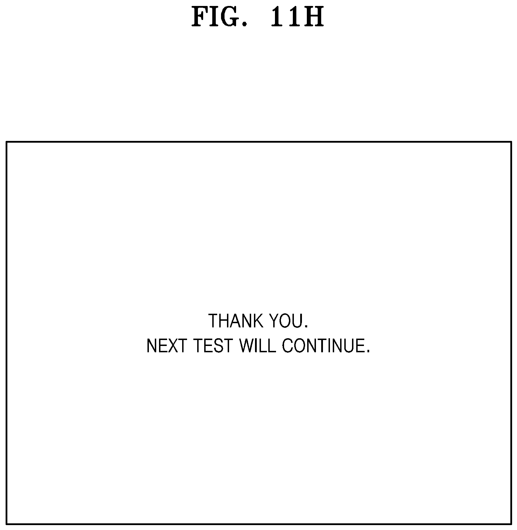

FIG. 11H illustrates a screen indicating completion of the performing of the (7-2)th test process and a start of a next test process.

Referring back to FIG. 3, the first processing unit 152 may perform the eighth test process. According to the present embodiment, the first processing unit 152 may perform the eighth test process a plurality of times, excluding a practice test.

When the eighth test process is performed, the first processing unit 152 may illustrate a pre-set face presented image expressing an emotion and a plurality of buttons for choosing intensity of the emotion, and provide, to the user terminal 200, the eighth instruction, in which it is instructed to choose one of the plurality of buttons as intensity of the emotion shown on the pre-set face presented image.

The first processing unit 152 may receive, from the user terminal 200, a result of choosing one of the plurality of buttons as the intensity of the emotion shown on the pre-set face presented image, in response to the eighth instruction, and calculate the eighth test score by comparing the result with a correct answer. According to the present embodiment, the eighth test score may be calculated to be high when there are many results determined to be correct answers compared to the total number of times the eighth test processes have been performed.

According to the present embodiment, before the eighth test process is performed, the first processing unit 152 may provide, in a text, a method of performing the eighth test process to the user terminal 200, and perform a practice test for the eighth test process.

According to the present embodiment, it may be evaluated that emotion intensity cognitive power is good when the eighth test score is higher than the eighth criterion cut-off score, and is declined when the eighth test score is lower than the eighth criterion cut-off score.

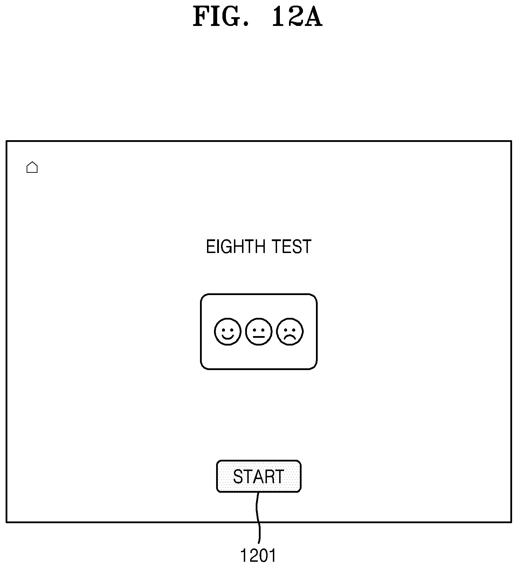

FIGS. 12A through 12G illustrate examples of screens provided to the user terminal 200 to perform the eighth test process.

FIG. 12A illustrates a start screen of the eighth test process. When an input of a start button 1201 is received, a next screen may be displayed.

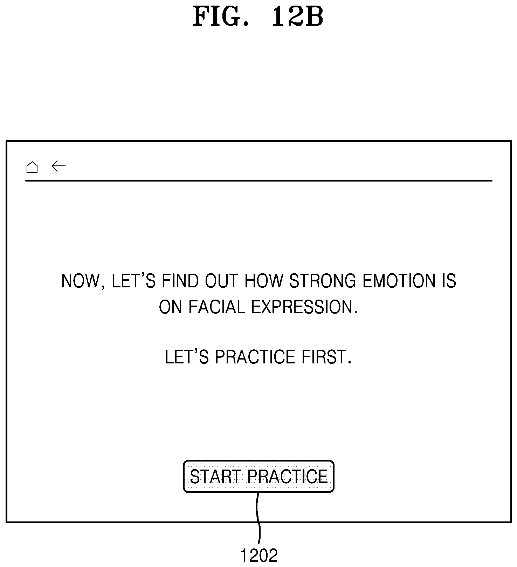

FIG. 12B illustrates a screen in which a method of performing the eighth test process is provided in a text and a start of a practice test process (eighth practice test process) for the eighth test process is notified. When a practice start button 1202 is input, a next screen may be displayed.

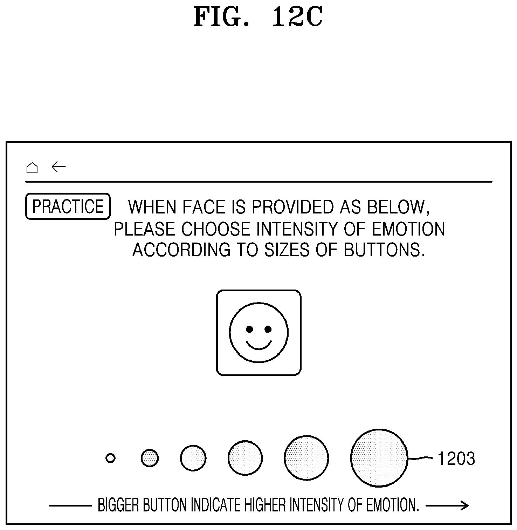

FIG. 12C illustrates a screen for performing the eighth practice test process. The user may check an eighth practice instruction, and then choose one of a plurality of buttons 1203 as intensity of an emotion shown on a face presented image. Here, a progress bar (not shown) performing at least one of a function of counting a time limit and a function of illustrating a work progress degree may be further provided. According to the present embodiment, a greater size of the plurality of buttons 1203 may indicate higher intensity of emotion.

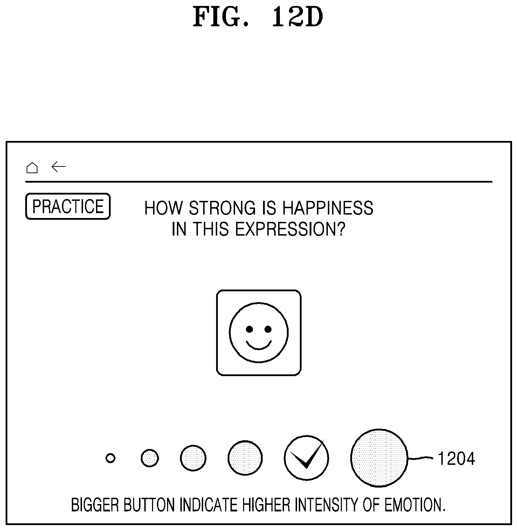

FIG. 12D illustrates a screen showing a result of performing the eighth test process. The intensity of emotion chosen by the user may be output on one of a plurality of buttons 1204.

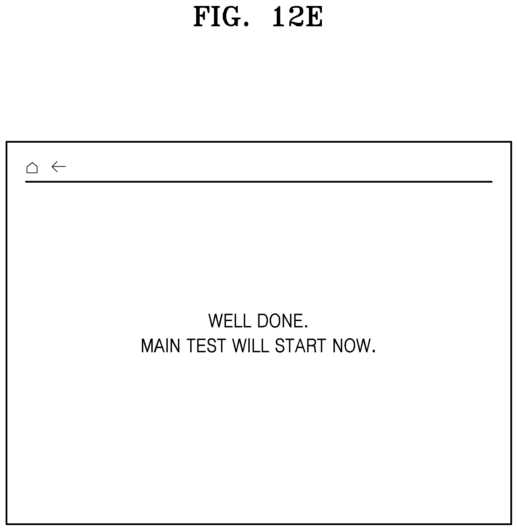

FIG. 12E illustrates a screen indicating completion of the performing of the eighth practice test process, and performing of a main test.

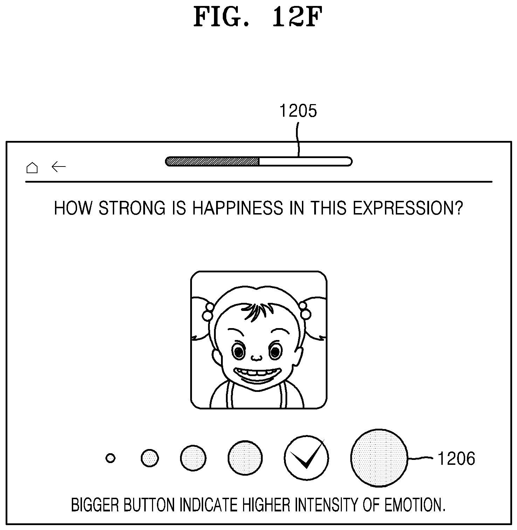

FIG. 12F illustrates an embodiment of a screen for performing the eighth test process. The user may check the eighth instruction, and then choose one of a plurality of buttons 1206 as intensity of an emotion shown on a face presented image while looking at a progress bar 1205. A next screen may be displayed when the user completes choosing one of the plurality of buttons 1206 or when counting of a time limit is completed.

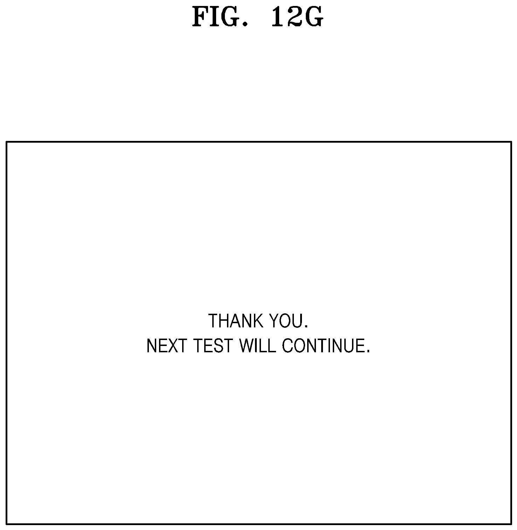

FIG. 12G illustrates a screen indicating completion of the performing of the eighth test process and a start of a next test process.

Referring back to FIG. 3, the first processing unit 152 may perform the ninth test process. According to the present embodiment, the first processing unit 152 may perform the ninth test process a plurality of times.

When the ninth test process is performed, the first processing unit 152 may illustrate pre-set presented paragraph, a record start button, and a record end button, and provide, to the user terminal 200, the ninth instruction, in which it is instructed to input the record start button, utter the pre-set presented paragraph, and then input the record end button when uttering of the paragraph is completed.

After receiving, from the user terminal 200, an input of the record start button in response to the ninth instruction, the first processing unit 152 may receive and store an utterance result regarding the pre-set presented paragraph, and receive an input of the record end button.

The first processing unit 152 may count a second spent time from when the input of the record start button is received to when the input of the record end button is received.

The first processing unit 152 may convert the utterance result of the pre-set presented paragraph into a paragraph utterance text, and compare the number of word segments included in the paragraph utterance text with a correct number of word segments.

The first processing unit 152 may calculate the ninth test score by reflecting a result of the comparing and a result of counting the second spent time. In the present embodiment, the ninth test score may be calculated differently depending on the number of word segments determined to be correct answers compared to the total number of times the ninth test processes have been performed, and the result of counting the second spent time. Even when there are many word segments determined to be correct answers compared to the total number of times the ninth test processes have been performed, the ninth test score may be low when the result of counting the second spent time is high. Also, even when there are little word segments determined to be correct answers compared to the total number of times the ninth test processes have been performed, the ninth test score may be high when the result of counting the second spent time is low.

In the present embodiment, before the ninth test process is performed, the first processing unit 152 may provide, in a text, a method of performing the ninth test process to the user terminal 200.

In the present embodiment, it may be evaluated that a reading ability is good when the ninth test score is higher than the ninth criterion cut-off score, and that the reading ability is declined when the ninth test score is lower than the ninth criterion cut-off score.

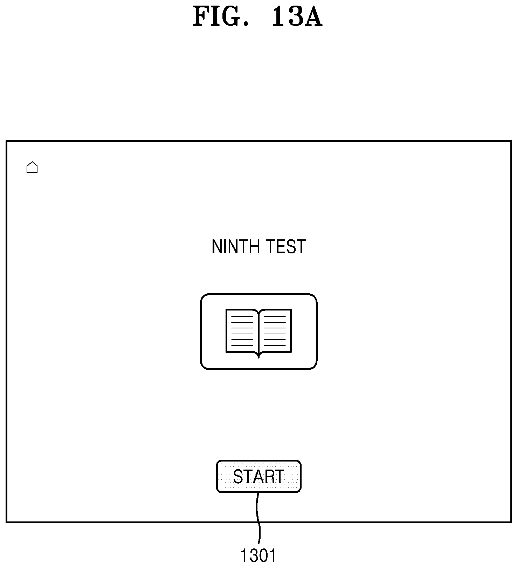

FIGS. 13A through 13D illustrate examples of screens provided to the user terminal 200 to perform the ninth test process.

FIG. 13A illustrates a start screen of the ninth test process. When an input of a start button 1301 is received, a next screen may be displayed.

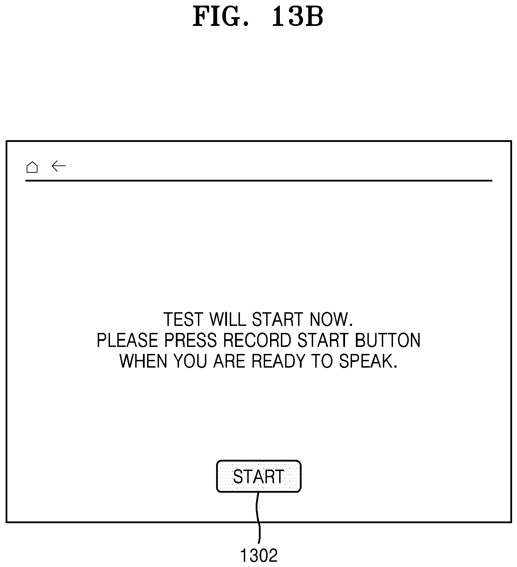

FIG. 13B illustrates a screen providing, in a text, a method of performing the ninth test process, and indicating a start of the ninth test process. When a start button 1302 is input, a next screen may be displayed.

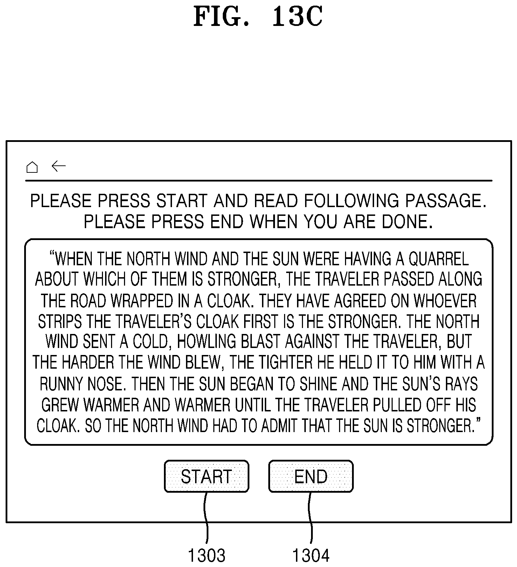

FIG. 13C illustrates a screen for performing a ninth practice test process. The user may check a ninth practice instruction, input a start button 1303, utter a presented paragraph, and input an end button 1304 when the uttering of the presented paragraph is completed. Here, a progress bar (not shown) performing at least one of a function of counting a time limit and a function of illustrating a work progress degree may be further provided.

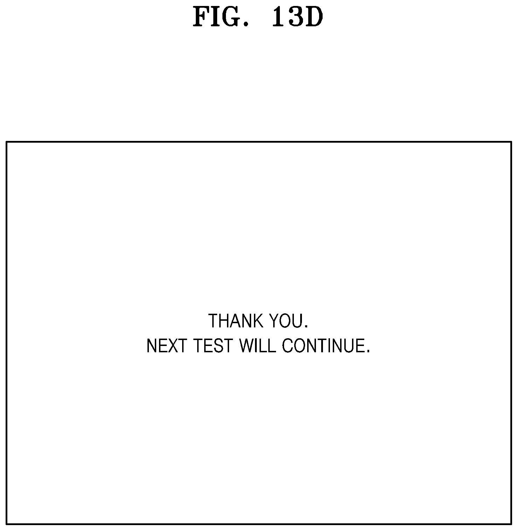

FIG. 13D illustrates a screen indicating completion of the performing of the ninth test process and a start of a next test process.

Referring to FIG. 3, the first processing unit 152 may perform the tenth test process including the (10-1)th test process and the (10-2)th test process. According to the present embodiment, the first processing unit 152 may perform the tenth test process a plurality of times, excluding a practice test.

When the (10-1)th test process is performed, the first processing unit 152 may illustrate a pre-set region and provide, to the user terminal 200, the (10-1)th instruction, in which it is instructed to recall locations and an order pre-set presented figures appear in the pre-set region and sequentially input the same.

While providing the (10-1)th instruction, the first processing unit 152 may provide, to the user terminal 200, a first demonstration video in which the pre-set presented figures sequentially appear in the pre-set region.

When the first demonstration video is ended, the first processing unit 152 may receive, from the user terminal 200, a result of recalling the first demonstration video and sequentially inputting the locations and order the pre-set presented figures appear in the pre-set region. Here, the user may recall the first demonstration video and sequentially input the locations and order the presented figures appear.

The first processing unit 152 may calculate the (10-1)th test score by comparing the result of sequentially inputting the locations and order the pre-set presented figures appear in the pre-set region with a correct answer. In the present embodiment, the (10-1)th test score may be calculated to be high when there are many results determined to be correct answers compared to the total number of times the (10-1)th test processes have been performed.

In the present embodiment, before the (10-1)th test process is performed, the first processing unit 152 may provide, in a text, a method of performing the (10-1)th test process to the user terminal 200 and perform a practice test for the (10-1)th test process.

The first processing unit 152 may perform the (10-2)th test process after the performing of the (10-1)th test process is completed.

When the (10-2)th test process is performed, the first processing unit 152 may illustrate a pre-set region and provide, to the user terminal 200, the (10-2)th instruction, in which it is instructed to recall locations and an order pre-set presented figures appear in the pre-set region and input the same in reverse.

While providing the (10-2)th instruction, the first processing unit 152 may provide, to the user terminal 200, a second demonstration video in which the pre-set presented figures sequentially appear in the pre-set region.

When the second demonstration video is ended, the first processing unit 152 may receive, from the user terminal 200, a result of recalling the second demonstration video and inputting the locations and order the pre-set presented figures appear in the pre-set region in reverse. Here, the user may recall the second demonstration video and input, in reverse, the locations and order the presented figures appear.

The first processing unit 152 may calculate the (10-2)th test score by comparing the result of inputting, in reverse, the locations and order the pre-set presented figures appear in the pre-set region with a correct answer. In the present embodiment, the (10-2)th test score may be calculated to be high when there are many results determined to be correct answers compared to the total number of times the (10-2)th test processes have been performed.

In the present embodiment, before the (10-2)th test process is performed, the first processing unit 152 may provide, in a text, a method of performing the (10-2)th test process to the user terminal 200 and perform a practice test for the (10-2)th test process.

According to a selective embodiment, the first processing unit 152 may calculate the tenth test score by adding the (10-1)th test score and the (10-2)th test score. Alternatively, the first processing unit 152 may not add the (10-1)th test score and the (10-2)th test score, but may use the (10-1)th test score and the (10-2)th test score each to evaluate cognitive function decline. In the present embodiment, it may be evaluated that attention and working memory are good when the tenth test score is higher than the tenth criterion cut-off score, and that the attention and working memory are declined when the tenth test score is lower than the tenth criterion cut-off score.

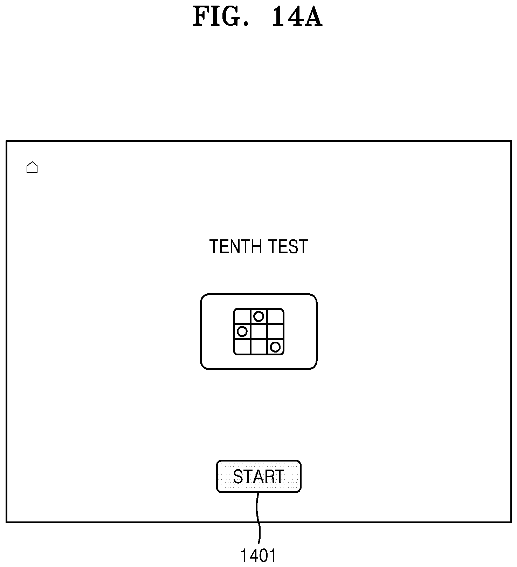

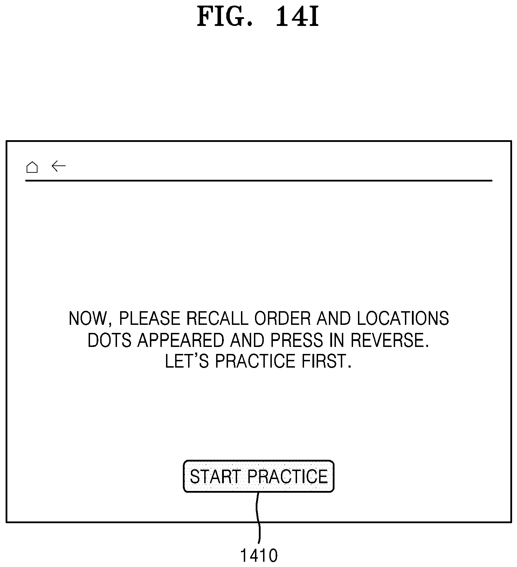

FIGS. 14A through 14O illustrate examples of screens provided to the user terminal 200 to perform the tenth test process. FIGS. 14A through 14H illustrate examples of screens for describing the (10-1)th test process and FIGS. 14I through 14O illustrate examples of screens for describing the (10-2)th test process.

FIG. 14A illustrates a start screen of the tenth test process. When an input of a start button 1401 is received, a screen for the (10-1)th test process may be displayed.

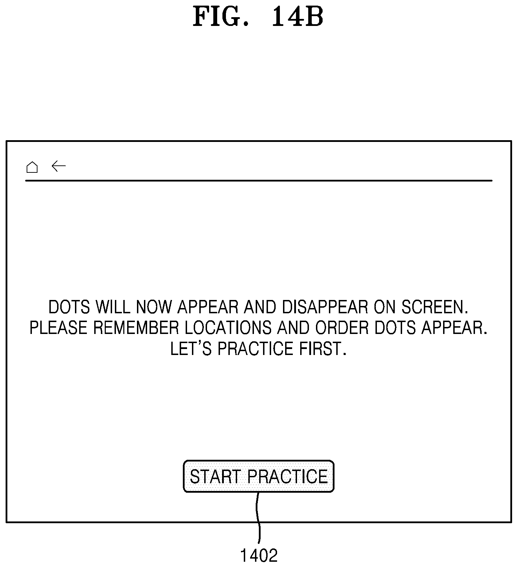

FIG. 14B illustrates a screen in which a method of performing the (10-1)th test process is provided in a text and a start of a practice test process ((10-1)th practice test process) for the (10-1)th test process is notified. When a practice start button 1402 is input, a next screen may be displayed.

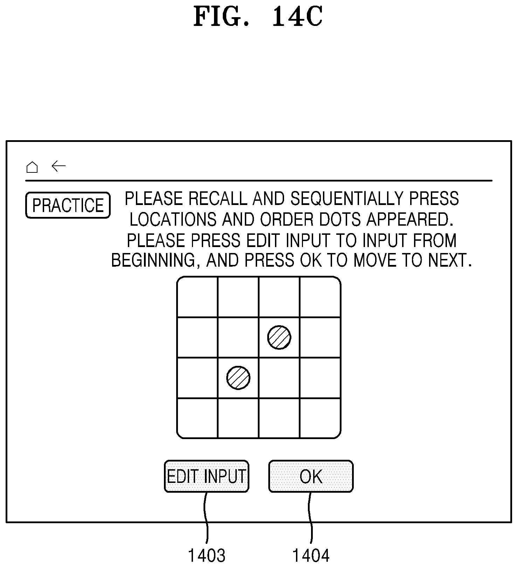

FIG. 14C illustrates an embodiment of a screen for performing the (10-1)th practice test process. FIG. 14C illustrates a first practice demonstration video together with a (10-1)th practice instruction. Also, an edit input button 1403 for editing an input and an OK button 1404 for completing the input may also be provided.

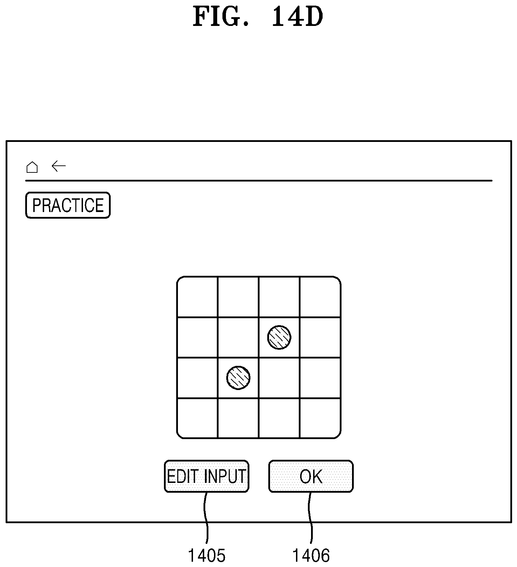

FIG. 14D illustrates another embodiment of a screen for performing the (10-1)th practice test process. The user may recall the first practice demonstration video of FIG. 14C and sequentially input locations and order presented figures appeared in a pre-set region. When the input needs to be edited, the user may input an edit input button 1405 and edit the input. When the input is completed, the user may input an OK button 1406.

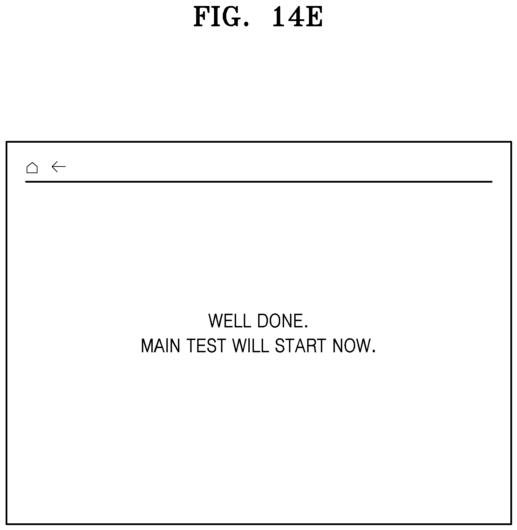

FIG. 14E illustrates a screen indicating completion of the performing of the (10-1)th practice test process, and performing of a main test.

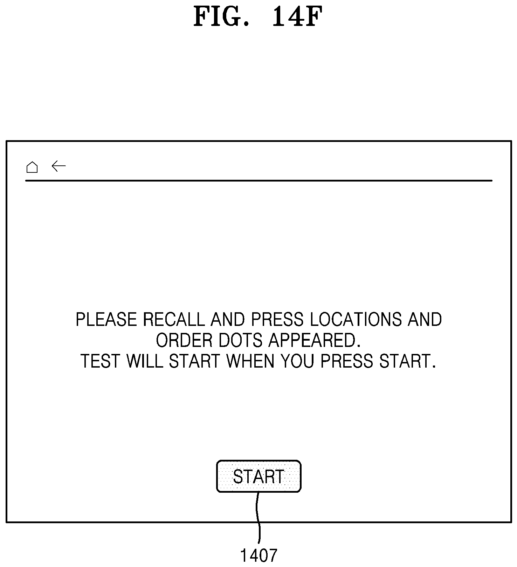

FIG. 14F illustrates a screen indicating the (10-1)th instruction and a start of the (10-1)th test process. When a start button 1407 is input, a next screen may be displayed.

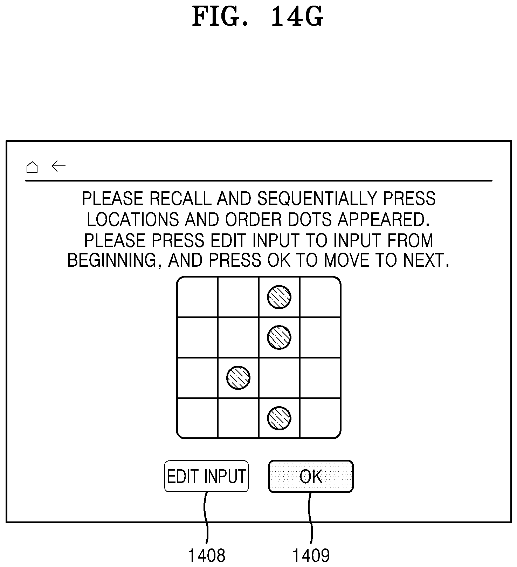

FIG. 14G illustrates a screen for performing the (10-1)th test process. FIG. 14G illustrates a screen displayed after the first demonstration video is reproduced. The user may recall the first demonstration video and sequentially input the locations and order the presented figures appeared in the pre-set region. When the input needs to be edited, the user may input an edit input button 1408 and edit the input. When the input is completed, the user may input an OK button 1409.

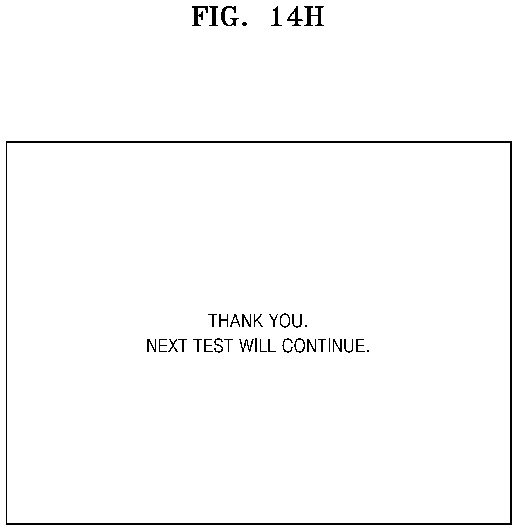

FIG. 14H illustrates a screen indicating completion of the performing of the (10-1)th test process and a start of a next test process.

FIG. 14I illustrates a screen indicating a start of a practice test process ((10-2)th practice test process) for the (10-2)th test process. When a practice start button 1410 is input, a next screen may be displayed.

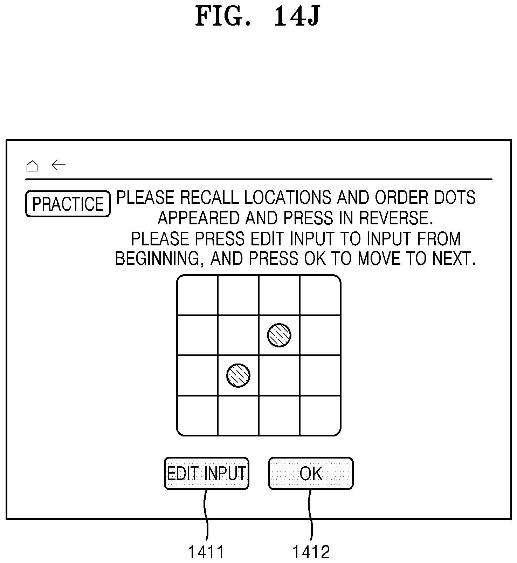

FIG. 14J illustrates an embodiment of a screen for performing the (10-2)th practice test process. FIG. 14J illustrates a second practice demonstration video together with a (10-2)th practice instruction. Also, an edit input button 1411 for editing an input and an OK button 1412 for completing the input may also be provided.

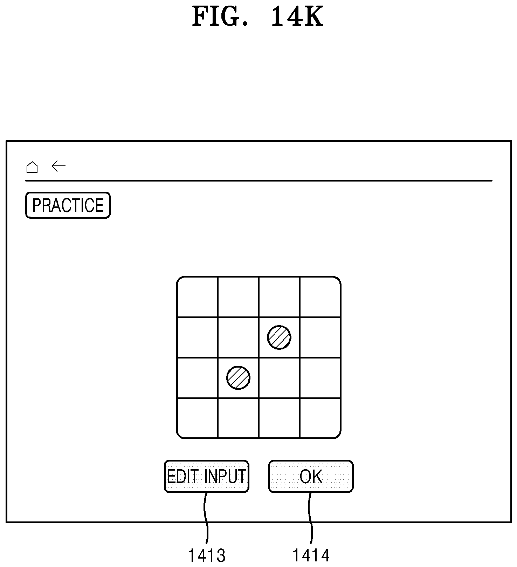

FIG. 14K illustrates another embodiment of a screen for performing the (10-2)th practice test process. The user may recall the second practice demonstration video of FIG. 14J and input, in reverse, the locations and order the presented figures appeared in the pre-set region. When the input needs to be edited, the user may input an edit input button 1413 and edit the input. When the input is completed, the user may input an OK button 1414.

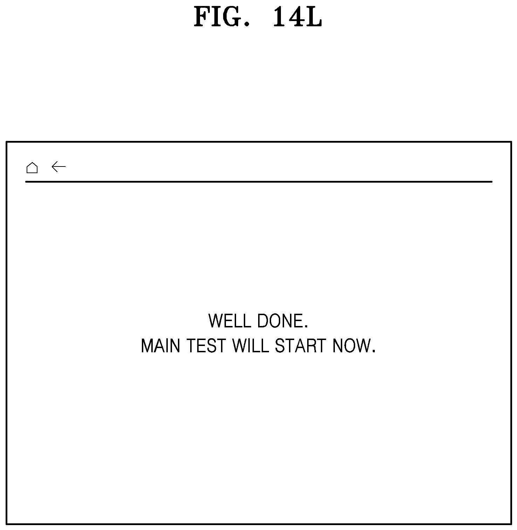

FIG. 14L illustrates a screen indicating completion of the performing of the (10-2)th practice test process, and performing of a main test.

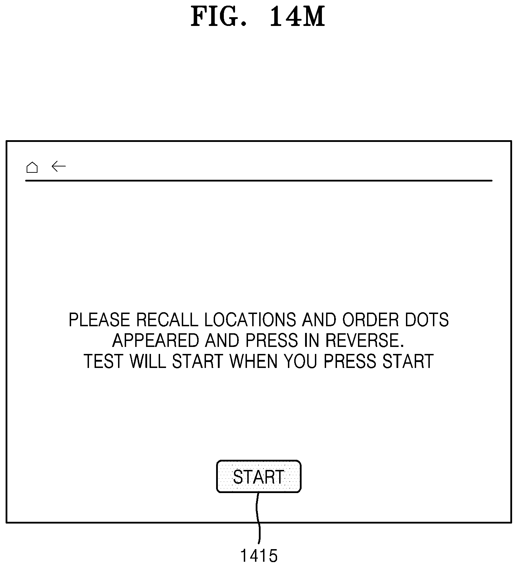

FIG. 14M illustrates a screen indicating the (10-2)th instruction and a start of the (10-2)th test process. When a start button 1415 is input, a next screen may be displayed.

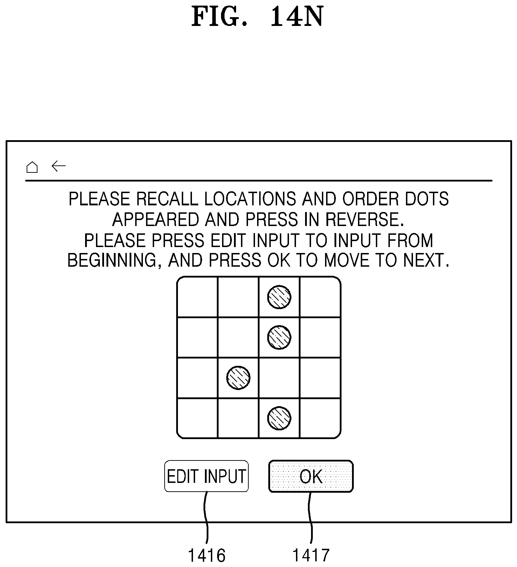

FIG. 14N illustrates a screen for performing the (10-2)th test process. FIG. 14N illustrates a screen displayed after the second demonstration video is reproduced. The user may recall the second demonstration video and input, in reverse, the locations and order the presented figures appeared in the pre-set region. When the input needs to be edited, the user may input an edit input button 1416 and edit the input. When the input is completed, the user may input an OK button 1417.

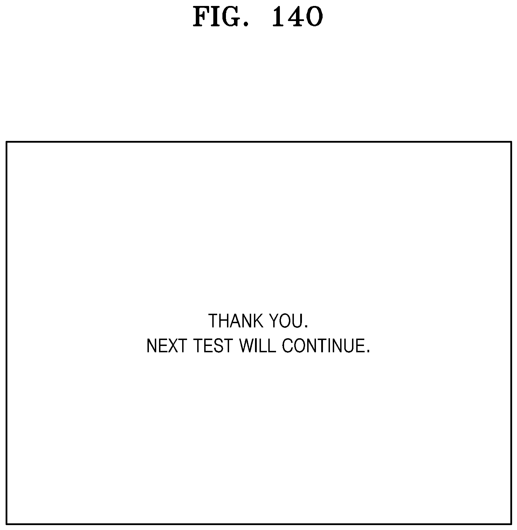

FIG. 14O illustrates a screen indicating completion of the performing of the (10-2)th test process and a start of a next test process.

Referring to FIG. 3, the first processing unit 152 may perform the eleventh test process including the (11-1)th test process and the (11-2)th test process. According to the present embodiment, the first processing unit 152 may perform the eleventh test process a plurality of times, excluding a practice test.

When the (11-1)th test process is performed, the first processing unit 152 may illustrate a first presented incomplete sentence, in which a word is omitted, and two first choice words, and provide, to the user terminal 200, the (11-1)th instruction, in which it is instructed to choose an accurate word for completing the first presented incomplete sentence from among the two first choice words.

The first processing unit 152 may receive, from the user terminal 200, a result of choosing an accurate word for completing the first presented incomplete sentence from among the two first choice words, in response to the (11-1)th instruction, and calculate a (11-1)th test score by comparing the result with a correct answer. In the present embodiment, the (11-1)th test score may be calculated to be high when there are many results determined to be correct answers compared to the total number of times the (11-1)th test processes have been performed.

In the present embodiment, before the (11-1)th test process is performed, the first processing unit 152 may provide, in a text, a method of performing the (11-1)th test process to the user terminal 200 and perform a practice test for the (11-1)th test process.

The first processing unit 152 may perform the (11-2)th test process after the performing of the (11-1)th test process is completed.

When the (11-2)th test process is performed, the first processing unit 152 may illustrate a second presented incomplete sentence, in which a word is omitted, and two second choice words, and provide, to the user terminal 200, the (11-2)th instruction, in which it is instructed to choose an awkward word for completing the second presented incomplete sentence from among the two second choice words.

The first processing unit 152 may receive, from the user terminal 200, a result of choosing an awkward word for completing the second presented incomplete sentence from among the two second choice words, in response to the (11-2)th instruction, and calculate a (11-2)th test score by comparing the result with a correct answer. In the present embodiment, the (11-2)th test score may be calculated to be high when there are many results determined to be correct answers compared to the total number of times the (11-2)th test processes have been performed.

In the present embodiment, before the (11-2)th test process is performed, the first processing unit 152 may provide, in a text, a method of performing the (11-2)th test process to the user terminal 200 and perform a practice test for the (11-2)th test process.

According to a selective embodiment, the first processing unit 152 may calculate the eleventh test score by adding the (11-1)th test score and the (11-2)th test score. Alternatively, the first processing unit 152 may not add the (11-1)th test score and the (11-2)th test score, but may use the (11-1)th test score and the (11-2)th test score each to evaluate cognitive function decline. In the present embodiment, it may be evaluated that language control is good when the eleventh test score is higher than the eleventh criterion cut-off score, and that the language control is declined when the eleventh test score is lower than the eleventh criterion cut-off score.

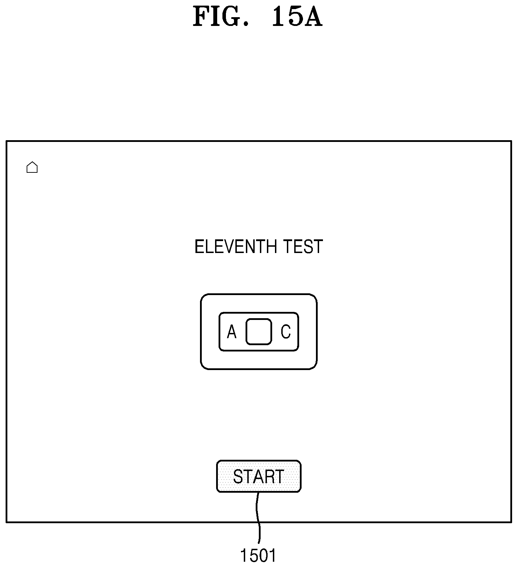

FIGS. 15A through 15K illustrate examples of screens provided to the user terminal 200 to perform the eleventh test process.

FIG. 15A illustrates a start screen of the eleventh test process. When an input of a start button 1501 is received, a next screen may be displayed.

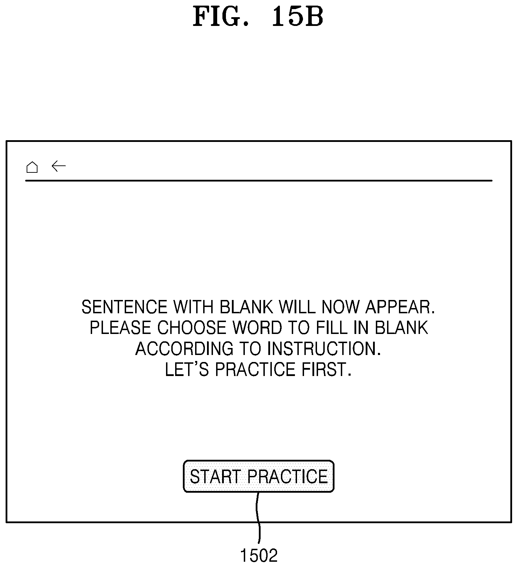

FIG. 15B illustrates a screen in which a method of performing the (11-1)th test process is provided in a text and a start of a practice test process (eleventh practice test process) for the (11-1)th test process is notified. When a practice start button 1502 is input, a next screen may be displayed.

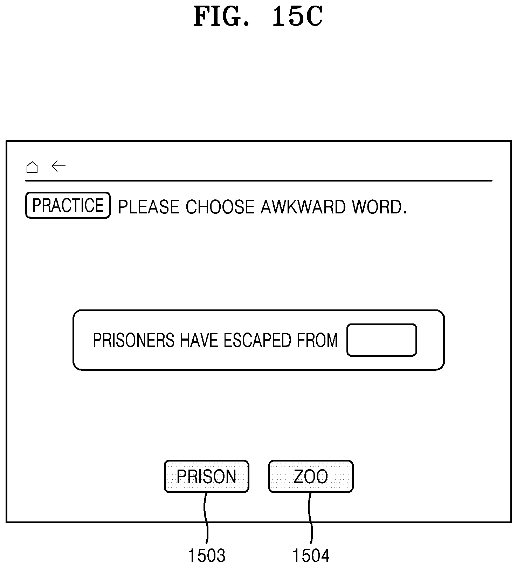

FIG. 15C illustrates an embodiment of a screen for performing a (11-2)th practice test process among the eleventh practice test process. FIG. 15C illustrates a (11-2)th practice instruction, a second practice presented incomplete sentence, and a first word button 1503 and a second word button 1504 for completing the second practice presented incomplete sentence.

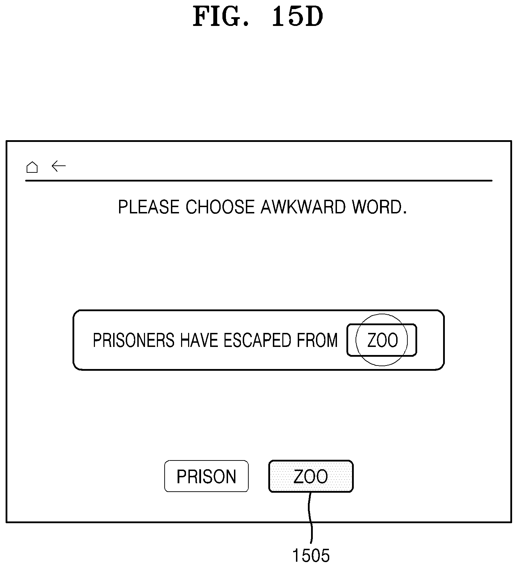

FIG. 15D illustrates a second word button 1505 chosen by the user from the screen of FIG. 15C, in response to the (11-2)th practice instruction. When the chosen word is a correct answer, a correct answer sign and/or a correct answer sound effect may be used to indicate the correct answer. Also, when the chosen word is a wrong answer, a wrong answer sign and/or a wrong answer sound effect may be used to indicate the wrong answer.

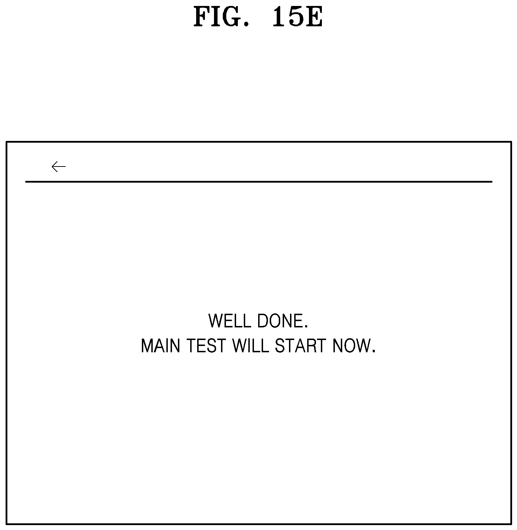

FIG. 15E illustrates a screen indicating completion of the performing of the eleventh practice test process, and performing of a main test.

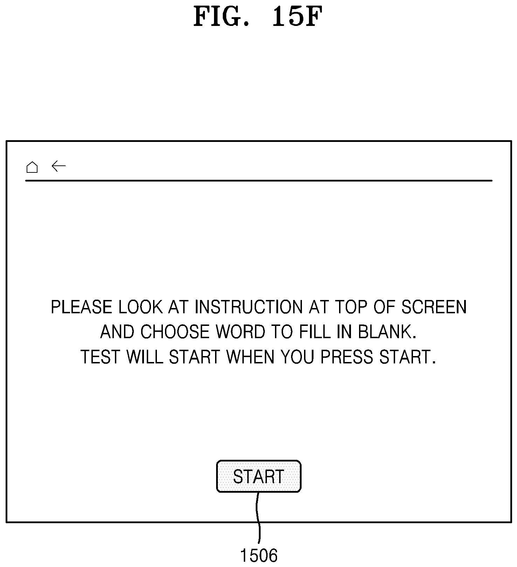

FIG. 15F illustrates a screen indicating a start of the eleventh test process. When a start button 1506 is input, a next screen may be displayed.

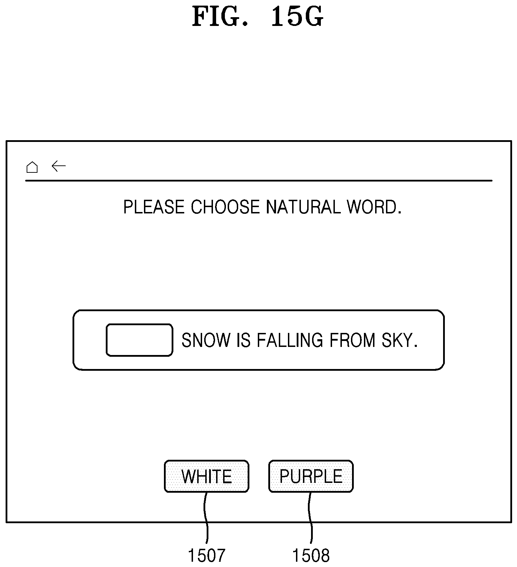

FIG. 15G illustrates an embodiment of a screen for performing the (11-1)th test process. FIG. 15G illustrates the (11-1)th instruction, a first presented incomplete sentence, and a first word button 1507 and a second word button 1508 for completing the first presented incomplete sentence.

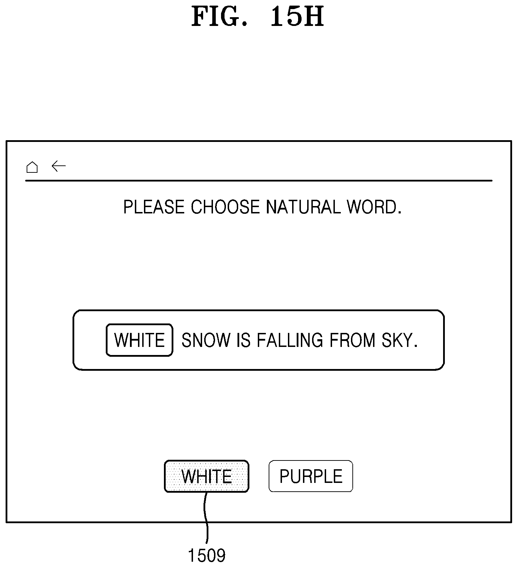

FIG. 15H illustrates a first word button 1509 chosen by the user from the screen of FIG. 15G, in response to the (11-1)th instruction.

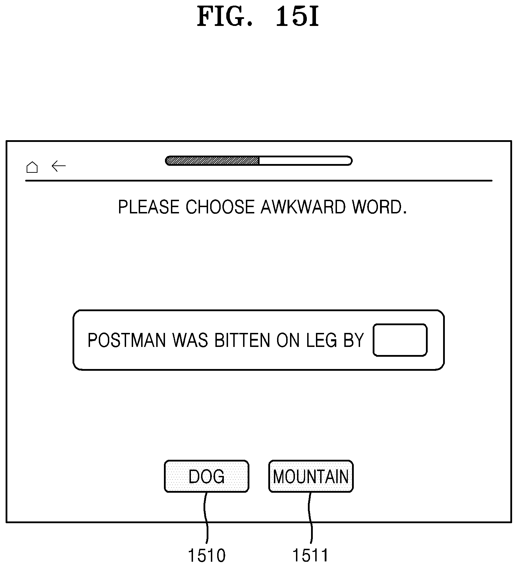

FIG. 15I illustrates an embodiment of a screen for performing the (11-2)th test process. FIG. 15I illustrates the (11-2)th instruction, a second presented incomplete sentence, and a first word button 1510 and a second word button 1511 for completing the second presented incomplete sentence.

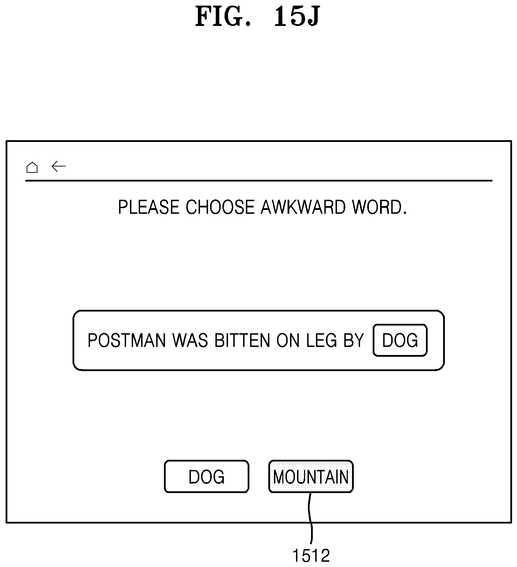

FIG. 15J illustrates a second word button 1512 chosen by the user from the screen of FIG. 15I, in response to the (11-2)th instruction.

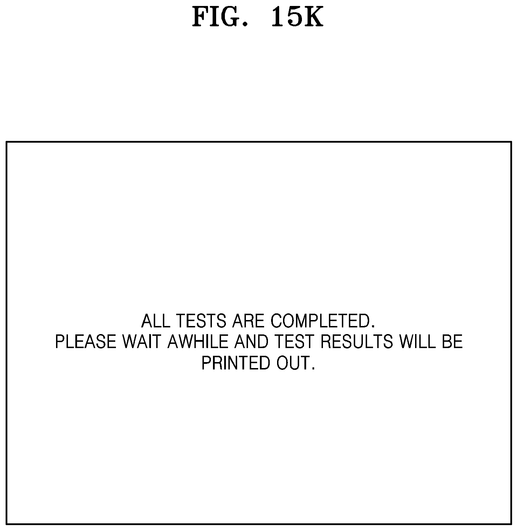

FIG. 15K illustrates a screen indicating completion of the performing of the eleventh test process and to stand by for test result printouts.

Referring back to FIG. 3, when the performing of the first test process through the eleventh test process by the first processing unit 152 is completed, the second processing unit 153 may perform the category classification process of classifying at least one test item from among a plurality of test items as one neurocognitive category from among a plurality of neurocognitive categories.

The second processing unit 153 may classify the second test item and the third test item as the first neurocognitive category. The second processing unit 153 may classify the eighth test item as the second neurocognitive category. The second processing unit 153 may classify the seventh test item and the ninth test item as the third neurocognitive category. The second processing unit 153 may classify the fifth test item and the sixth test item as the fourth neurocognitive category. The second processing unit 153 may classify the first test item, the fourth test item, the tenth test item, and the eleventh test item as the fifth neurocognitive category.

When the category classification process is completed, the second processing unit 153 may perform the evaluation process of evaluating whether there is cognitive function decline in a neurocognitive category, based on a test score of a test item classified as the neurocognitive category.

In the present embodiment, when it is evaluated that there is cognitive function decline in at least one of the first neurocognitive category through the fifth neurocognitive category, the third processing unit 154 may generate an evaluation result outputting that the user may have mild cognitive impairment.

In the present embodiment, the evaluation process may include the first evaluation process through the fifth evaluation process.

The third processing unit 154 may perform the first evaluation process of evaluating whether there is cognitive function decline in the first neurocognitive category, based on the second test score and the third test score.

During the first evaluation process, the third processing unit 154 may compare the second test score for the second test item with a second reference score, and generate a first comparison result when the second test score is less than the second reference score. In the present embodiment, the second test score may include a language clue memory test score. In the present embodiment, the second reference score may include a second test score of a normal group, in which a second Z-score is a second standard deviation (for example, -1.5 SD).

In the present embodiment, the first comparison result may include a (1-1)th comparison result and a (1-2)th comparison result. The (1-1)th comparison result may be generated when the second test score is less than the second reference score. It may be determined that the language memory of the user is declined, based on the (1-1)th comparison result. The (1-2)th comparison result may be generated when the second test score is equal to or greater than the second reference score. It may be determined that the language memory of the user is good, based on the (1-2)th comparison result. In the present embodiment, the first comparison result may be replaced by the (1-1)th comparison result.

The third processing unit 154 may compare the third test score for the third test item with a third reference score, and generate a second comparison result when the third test score is less than the third reference score. In the present embodiment, the third test score may include a visual graphic memory test score. In the present embodiment, the third reference score may include a third test score of a normal group, in which a third Z-score is a third standard deviation (for example, -1.5 SD).

In the present embodiment, the second comparison result may include a (2-1)th comparison result and a (2-2)th comparison result. The (2-1)th comparison result may be generated when the third test score is less than the third reference score. It may be determined that the visual perception memory of the user is declined, based on the (2-1)th comparison result. The (2-2)th comparison result may be generated when the third test score is equal to or greater than the third reference score. It may be determined that the visual perception memory of the user is good, based on the (2-2)th comparison result. In the present embodiment, the second comparison result may be replaced by the (2-1)th comparison result.

The third processing unit 154 may evaluate that there is cognitive function decline in the first neurocognitive category, i.e., memory, when at least one of the first comparison result and the second comparison result is generated.

The third processing unit 154 may perform the second evaluation process of evaluating whether there is cognitive function decline in the second neurocognitive category, based on the eighth test score.

During the second evaluation process, the third processing unit 154 may compare the eighth test score for the eighth test item with an eighth reference score, and generate a third comparison result when the eighth test score is less than the eighth reference score. In the present embodiment, the eighth test score may include a face emotion test score. In the present embodiment, the eighth reference score may include an eighth test score of a normal group, in which an eighth Z-score is an eighth standard deviation (for example, -1.5 SD).

In the present embodiment, the third comparison result may include a (3-1)th comparison result and a (3-2)th comparison result. The (3-1)th comparison result may be generated when the eighth test score is less than the eighth reference score. It may be determined that the emotion intensity cognitive power of the user is declined, based on the (3-1)th comparison result. The (3-2)th comparison result may be generated when the eighth test score is equal to or greater than the eighth reference score. It may be determined that the emotion intensity cognitive power of the user is good, based on the (3-2)th comparison result. In the present embodiment, the third comparison result may be replaced by the (3-1)th comparison result.

The third processing unit 154 may evaluate that there is cognitive function decline in the second neurocognitive category, i.e., social cognition, when the third comparison result is generated.

The third processing unit 154 may perform the third evaluation process of evaluating whether there is cognitive function decline in the third neurocognitive category, based on the seventh test score and the ninth test score.

During the third evaluation process, the third processing unit 154 may compare the seventh test score for the seventh test item with a seventh reference score, and generate a fourth comparison result when the seventh test score is less than the seventh reference score. In the present embodiment, the seventh test score may include a size weight test score. In the present embodiment, the seventh reference score may include a seventh test score of a normal group, in which a seventh Z-score is a seventh standard deviation (for example, -1.5 SD).

In the present embodiment, the fourth comparison result may include a (4-1)th comparison result and a (4-2)th comparison result. The (4-1)th comparison result may be generated when the seventh test score is less than the seventh reference score. It may be determined that the semantic memory of the user is declined, based on the (4-1)th comparison result. The (4-2)th comparison result may be generated when the seventh test score is equal to or greater than the seventh reference score. It may be determined that the semantic memory of the user is good, based on the (4-2)th comparison result. In the present embodiment, the fourth comparison result may be replaced by the (4-1)th comparison result.

The third processing unit 154 may compare the ninth test score for the ninth test item with a ninth reference score, and generate a fifth comparison result when the ninth test score is less than the ninth reference score. In the present embodiment, the ninth test score may include a paragraph reading test score. In the present embodiment, the ninth reference score may include a ninth test score of a normal group, in which a ninth Z-score is a ninth standard deviation (for example, -1.5 SD).

In the present embodiment, the fifth comparison result may include a (5-1)th comparison result and a (5-2)th comparison result. The (5-1)th comparison result may be generated when the ninth test score is less than the ninth reference score. It may be determined that the reading ability of the user is declined, based on the (5-1)th comparison result. The (5-2)th comparison result may be generated when the ninth test score is equal to or greater than the ninth reference score. It may be determined that the reading ability of the user is good, based on the (5-2)th comparison result. In the present embodiment, the fifth comparison result may be replaced by the (5-1)th comparison result.

The third processing unit 154 may evaluate that there is cognitive function decline in the third neurocognitive category, i.e., language, when at least one of the fourth comparison result and the fifth comparison result is generated.

The third processing unit 154 may perform the fourth evaluation process of evaluating whether there is cognitive function decline in the fourth neurocognitive category, based on the fifth test score and the sixth test score.

During the fourth evaluation process, the third processing unit 154 may compare the fifth test score for the fifth test item with a fifth reference score, and generate a sixth comparison result when the fifth test score is less than the fifth reference score. In the present embodiment, the fifth test score may include a line angle perception test score. In the present embodiment, the fifth reference score may include a fifth test score of a normal group, in which a fifth Z-score is a fifth standard deviation (for example, -1.5 SD).

In the present embodiment, the sixth comparison result may include a (6-1)th comparison result and a (6-2)th comparison result. The (6-1)th comparison result may be generated when the fifth test score is less than the fifth reference score. It may be determined that the visuospatial perception of the user is declined, based on the (6-1)th comparison result. The (6-2)th comparison result may be generated when the fifth test score is equal to or greater than the fifth reference score. It may be determined that the visuospatial perception of the user is good, based on the (6-2)th comparison result. In the present embodiment, the sixth comparison result may be replaced by the (6-1)th comparison result.

The third processing unit 154 may compare the sixth test score for the sixth test item with a sixth reference score, and generate a seventh comparison result when the sixth test score is less than the sixth reference score. In the present embodiment, the sixth test score may include a color perception test score. In the present embodiment, the sixth reference score may include a sixth test score of a normal group, in which a sixth Z-score is a sixth standard deviation (for example, -1.5 SD).

In the present embodiment, the seventh comparison result may include a (7-1)th comparison result and a (7-2)th comparison result. The (7-1)th comparison result may be generated when the sixth test score is less than the sixth reference score. It may be determined that the color classifying ability of the user is declined, based on the (7-1)th comparison result. The (7-2)th comparison result may be generated when the sixth test score is equal to or greater than the sixth reference score. It may be determined that the color classifying ability of the user is good, based on the (7-2)th comparison result. In the present embodiment, the seventh comparison result may be replaced by the (7-1)th comparison result.

The third processing unit 154 may evaluate that there is cognitive function decline in the fourth neurocognitive category, i.e., perception, when at least one of the sixth comparison result and the seventh comparison result is generated.

The third processing unit 154 may perform the fifth evaluation process of evaluating whether there is cognitive function decline in the fifth neurocognitive category, based on the first test score, the fourth test score, the tenth test score, and the eleventh test score.

During the fifth evaluation process, the third processing unit 154 may compare the first test score for the first test item with a first reference score, and generate an eighth comparison result when the first test score is less than the first reference score. In the present embodiment, the first test score may include a fluency test score. In the present embodiment, the first reference score may include a first test score of a normal group, in which a first Z-score is a first standard deviation (for example, -1.5 SD).

In the present embodiment, the eighth comparison result may include a (8-1)th comparison result and a (8-2)th comparison result. The (8-1)th comparison result may be generated when the first test score is less than the first reference score. It may be determined that the fluency of the user is declined, based on the (8-1)th comparison result. The (8-2)th comparison result may be generated when the first test score is equal to or greater than the first reference score. It may be determined that the fluency of the user is good, based on the (8-2)th comparison result. In the present embodiment, the eighth comparison result may be replaced by the (8-1)th comparison result.

The third processing unit 154 may compare the fourth test score for the fourth test item with a fourth reference score, and generate a ninth comparison result when the fourth test score is less than the fourth reference score. In the present embodiment, the fourth test score may include a Stroop test score. In the present embodiment, the fourth reference score may include a fourth test score of a normal group, in which a fourth Z-score is a fourth standard deviation (for example, -1.5 SD).

In the present embodiment, the ninth comparison result may include a (9-1)th comparison result and a (9-2)th comparison result. The (9-1)th comparison result may be generated when the fourth test score is less than the fourth reference score. It may be determined that the inhibitory function of the user is declined, based on the (9-1)th comparison result. The (9-2)th comparison result may be generated when the fourth test score is equal to or greater than the fourth reference score. It may be determined that the inhibitory function of the user is good, based on the (9-2)th comparison result. In the present embodiment, the ninth comparison result may be replaced by the (9-1)th comparison result.

The third processing unit 154 may compare the tenth test score for the tenth test item with a tenth reference score, and generate a tenth comparison result when the tenth test score is less than the tenth reference score. In the present embodiment, the tenth test score may include a visuospatial attention test score. In the present embodiment, the tenth reference score may include a tenth test score of a normal group, in which a tenth Z-score is a tenth standard deviation (for example, -1.5 SD).

In the present embodiment, the tenth comparison result may include a (10-1)th comparison result and a (10-2)th comparison result. The (10-1)th comparison result may be generated when the tenth test score is less than the tenth reference score. It may be determined that the attention and working memory of the user are declined, based on the (10-1)th comparison result. The (10-2)th comparison result may be generated when the tenth test score is equal to or greater than the tenth reference score. It may be determined that the attention and working memory of the user are good, based on the (10-2)th comparison result. In the present embodiment, the tenth comparison result may be replaced by the (10-1)th comparison result.

The third processing unit 154 may compare the eleventh test score for the eleventh test item with an eleventh reference score, and generate an eleventh comparison result when the eleventh test score is less than the eleventh reference score. In the present embodiment, the eleventh test score may include a Hayling test score. In the present embodiment, the eleventh reference score may include an eleventh test score of a normal group, in which an eleventh Z-score is an eleventh standard deviation (for example, -1.5 SD).

In the present embodiment, the eleventh comparison result may include a (11-1)th comparison result and a (11-2)th comparison result. The (11-1)th comparison result may be generated when the eleventh test score is less than the eleventh reference score. It may be determined that the language control of the user is declined, based on the (11-1)th comparison result. The (11-2)th comparison result may be generated when the eleventh test score is equal to or greater than the eleventh reference score. It may be determined that the language control of the user is good, based on the (11-2)th comparison result. In the present embodiment, the eleventh comparison result may be replaced by the (11-1)th comparison result.

The third processing unit 154 may evaluate that there is cognitive function decline in the fifth neurocognitive category, i.e., attention execution, when at least one of the eighth comparison result, the ninth comparison result, the tenth comparison result, and the eleventh comparison result is generated.

After completing the evaluation process, the transmitting unit 155 may transmit the evaluation result to the user terminal 200 and/or a terminal (not shown) of a practitioner (for example, a doctor).

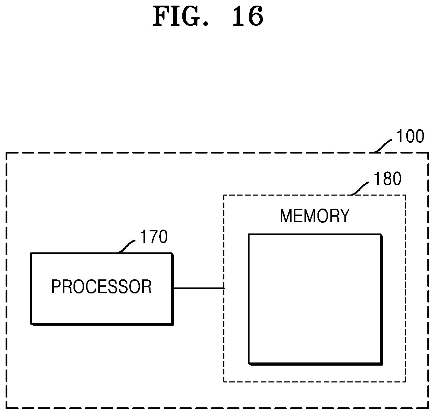

FIG. 16 is a block diagram for schematically describing a configuration of the test apparatus 100 for evaluating cognitive function decline, according to another embodiment. Hereinafter, descriptions about details that overlap those of FIGS. 1 through 15 are omitted. Referring to FIG. 16, the test apparatus 100 according to another embodiment may include a processor 170 and a memory 180.

In the present embodiment, the processor 170 may process functions performed by the communication unit 110, the storage medium 120, the program storage unit 130, the database 140, the test management unit 150, and the control unit 160 shown in FIGS. 2 and 3.

The processor 170 may control all operations of the test apparatus 100. Here, the processor may denote a hardware-embedded data processing device including a physically structured circuit to perform a function represented by an instruction or a code included in a program. Examples of the hardware-embedded data processing device may include processing devices, such as a microprocessor, a central processing unit, a processor core, a multiprocessor, ASIC, and FPGA, but the scope of the disclosure is not limited thereto.

The memory 180 may be operatively connected to the processor 170, and may store at least one code in association with an operation performed by the processor 170.

Also, the memory 180 may temporarily or permanently store data processed by the processor 170, and may include data built in the database 140. Here, the memory 180 may include a magnetic storage medium or a flash storage medium, but the scope of the disclosure is not limited thereto. The memory 180 may include an internal memory and/or an external memory, and may include a volatile memory, such as a DRAM, an SRAM, or an SDRAM, a non-volatile memory, such as an OTPROM, a PROM, an EPROM, an EEPROM, a mask ROM, a flash ROM, an NAND flash memory, or a NOR flash memory, a flash drive, such as an SSD, a CF card, an SD card, a micro-SD card, a mini-SD card, an XD card, or a memory stick, or a storage device, such as an HDD.

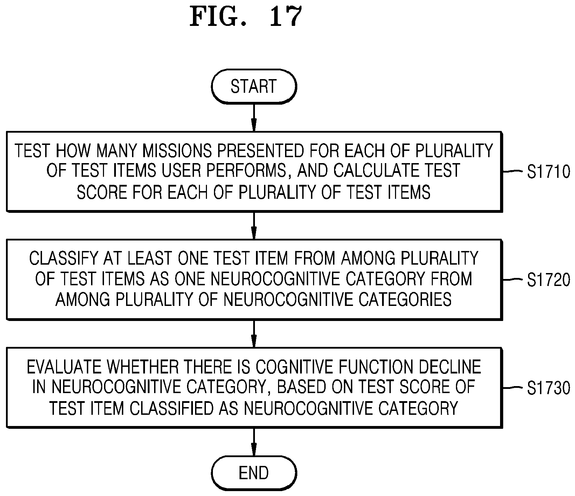

FIGS. 17 through 20 are flowcharts of test methods for evaluating cognitive function decline, according to embodiments. Hereinafter, descriptions about details that overlap those of FIGS. 1 through 16 are omitted. The test method for evaluating cognitive function decline, according to the present embodiment will be described based on an assumption that the test apparatus 100 performs the test method in the processor 170 with the help of peripheral components.

Referring to FIG. 17, in operation S1710, the processor 170 may test how many missions presented for each of the plurality of test items the user performs, and calculate the test score for each of the plurality of test items. In the present embodiment, operation S1710 may be referred to as a testing stage in the claims below.

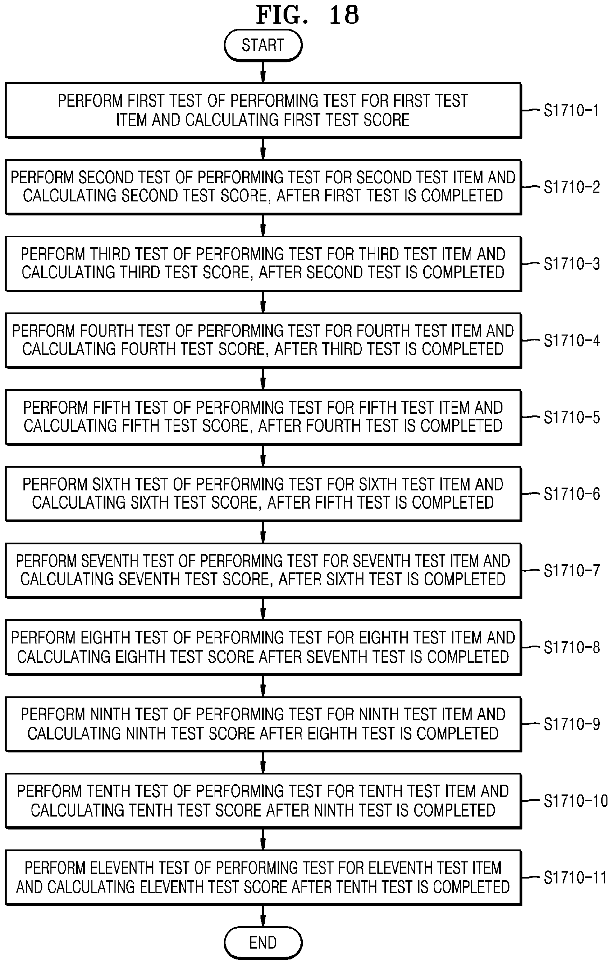

FIG. 18 illustrates a detailed flowchart of the testing stage. Referring to FIG. 18, in operation S1710-1, the processor 170 may perform a first testing stage of performing a test for the first test item, in which it is evaluated how many words corresponding to a pre-set presented category are uttered within a time limit and how much an instructed design is drawn within the time limit, and calculating the first test score.

In operation S1710-2, the processor 170 may perform a second testing stage of performing a test for the second test item, in which words are learned while instructing a pre-set category clue and it is evaluated how many learned words are recalled after a pre-set time, and calculating the second test score, after the first testing stage is completed.

In operation S1710-3, the processor 170 may perform a third testing stage of performing a test for the third test item, in which pre-set presented images are learned and it is evaluated how many learned presented images are recalled after a pre-set time, and calculating the third test score, after the second testing stage is completed.

In operation S1710-4, the processor 170 may perform a fourth testing stage of performing a test for the fourth test item, in which it is evaluated whether a pre-set presented word and a color of the pre-set presented word are accurately uttered, and calculating the fourth test score, after the third testing stage is completed.

In operation S1710-5, the processor 170 may perform a fifth testing stage of performing a test for the fifth test item, in which it is evaluated whether a line having a similar angle as a pre-set presented line is found, and calculating the fifth test score, after the fourth testing stage is completed.

In operation S1710-6, the processor 170 may perform a sixth testing stage of performing a test for the sixth test item, in which it is evaluated whether a figure of a color different from a color of a pre-set presented figure is found, and calculating the sixth test score, after the fifth testing stage is completed.

In operation S1710-7, the processor 170 may perform a seventh testing stage of performing a test for the seventh test item, in which it is evaluated whether sizes and weights of pre-set presented objects are arranged in a descending order, and calculating the seventh test score, after the sixth testing stage is completed.

In operation S1710-8, the processor 170 may perform an eighth testing stage of performing a test for the eighth test item, in which it is evaluated whether intensity of an emotion shown on a pre-set face presented image is identified, and calculating the eighth test score, after the seventh testing stage is completed.

In operation S1710-9, the processor 170 may perform a ninth testing stage of performing a test for the ninth test item, in which it is evaluated whether a pre-set presented sentence is accurately uttered, and calculating the ninth test score, after the eighth testing stage is completed.

In operation S1710-10, the processor 170 may perform a tenth testing stage of performing a test for the tenth test item, in which it is evaluated whether locations and order of pre-set presented figures are recalled, and calculating the tenth test score, after the ninth testing stage is completed.

In operation S1710-11, the processor 170 may perform an eleventh testing stage of performing a test for the eleventh test item, in which it is evaluated whether a word corresponding to a condition is accurately chosen such that a pre-set presented incomplete sentence becomes a complete sentence, and calculating the eleventh test score, after the tenth testing stage is completed.

Referring back to FIG. 17, in operation S1720, the processor 170 may classify at least one test item from among the plurality of test items as one neurocognitive category from among a plurality of neurocognitive categories, after the testing stage is completed. In the present embodiment, operation S1720 may be referred to as a category classifying stage in the claims below.

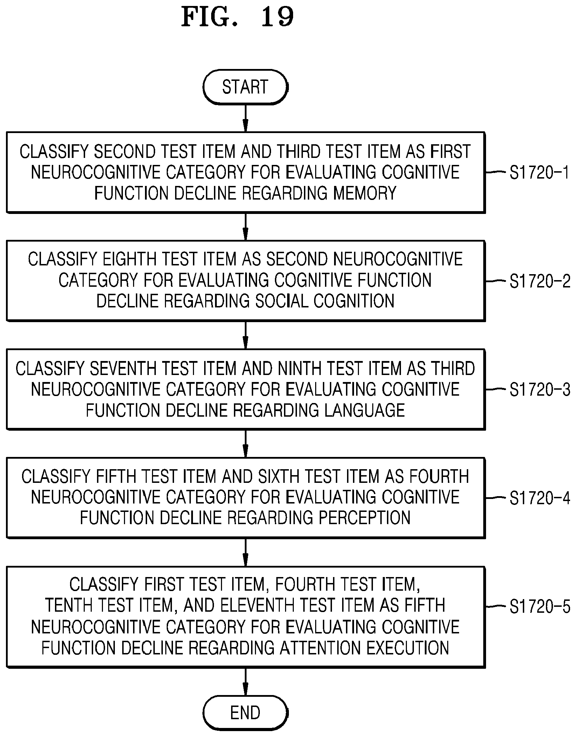

FIG. 19 illustrates a detailed flowchart of the category classifying stage. Referring to FIG. 19, in operation S1720-1, the processor 170 may classify the second test item and the third test item as the first neurocognitive category for evaluating cognitive function decline regarding memory.

In operation S1720-2, the processor 170 may classify the eighth test item as the second neurocognitive category for evaluating cognitive function decline regarding social cognition.

In operation S1720-3, the processor 170 may classify the seventh test item and the ninth test item as the third neurocognitive category for evaluating cognitive function decline regarding language.

In operation S1720-4, the processor 170 may classify the fifth test item and the sixth test item as the fourth neurocognitive category for evaluating cognitive function decline regarding perception.

In operation S1720-5, the processor 170 may classify the first test item, the fourth test item, the tenth test item, and the eleventh test item as the fifth neurocognitive category for evaluating cognitive function decline regarding attention execution.

Referring back to FIG. 17, in operation S1730, the processor 170 may evaluate whether there is cognitive function decline in a neurocognitive category, based on a test score of a test item classified as the neurocognitive category, after the category classifying stage is completed. In the present embodiment, operation S1730 may be referred to as an evaluating stage in the claims below.

In embodiments, if it is determined that there is cognitive function decline in a neurocognitive category, the processor 170 may perform actions for treating the user. For example, the processor 170 may search hospitals that may treat the cognitive function decline based on the location of the user. The location of the user may be determined based on the location of device of the user, such as the user terminal 200 in FIG. 1. The location of the device of the user may be obtained, e.g., by receiving a GPS signal from the user terminal 200. Then, the processor 170 may transmit information about the searched hospitals to the user terminal 200 such that the screen of the user terminal 200 displays the information about the searched hospitals such as locations of the hospitals, doctors at the hospitals, available treatments, available medical devices, and the like. The processor 170 may reserve an appointment with a doctor at one of the hospitals for the user and transmit information about the appointment to the doctor.

As another example, if it is determined that there is cognitive function decline in a neurocognitive category, the processor 170 may determine a suggested test, such as, an MRI exam, a CT exam, a PET exam, a blood test, a Cerebrospinal fluid test based on the test result, and transmit the suggested test to the user terminal 200 such that the screen of the user terminal 200 displays the information about the suggested test. The information about the suggested test may be transmitted to a primary doctor of the user.

As another example, if it is determined that there is cognitive function decline in a neurocognitive category, the processor 170 may provide additional questions to the user, such as questions regarding physical Activity of daily living and instrumental activity of daily living, questions for determining whether the cognitive function decline is due to senile depression, questions for confirming behavioral and psychological Symptom in Dementia.

In embodiments, if it is determined that the user may have a certain disease or a dementia based on the test results, the processor 170 may formulate additional tests customized for the user. For example, if the user is suspected to have Parkinson disease, Parkinson disease dementia, or Lewy body dementia, the processor 170 may perform a color vision test to check the user's ability to distinguish between colors. As another example, if the user is suspected to have frontotemporal dementia, the processor 170 may perform additional tests such as a theory of mind test, a facial emotion test, and the like, in order to more accurately evaluate the level of social cognition of the user.

In embodiments, after the processor 170 provides suggestions for treating the cognitive function decline, the processor 170 deletes the calculated scores and the evaluation results.

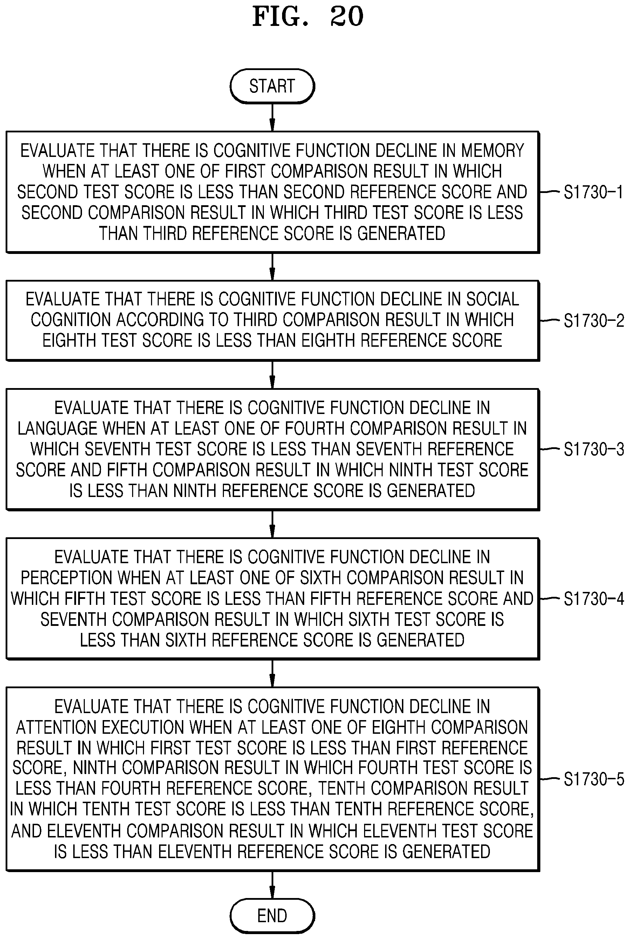

FIG. 20 illustrates a detailed flowchart of the evaluating stage. Referring to FIG. 20, in operation S1730-1, the processor 170 may perform the first evaluation process. During the first evaluation process, the processor 170 may compare the second test score for the second test item with the second reference score, and generate the first comparison result when the second test score is less than the second reference score. The processor 170 may compare the third test score for the third test item with the third reference score, and generate the second comparison result when the third test score is less than the third reference score. The processor 170 may evaluate that there is cognitive function decline in the first neurocognitive category, i.e., memory, when at least one of the first comparison result and the second comparison result is generated.

When evaluating whether there is cognitive function decline in the memory, only the second test item and/or the third test item are used from among the first test item through the eleventh test item, and thus an evaluation result may be calculated by using lesser computer resources and higher processing speed compared to resources and a processing speed when all of the first test item through the eleventh test item are used, and accuracy and reliability of cognitive function decline evaluation may be increased.

In operation S1730-2, the processor 170 may perform the second evaluation process. During the second evaluation process, the processor 170 may compare the eighth test score for the eighth test item with the eighth reference score, and generate the third comparison result when the eighth test score is less than the eighth reference score. The processor 170 may evaluate that there is cognitive function decline in the second neurocognitive category, i.e., social cognition, when the third comparison result is generated.

When evaluating whether there is cognitive function decline in the social cognition, only the eighth test item is used from among the first test item through the eleventh test item, and thus an evaluation result may be calculated by using lesser computer resources and higher processing speed compared to the resources and the processing speed when all of the first test item through the eleventh test item are used, and the accuracy and the reliability of the cognitive function decline evaluation may be increased. If it is determined that there is cognitive function decline in the social cognition, the processor 170 may perform additional tests such as a theory of mind test, a facial emotion test, and the like, in order to more accurately evaluate the level of social cognition of the user.

In operation S1730-3, the processor 170 may perform the third evaluation process. During the third evaluation process, the processor 170 may compare the seventh test score for the seventh test item with the seventh reference score, and generate the fourth comparison result when the seventh test score is less than the seventh reference score. The processor 170 may compare the ninth test score for the ninth test item with the ninth reference score, and generate the fifth comparison result when the ninth test score is less than the ninth reference score. The processor 170 may evaluate that there is cognitive function decline in the third neurocognitive category, i.e., language, when at least one of the fourth comparison result and the fifth comparison result is generated.

When evaluating whether there is cognitive function decline in the language, only the seventh test item and/or the ninth test item are used from among the first test item through the eleventh test item, and thus an evaluation result may be calculated by using lesser computer resources and higher processing speed compared to the resources and the processing speed when all of the first test item through the eleventh test item are used, and the accuracy and the reliability of the cognitive function decline evaluation may be increased.

In operation S1730-4, the processor 170 may perform the fourth evaluation process. During the fourth evaluation process, the processor 170 may compare the fifth test score for the fifth test item with the fifth reference score, and generate the sixth comparison result when the fifth test score is less than the fifth reference score. The processor 170 may compare the sixth test score for the sixth test item with the sixth reference score, and generate the seventh comparison result when the sixth test score is less than the sixth reference score. The processor 170 may evaluate that there is cognitive function decline in the fourth neurocognitive category, i.e., perception, when at least one of the sixth comparison result and the seventh comparison result is generated.

When evaluating whether there is cognitive function decline in the perception, only the fifth test item and/or the sixth test item are used from among the first test item through the eleventh test item, and thus an evaluation result may be calculated by using lesser computer resources and higher processing speed compared to the resources and the processing speed when all of the first test item through the eleventh test item are used, and the accuracy and the reliability of the cognitive function decline evaluation may be increased.

In operation S1730-5, the processor 170 may perform the fifth evaluation process. During the fifth evaluation process, the third processor 170 may compare the first test score for the first test item with the first reference score, and generate the eighth comparison result when the first test score is less than the first reference score. The processor 170 may compare the fourth test score for the fourth test item with the fourth reference score, and generate the ninth comparison result when the fourth test score is less than the fourth reference score. The processor 170 may compare the tenth test score for the tenth test item with the tenth reference score, and generate the tenth comparison result when the tenth test score is less than the tenth reference score. The processor 170 may compare the eleventh test score for the eleventh test item with the eleventh reference score, and generate the eleventh comparison result when the eleventh test score is less than the eleventh reference score. The processor 170 may evaluate that there is cognitive function decline in the fifth neurocognitive category, i.e., attention execution, when at least one of the eighth comparison result, the ninth comparison result, the tenth comparison result, and the eleventh comparison result is generated.

When evaluating whether there is cognitive function decline in the attention execution, only the first test item, the fourth test item, the tenth test item, and/or the eleventh test item are used from among the first test item through the eleventh test item, and thus an evaluation result may be calculated by using lesser computer resources and higher processing speed compared to the resources and the processing speed when all of the first test item through the eleventh test item are used, and the accuracy and the reliability of the cognitive function decline evaluation may be increased.

The embodiments according to the disclosure described above may be implemented in the form of a computer program executable by various components on a computer, and such a computer program may be recorded on a computer-readable medium. Here, the computer-readable medium may include hardware devices specially designed to store and execute program instructions, such as magnetic media, such as a hard disk, a floppy disk, and a magnetic tape, optical recording media, such as CD-ROM and DVD, magneto-optical media such as a floptical disk, and read-only memory (ROM), random-access memory (RAM), and a flash memory.

The computer program may be specially designed for the disclosure or well known to one of ordinary skill in the computer software field. Examples of the computer program include not only machine codes generated by a compiler, but also high-level language codes executable by a computer by using an interpreter or the like.

The term "the" and similar referential terms in the specification (specifically in the claims) of the disclosure may be used for both the singular and the plural. Further, when a range is described in the disclosure, the disclosure includes inventions to which individual values belonging to the range are applied (unless otherwise stated), and it is considered that each individual value configuring the range is described in the detailed description of the disclosure.

Unless an order is clearly stated or unless otherwise stated, operations configuring a method according to the disclosure may be performed in an appropriate order. The disclosure is not necessarily limited by an order the operations are described. In the disclosure, the use of all examples or exemplary terms (for example, "etc.") is merely for describing the disclosure in detail and the scope of the disclosure is not limited by those examples or exemplary terms unless defined in the claims. Also, it would be obvious to one of ordinary skill in the art that various modifications, combinations, and changes may be configured according to design conditions and factors within the scope of claims or equivalents.

Therefore, the spirit of the disclosure should not be determined limitedly based on the above-described embodiments, and not only the appended claims but also all ranges equivalent to or equivalently changed from the claims are within the scope of the spirit of the disclosure.

According to the disclosure, by providing a user interface that is explicit and easy to understand during a test of evaluating cognitive function decline online, an aged user may perform the test of evaluating cognitive function decline without difficulty.

Also, during a test of evaluating cognitive function decline online, the test is performed by directly involving a user and reducing involvement of a tester, and thus test accuracy regarding the user may be improved.

In addition, a test result is immediately checked after a test of evaluating cognitive function decline online is completed, and a stored past result is checked for tracing, and thus a quick and accurate diagnosis and preventive measures may be presented to a patient.

The effects of the disclosure are not limited to those mentioned above, and other effects that are not mentioned may be clearly understood by one of ordinary skill in the art from the scope of claims.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A test method for evaluating cognitive function decline, the test method being executed by a processor of a test apparatus for evaluating cognitive function decline, and comprising:
performing a testing stage of testing how many missions presented for each of a plurality of test items a user performs, and calculating a test score for each of the plurality of test items;
performing a category classifying stage of classifying at least one test item from among the plurality of test items as one neurocognitive category from among a plurality of neurocognitive categories; and
performing an evaluating stage of evaluating whether there is cognitive function decline in the one neurocognitive category, based on a test score of the at least one test item classified as the one neurocognitive category.

2. The test method of claim 1, wherein the performing of the testing stage comprises performing a second testing stage of performing a test for a second test item, in which words are learned while instructing a pre-set category clue and it is evaluated how many learned words are recalled after a pre-set time, and calculating a second test score,
wherein the performing of the second testing stage comprises:
performing a (2-1)th testing stage; and
performing a (2-2)th testing stage when a pre-set time has elapsed after the performing of the (2-1)th testing stage is completed.

3. The test method of claim 2, wherein the performing of the (2-1)th testing stage comprises:
providing a (2-1-1)th instruction in which a plurality of presented words are illustrated and it is instructed to choose a word corresponding to the pre-set category clue from among the plurality of presented words;
receiving a word choice result of choosing one word from among the plurality of presented words in response to the (2-1-1)th instruction;
calculating a (2-1-1)th test score by comparing the word choice result with a correct word;
providing a (2-1-2)th instruction in which an illustration of the plurality of presented words is not provided and it is instructed to recall and utter a word belonging to the pre-set category clue from among the plurality of presented words that have been illustrated previously;
calculating a (2-1-2)th test score by receiving an utterance result for the word belonging to the pre-set category clue, in response to the (2-1-2)th instruction, converting the utterance result into an utterance text, and comparing the utterance text with a correct text; and
calculating a (2-1)th test score by adding the (2-1-1)th test score and the (2-1-2)th test score.

4. The test method of claim 3, wherein the performing of the (2-2)th testing stage comprises:
providing a (2-2-1)th instruction in which it is instructed to recall and utter a presented word corresponding to the category clue from among the plurality of presented words illustrated when the (2-1)th testing stage was performed;
calculating a (2-2-1)th test score by receiving an utterance result of the presented word corresponding to the category clue from among the plurality of presented words illustrated when the (2-1)th testing stage was performed, in response to the (2-2-1)th instruction, converting the utterance result into an utterance text, and comparing the utterance text with a correct text;
providing, together with a pre-set presented word, a (2-2-2)th instruction in which it is instructed to choose whether the pre-set presented word is a word illustrated in a previous test;
calculating a (2-2-2)th test score by comparing a result of choosing whether the pre-set presented word is a word illustrated in the previous test, in response to the (2-2-2)th instruction, with a correct word; and
calculating a (2-2)th test score by adding the (2-2-1)th test score and the (2-2-2)th test score.

5. The test method of claim 2, further comprising:
before the performing of the (2-1)th testing stage, providing, in a text, a method of performing the (2-1)th testing stage; and
before the performing of the (2-2)th testing stage, providing, in a text, a method of performing the (2-2)th testing stage.

6. The test method of claim 1, wherein the performing of the testing stage comprises:
performing a fourth testing stage of performing a test for a fourth test item, in which it is evaluated whether a pre-set presented word and a color of the pre-set presented word are accurately uttered, and calculating a fourth test score,
wherein the performing of the fourth testing stage comprises:
performing a (4-1)th testing stage; and
performing a (4-2)th testing stage after the performing of the (4-1)th testing stage is completed.

7. The test method of claim 6, wherein the performing of the (4-1)th testing stage comprises:
providing a (4-1)th instruction in which the pre-set presented word and two choice words are illustrated and it is instructed to choose a same word as the pre-set presented word from among the two choice words; and
calculating a (4-1)th test score by receiving a result of choosing the same word as the pre-set presented word from among the two choice words, in response to the (4-1)th instruction, and comparing the result of choosing with a correct answer.

8. The test method of claim 7, wherein the performing of the (4-2)th testing stage comprises:
providing a (4-2)th instruction in which the pre-set presented word and two color choice words are illustrated and it is instructed to choose a word having a same color as the pre-set presented word from among the two color choice words; and
calculating a (4-2)th test score by receiving a result of choosing the word having the same color as the pre-set presented word from among the two color choice words, in response to the (4-2)th instruction, and comparing the result of choosing with a correct answer.

9. The test method of claim 6, further comprising:
before the performing of the (4-1)th testing stage, providing, in a text, a method of performing the (4-1)th testing stage, and performing a practice test for the (4-1)th testing stage; and
before the performing of the (4-2)th testing stage, providing, in a text, a method of performing the (4-2)th testing stage, and performing a practice test for the (4-2)th testing stage.

10. A computer-readable recording medium having stored therein a computer program for executing the test method of claim 1, by using a computer.

11. A test apparatus for evaluating cognitive function decline, the test apparatus comprising:
a processor; and
a memory operatively connected to the processor and storing at least one code performed by the processor,
wherein the memory stores at least one code which, when executed through the processor, causes the processor to:
perform test processes of testing how may missions presented for each of a plurality of test items a user performs, and calculating a test score for each of the plurality of test items;
perform a category classification process of classifying at least one test item from among the plurality of test items as one neurocognitive category from among a plurality of neurocognitive categories; and
perform an evaluation process of evaluating whether there is cognitive function decline in the one neurocognitive category, based on a test score of the at least one test item classified as the one neurocognitive category.
